# EUROPEAN PATENT APPLICATION

(11) **EP 2 832 722 A1**
(43) Date of publication of application: **04.02.2015**
(21) Application number: 13767453.7
(22) Date of filing: 29.03.2013
(51) Int. Cl.: C07D 209/10, A61K 31/405, A61K 31/4184, A61K 31/438, A61K 31/454, A61K 31/4545, A61K 31/5377, A61K 31/5386, A61K 31/55, A61P 1/14, A61P 7/10, A61P 9/10, A61P 9/12, A61P 17/14, A61P 25/00, A61P 25/08, A61P 25/14, A61P 25/16, A61P 25/18, A61P 25/22, A61P 25/28

(54) **FUSED AZOLE DERIVATIVE**

(30) Priority: 30.03.2012 JP 2012079473
(71) Applicant: Taisho Pharmaceutical Co., Ltd., Tokyo 170-8633 (JP)
(72) Inventor: YOSHINAGA, Mitsukane, Tokyo 170-8633 (JP); ISHIZAKA, Tomoko, Tokyo 170-8633 (JP); WAKASUGI, Daisuke, Tokyo 170-8633 (JP); SHIROKAWA, Shin-ichi, Tokyo 170-8633 (JP); HATTORI, Nobutaka, Tokyo 170-8633 (JP); KASHIWA, Shuhei, Tokyo 170-8633 (JP); KUWADA, Takeshi, Tokyo 170-8633 (JP); SHIMAZAKI, Youichi, Tokyo 170-8633 (JP)
(74) Representative: Hoffmann Eitle
(86) International application number: PCT/JP2013/059457
(87) International publication number: WO 2013/147117

(57) **Abstract**

The present invention provides agents for treating or preventing diseases such as mood disorder, anxiety disorder, schizophrenia, Alzheimer's disease, Parkinson's disease, Huntington's chorea, eating disorder, hypertension, gastrointestinal disease, drug addiction, epilepsy, cerebral infarction, cerebral ischemia, cerebral edema, head injury, inflammation, immune-related disease, alopecia. Specifically, the invention provides fused azole derivatives represented by general formula (I) or pharmaceutically acceptable salts thereof that have an antagonistic action against the arginine-vasopressin 1b receptor:

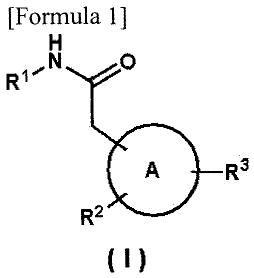

## Description

### TECHNICAL FIELD

The present invention relates to a compound with a fused azole skeleton that has an antagonistic action against the arginine-vasopressin (AVP) V1b receptor and to pharmaceutical compositions comprising the compound as an active ingredient, in particular, to agents for treating or preventing diseases such as mood disorder (including depression), anxiety disorder, schizophrenia, Alzheimer's disease, Parkinson's disease, Huntington's chorea, eating disorder, hypertension, gastrointestinal disease, drug addiction, epilepsy, cerebral infarction, cerebral ischemia, cerebral edema, head injury, inflammation, immune-related disease, and alopecia.

### BACKGROUND ART

The arginine-vasopressin (AVP) is a peptide composed of nine amino acids that is biosynthesized mainly in the hypothalamus and closely involved as a posterior pituitary hormone in the regulation of plasma osmolarity, blood pressure, and body fluid volume.

Three subtypes of AVP receptors, V1a, V1b, and V2 receptors, have been cloned until now. They are all known to be seven-transmembrane receptors. The V2 receptor is coupled to Gs to increase the cAMP level. The V1a receptor is coupled to Gq/11 to facilitate PI response and increase the intracellular Ca level. The V1a receptor is expressed in the brain, liver, adrenal gland, and vascular smooth muscle, for example, and is involved in the vasoconstrictive action. As is the V1a receptor, the V1b receptor is also coupled to Gq/11 to facilitate PI response (see Non-Patent Documents 1 and 2). The V1b receptor is found most commonly in the pituitary gland (expressed in 90% or more of ACTH secreting cells of the anterior lobe) and is estimated to participate in the AVP-mediated secretion of ACTH from the anterior pituitary. Other than in the pituitary gland, the V1b receptor is widely distributed in the brain and occurs in large amounts not only in the limbic cortex system including the hippocampus, amygdala and entorhinal cortex but also in the cerebral cortex, the olfactory bulb, and the raphe nuclei which are the nuclei of origin of the serotonin nervous system (see Non-Patent Documents 3 and 4).

In recent years, involvement of the V1b receptor in mood disorder or anxiety disorder has been suggested, and usefulness of V1b receptor antagonists is being studied. The V1b receptor KO mice exhibit reduced aggressive behavior (see Non-Patent Document 5). In addition, injection of a V1b receptor antagonist into the septal area prolonged the time spent in the open arms (anxiolytic-like action) in an elevated plus-maze test (see Non-Patent Document 6). In recent years, a peripherally administrable 1,3-dihydro-2H-indol-2-one compound has been created as a V1b receptor specific antagonist (see Patent Documents 1 to 3). Furthermore, the 1,3-dihydro-2H-indol-2-one compound has been reported to show antidepressant and anxiolytic actions in various animal models (see Non-Patent Documents 7 and 8). The compound disclosed in Patent Document 1 has high affinity for the V1b receptor (1×10⁻⁹ mol/L to 4×10⁻⁹ mol/L) on which it selectively acts; this compound, however, antagonizes AVP, AVP + CRF, and restraint stress-induced ACTH increases.

Furthermore, V1b receptor antagonists are also suggested to be useful against depression-like symptoms that emerge after withdrawal of addictive drugs. A recent report has revealed an increased AVP level in the paraventricular hypothalamic nucleus after withdrawal of the addictive drug cocaine taken chronically at escalated dose, as well as an increased V1b receptor in the anterior pituitary, and activation of the hypothalamus-pituitary gland-adrenal cortex system which is involved in stress response (see Non-Patent Document 12). It has also been reported that an increased plasma ACTH level as observed after drug withdrawal is antagonized by V1b receptor antagonistic drugs (see Non-Patent Document 12). These suggest the usefulness of V1b receptor antagonists against depression-like symptoms associated with drug dependence. Further in addition, V1b receptor antagonists lowered the alcohol intake by alcohol-preferring rats, suggesting their potential effectiveness against drug dependence (see Non-Patent Document 13).

Recently, V1b receptor antagonists having different structures from the 1,3-dihydro-2H-indol-2-one compound have been reported and they are quinazolin-4-one derivatives (see Patent Documents 4 and 10), β-lactam derivatives (see Patent Documents 5 and 7), azinon/diazinon derivatives (see Patent Document 6), benzimidazolone derivatives (Patent Document 8), isoquinolinone derivatives (see Patent Documents 9 and 10), pyridopyrimidin-4-one derivatives (see Patent Document 11), pyrrolo[1,2-a]pyrazine derivatives (see Patent Document 12), pyrazolo[1,2-a]pyrazine derivatives (see Patent document 13), quinoline derivatives (see Patent Document 14), tetrahydroquinoline sulfonamide derivatives (see Non-Patent Document 9), thiazole derivatives (see Non-Patent Document 10), and sulfonamide derivatives (see Non-Patent Document 11). However, no report has been made of the compounds disclosed in the present invention that have a fused azole skeleton.

### CITATION LIST

### PATENT DOCUMENTS

Patent Document 1: WO2001/055130
Patent Document 2: WO2005/021534
Patent Document 3: WO2005/030755
Patent Document 4: WO2006/095014
Patent Document 5: WO2006/102308
Patent Document 6: WO2006/133242
Patent Document 7: WO2007/109098
Patent Document 8: WO2008/025736
Patent Document 9: WO2008/033757
Patent Document 10: WO2008/033764
Patent Document 11: WO2009/017236
Patent Document 12: WO2009/130231
Patent Document 13: WO2009/130232
Patent Document 14: WO2011/096461

### NON-PATENT DOCUMENTS

Non-Patent Document 1: Sugimoto T, Kawashima G, J. Biol. Chem., 269, 27088-27092, 1994
Non-Patent Document 2: Lolait S, Brownstein M, PNAS, 92, 6783-6787, 1995
Non-Patent Document 3: Vaccari C, Ostrowski N, Endocrinology, 139, 5015-5033, 1998
Non-Patent Document 4: Hernando F, Burbach J, Endocrinology, 142, 1659-1668, 2001
Non-Patent Document 5: Wersinger SR,Young WS, Mol. Psychiatry, 7, 975-984, 2002
Non-Patent Document 6: Liebsch G, Engelmann M, Neurosci. Lett., 217, 101-104, 1996
Non-Patent Document 7: Gal CS, Le Fur G, 300, JPET, 1122-1130, 2002
Non-Patent Document 8: Griebel G, Soubrie P, PNAS, 99, 6370-6375, 2002
Non-Patent Document 9: Jack D. Scott, et al., Bioorganic & Medicinal Chemistry Letters, 19, 21, 6018-6022, 2009
Non-Patent Document 10: Chris A S, et. al., Bioorganic & Medicinal Chemistry Letters, 21, 92-96, 2011
Non-Patent Document 11: James B, et. al., Bioorganic & Medicinal Chemistry Letters, 21, 3603-3607, 2011.
Non-Patent Document 12: Zhou Y, et al., Neuropsychopharmacology, 36, 2062-2075,2011.
Non-Patent Document 13: Zhou Y, et al., Alcohol Clin Exp Res, 35, 1876-1883, 2011.

### SUMMARY OF INVENTION

### TECHNICAL PROBLEM

It is an object of the present invention to find novel compounds having a V1b receptor antagonistic action and to provide agents for treating or preventing diseases such as mood disorder (including depression), anxiety disorder, schizophrenia, Alzheimer's disease, Parkinson's disease, Huntington's chorea, eating disorder, hypertension, gastrointestinal disease, drug addiction, epilepsy, cerebral infarction, cerebral ischemia, cerebral edema, head injury, inflammation, immune-related disease, and alopecia.

### SOLUTION TO PROBLEM

As a result of diligent studies, the present inventors have found novel compounds with a fused azole skeleton that have a V1b receptor antagonistic action (the compounds are hereinafter referred to as "fused azole derivatives") and this has led to the accomplishment of the present invention.

Thus, the present invention includes the following embodiments:
(1) A fused azole derivative represented by Formula (I): 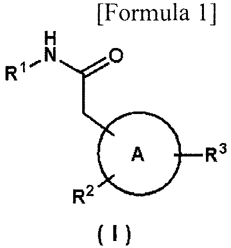 [in Formula (I),
   R¹ represents a C₁₋₅ alkyl (the C₁₋₅ alkyl is optionally substituted by one to three groups selected from the group consisting of hydroxy, halogen atoms, cyano, C₃₋₇ cycloalkyl, and C₁₋₅ alkoxy), C₃₋₇ cycloalkyl, or 4- to 8-membered saturated heterocycle;
   R² represents aryl or heteroaryl (the aryl and heteroaryl are optionally substituted by one or two groups selected from the group consisting of C₁₋₅ alkoxy, C₁₋₅ alkyl, halogen atoms, trifluoromethyl, trifluoromethoxy, cyano, hydroxy, difluoromethoxy, and C₁₋₅ alkylsulfonyl); R³ represents either the following formula (II), (III) or (IIIa): 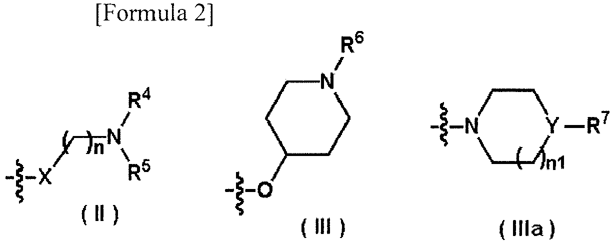
   X represents a single bond, an oxygen atom or phenylene (the phenylene is optionally substituted by a halogen atom);
   n represents an integer of 1 to 4;
   R⁴ and R⁵ which may be the same or different each represent a hydrogen atom, C₁₋₅ alkyl (the C₁₋₅ alkyl is optionally substituted by one to three groups selected from the group consisting of hydroxy, halogen atoms, cyano, C₃₋₇ cycloalkyl, and C₁₋₅ alkoxy), C₃₋₇ cycloalkyl, or a 4-to 8-membered saturated or unsaturated heterocycle containing one or more nitrogen, oxygen or sulfur atoms in the ring (the 4- to 8-membered saturated or unsaturated heterocycle is optionally substituted by one or two groups selected from the group consisting of hydroxy, C₁₋₅ alkyl, C₁₋₅ alkoxy, halogen atoms, cyano, C₂₋₅ alkanoyl, and trifluoromethyl), or
   R⁴ and R⁵, together with the adjoining nitrogen atom, may form a 4- to 8-membered saturated or unsaturated heterocycle optionally containing one or more nitrogen, oxygen or sulfur atoms in the ring in addition to the adjoining nitrogen atom (the 4- to 8-membered saturated or unsaturated heterocycle is optionally substituted by one or two groups selected from the group consisting of hydroxy, C₁₋₅ alkyl (the C₁₋₅ alkyl is optionally substituted by one or two hydroxyl groups), C₁₋₅ alkoxy, halogen atoms, cyano, C₂₋₅ alkanoyl, oxo, aminocarbonyl, mono-C₁₋₅ alkylaminocarbonyl, di-C₁₋₅ alkylaminocarbonyl, and trifluoromethyl, and the 4-to 8-membered saturated or unsaturated heterocycle optionally has a C₁₋₅ alkylene group crosslinking two different carbon atoms in the ring), 2-oxa-6-azaspiro[3.3]hept-6-yl, or 2-oxa-7-azaspiro[3.5]non-7-yl;
   R⁶ represents C₁₋₅ alkyl, C₃₋₇ cycloalkyl, or a 4- to 8-membered saturated heterocycle;
   Y represents a nitrogen atom or the formula CH;
   when Y is a nitrogen atom, R⁷ represents C₁₋₅ alkyl;
   when Y is the formula CH, R⁷ represents a 4- to 8-membered nitrogen-containing saturated heterocycle;
   n1 represents an integer of 1 or 2;
   ring A represents any one of the structures in the following formula group (IV)] 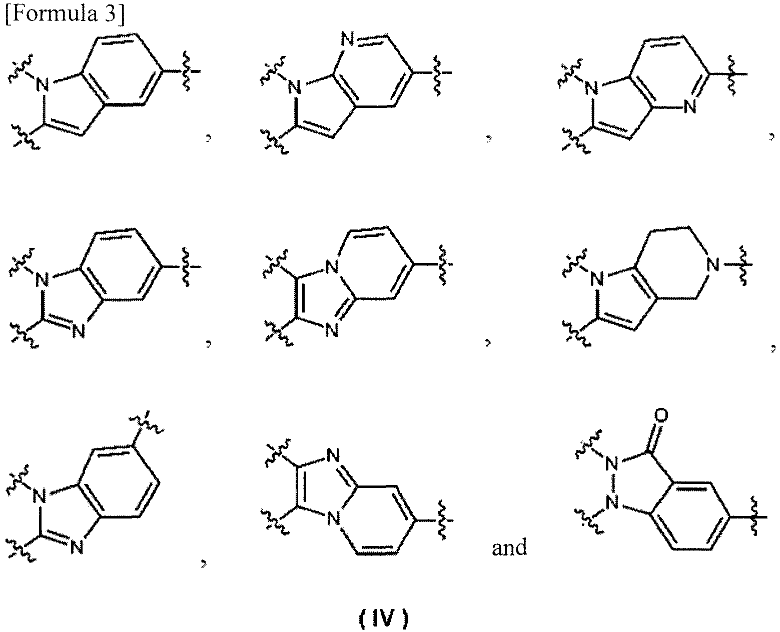 or a pharmaceutically acceptable salt thereof;
(2) A fused azole derivative represented by Formula (I): 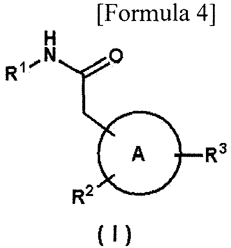 [in Formula (I),
   R¹ represents a C₁₋₅ alkyl (the C₁₋₅ alkyl is optionally substituted by one to three groups selected from the group consisting of hydroxy, halogen atoms, cyano, C₃₋₇ cycloalkyl, and C₁₋₅ alkoxy), C₃₋₇ cycloalkyl, or 4- to 8-membered saturated heterocycle;
   R² represents aryl or heteroaryl (the aryl and heteroaryl are optionally substituted by one or two groups selected from the group consisting of C₁₋₅ alkoxy, C₁₋₅ alkyl, halogen atoms, trifluoromethyl, trifluoromethoxy, cyano, hydroxy, difluoromethoxy, and C₁₋₅ alkylsulfonyl);
   R³ represents either the following formula (II) or (III): 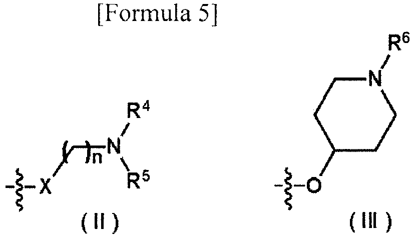
   X represents a single bond, an oxygen atom or phenylene (the phenylene is optionally substituted by a halogen atom);
   n represents an integer of 1 to 4;
   R⁴ and R³ which may be the same or different each represent a hydrogen atom, C₁₋₅ alkyl (the C₁₋₅ alkyl is optionally substituted by one to three groups selected from the group consisting of hydroxy, halogen atoms, cyano, C₃₋₇ cycloalkyl, and C₁₋₅ alkoxy), C₃₋₇ cycloalkyl, or a 4-to 8-membered saturated or unsaturated heterocycle containing one or more nitrogen, oxygen or sulfur atoms in the ring (the 4- to 8-membered saturated or unsaturated heterocycle is optionally substituted by one or two groups selected from the group consisting of hydroxy, C₁₋₅ alkyl, C₁₋₅ alkoxy, halogen atoms, cyano, C₂₋₅ alkanoyl, and trifluoromethyl), or
   R⁴ and R⁵, together with the adjoining nitrogen atom, may form a 4- to 8-membered saturated or unsaturated heterocycle optionally containing one or more nitrogen, oxygen or sulfur atoms in the ring in addition to the adjoining nitrogen atom (the 4- to 8-membered saturated or unsaturated heterocycle is optionally substituted by one or two groups selected from the group consisting of hydroxy, C₁₋₅ alkyl (the C₁₋₅ alkyl is optionally substituted by one or two hydroxyl groups), C₁₋₅ alkoxy, halogen atoms, cyano, C₂₋₅ alkanoyl, oxo, aminocarbonyl, mono-C₁₋₅ alkylaminocarbonyl, di-C₁₋₅ alkylaminocarbonyl, and trifluoromethyl, and the 4-to 8-membered saturated or unsaturated heterocycle optionally has a C₁₋₅ alkylene group crosslinking two different carbon atoms in the ring), 2-oxa-6-azaspiro[3.3]hept-6-yl, or 2-oxa-7-azaspiro[3.5]non-7-yl;
   R⁶ represents C₁₋₅ alkyl, C₃₋₇ cycloalkyl, or a 4- to 8-membered saturated heterocycle;
   ring A represents any one of the structures in the following formula group (IV)] 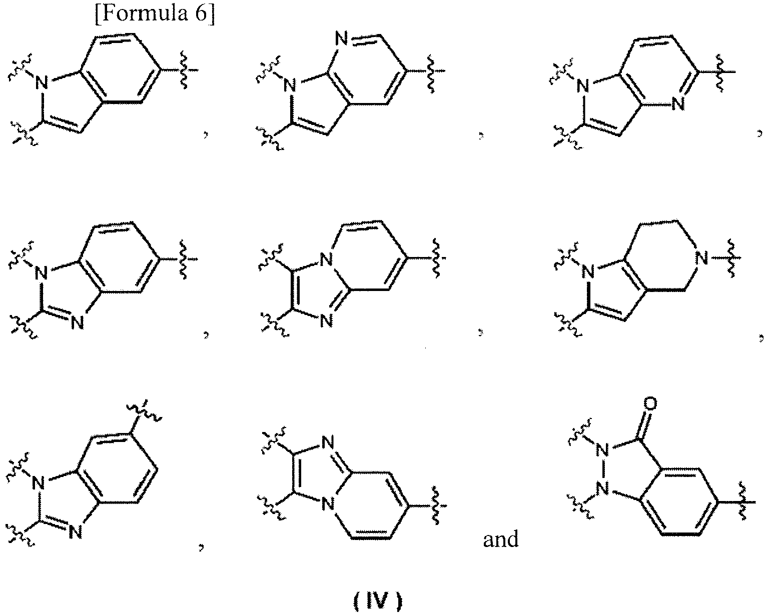 or a pharmaceutically acceptable salt thereof;
(3) The fused azole derivative or a pharmaceutically acceptable salt thereof according to (1) or (2), wherein in Formula (I),
   R¹ is C₁₋₅ alkyl;
   R² represents aryl or heteroaryl (the aryl and heteroaryl are optionally substituted by one or two groups selected from the group consisting of C₁₋₅ alkoxy and halogen atoms);
   R⁴ and R⁵ which may be the same or different are each C₁₋₅ alkyl; or
   R⁴ and R⁵, together with the adjoining nitrogen atom, may form a 4- to 8-membered saturated heterocycle optionally containing one or more oxygen atoms in the ring in addition to the adjoining nitrogen atom (the 4- to 8-membered saturated heterocycle is optionally substituted by one or two groups selected from the group consisting of hydroxy and C₁₋₅ alkyl, and the 4-to 8-membered saturated heterocycle optionally has a C₁₋₅ alkylene group crosslinking two different carbon atoms in the ring), 2-oxa-6-azaspiro[3.3]hept-6-yl, or 2-oxa-7-azaspiro[3.5]non-7-yl;
   R⁶ is C₃₋₇ cycloalkyl;
(4) The fused azole derivative or a pharmaceutically acceptable salt thereof according to any one of (1) to (3), wherein in Formula (I),
   R² is phenyl or pyridyl (the phenyl and pyridyl are optionally substituted by one or two groups selected from the group consisting of C₁₋₅ alkoxy and halogen atoms);
(5) The fused azole derivative or a pharmaceutically acceptable salt thereof according to any one of (1) to (4), wherein in Formula (I),
   ring A represents any one of the structures in the following formula group (IX): 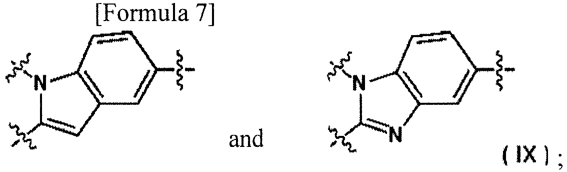
(6) An agent for treating or preventing mood disorder (including depression), anxiety disorder, schizophrenia, Alzheimer's disease, Parkinson's disease, Huntington's chorea, eating disorder, hypertension, gastrointestinal disease, drug addiction, epilepsy, cerebral infarction, cerebral ischemia, cerebral edema, head injury, inflammation, immune-related disease, or alopecia, the agent comprising the fused azole derivative or pharmaceutically acceptable salt thereof according to any one of (1) to (5) as an active ingredient; and
(7) A pharmaceutical composition comprising the fused azole derivative or pharmaceutically acceptable salt thereof according to any one of (1) to (5) as an active ingredient.

### ADVANTAGEOUS EFFECTS OF INVENTION

It has now become clear that the novel fused azole derivatives of the present invention not only show an affinity for the V1b receptor but also exhibit an antagonistic action against a stimulus to the receptor by a physiological ligand.

### DESCRIPTION OF EMBODIMENTS

The terms used in the specification have the following meanings.

The term "halogen atom" refers to a fluorine atom, a chlorine atom, a bromine atom, or an iodine atom.

The term "C₁₋₅ alkyl" refers to a linear or branched alkyl group having 1 to 5 carbon atoms, and examples thereof include methyl, ethyl, n-propyl, isopropyl, n-butyl, isobutyl, sec-butyl, tert-butyl, n-pentyl, isopentyl, neopentyl, and tert-pentyl.

The term "C₃₋₇ cycloalkyl" may be exemplified by a cyclopropyl, cyclobutyl, cyclopentyl, cyclohexyl, or cycloheptyl group.

The term "C₁₋₅ alkoxy" refers to a linear or branched alkoxy group having 1 to 5 carbon atoms, and examples thereof include methoxy, ethoxy, n-propoxy, isopropoxy, n-butoxy, isobutoxy, sec-butoxy, tert-butoxy, n-pentyloxy, isopentyloxy, neopentyloxy, and tert-pentyloxy.

The term "C₁₋₅ alkylsulfonyl" refers to a sulfonyl group substituted by the "C₁₋₅ alkyl" defined above, and examples thereof include methylsulfonyl, ethylsulfonyl, n-propylsulfonyl, isopropylsulfonyl, n-butylsulfonyl, isobutylsulfonyl, sec-butylsulfonyl, tert-butylsulfonyl, n-pentylsulfonyl, isopentylsulfonyl, neopentylsulfonyl, and tert-pentylsulfonyl.

The term "C₂₋₅ alkanoyl" refers to a linear or branched alkanoyl group having 2 to 5 carbon atoms, and examples thereof include acetyl, propionyl, butyryl, isobutyryl, valeryl, isovaleryl, and pivaloyl.

The term "mono-C₁₋₅ alkylaminocarbonyl" refers to a carbonyl group substituted by an amino having one "C₁₋₅ alkyl" group defined above as a substituent, and examples thereof include methylaminocarbonyl, ethylaminocarbonyl, n-propylaminocarbonyl, isopropylaminocarbonyl, n-butylaminocarbonyl, isobutylaminocarbonyl, sec-butylaminocarbonyl, tert-butylaminocarbonyl, n-pentylaminocarbonyl, isopentylaminocarbonyl, and neopentylaminocarbonyl.

The term "di-C₁₋₅ alkylaminocarbonyl" refers to a carbonyl group substituted by an amino having two identical or different "C₁₋₅ alkyl" groups defined above as substituents, and examples thereof include dimethylaminocarbonyl, diethylaminocarbonyl, di(n-propyl)aminocarbonyl, di(isopropyl)aminocarbonyl, ethylmethylaminocarbonyl, methyl(n-propyl)aminocarbonyl, and isopropyl(methyl)aminocarbonyl.

The term "aryl" refers to a monocyclic or bicyclic aromatic carbocycle, and examples thereof include phenyl, 1-naphthyl, and 2-naphthyl.

The term "heteroaryl" refers to a mono- or bi-cyclic aromatic group having 1 to 9 carbon atoms and also having at least one hetero atom selected from the group consisting of oxygen, nitrogen, and sulfur atoms, and examples thereof include thienyl, furyl, pyrazolyl, imidazolyl, triazolyl, tetrazolyl, thiazolyl, isothiazolyl, thiadiazolyl, oxazolyl, isoxazolyl, oxadiazolyl, pyridyl, pyrimidinyl, quinolyl, indolyl, and benzofuranyl.

The term "4- to 8-membered saturated heterocycle" may be exemplified by oxetan-3-yl, azetidin-1-yl, 1-pyrrolidinyl, piperidino, 2-piperidyl, 3-piperidyl, 1-piperazinyl, morpholin-4-yl, morpholin-3-yl, thiomorpholin-4-yl, thiomorpholin-3-yl, azepan-1-yl, 1,4-oxazepan-4-yl, and azocan-1-yl.

The term "4- to 8-membered saturated or unsaturated heterocycle containing one or more nitrogen, oxygen or sulfur atoms in the ring" may be exemplified by oxetan-3-yl, azetidin-1-yl, 1-pyrrolidinyl, piperidino, 2-piperidyl, 3-piperidyl, 1-piperazinyl, morpholin-4-yl, morpholin-3-yl, thiomorpholin-4-yl, thiomorpholin-3-yl, azepan-1-yl, 1,4-oxazepan-4-yl, and azocan-1-yl.

The term "a 4- to 8-membered saturated or unsaturated heterocycle formed together with the adjoining nitrogen atom and optionally containing one or more nitrogen, oxygen or sulfur atoms in the ring in addition to the adjoining nitrogen atom" may be exemplified by azetidin-1-yl, 1-pyrrolidinyl, piperidino, 1-piperazinyl, morpholin-4-yl, thiomorpholin-4-yl, azepan-1-yl, 1, 4-oxazepan-4-yl, azocan-1-yl, 5,6-dihydropyridin-1(2H)-yl, 1,4-diazepan-1-yl, and 1,2,3,6-tetrahydropyridin-1-yl.

The term "C₁₋₅ alkylene" refers to a divalent group having one hydrogen atom removed from the "C₁₋₅ alkyl" defined above, and examples thereof include methylene, ethylene, methylmethylene, trimethylene, methylethylene, tetramethylene, ethylethylene, and pentamethylene.

The term "4- to 8-membered saturated or unsaturated heterocycle having a C₁₋₅ alkylene group crosslinking two different carbon atoms in the ring" as referred to in connection with the "4- to 8-membered saturated or unsaturated heterocycle formed together with the adjoining nitrogen atom and optionally containing one or more nitrogen, oxygen or sulfur atoms in the ring in addition to the adjoining nitrogen atom" defined above, may be exemplified by 8-azabicyclo[3.2.1]oct-8-yl (tropinyl), 8-oxa-3-azabicyclo[3.2.1]oct-3-yl, and 3-oxa-8-azabicyclo[3.2.1]oct-8-yl. An example of the 8-azabicyclo[3.2.1]oct-8-yl that is substituted by hydroxy is 3-hydroxy-8-azabicyclo[3.2.1]oct-8-yl.

The term "4- to 8-membered saturated heterocycle formed together with the adjoining nitrogen atom and optionally containing one or two oxygen atoms in the ring in addition to the adjoining nitrogen atom" may be exemplified by azetidin-1-yl, 1-pyrrolidinyl, piperidino, morpholin-4-yl, azepan-1-yl, and azocan-1-yl.

The term "4- to 8-membered saturated heterocycle having a C₁₋₅ alkylene group crosslinking two different carbon atoms in the ring" as referred to in connection with the "4-to 8-membered saturated heterocycle formed together with the adjoining nitrogen atom and optionally containing one or more oxygen atoms in the ring in addition to the adjoining nitrogen atom" defined above, may be exemplified by 8-azabicyclo[3.2.1]oct-8-yl (tropinyl), 8-oxa-3-azabicyclo[3.2.1]oct-3-yl, and 3-oxa-8-azabicyclo[3.2.1]oct-8-yl.

The term "4- to 8-membered nitrogen-containing saturated heterocycle" may be exemplified by azetidin-1-yl, 1-pyrrolidinyl, piperidino, morpholin-4-yl, azepan-1-yl, and azocan-1-yl.

In the present invention, R¹ is preferably C₁₋₅ alkyl. More preferably, R¹ is isopropyl or tert-butyl. Even more preferably, R¹ is isopropyl.

In the present invention, R² is preferably phenyl or pyridyl (the phenyl and pyridyl are optionally substituted by one or two groups selected from the group consisting of C₁₋₅ alkoxy and halogen atoms).

In a particularly preferred case, R² is a group represented by any one of the structures in the following formula group (V).

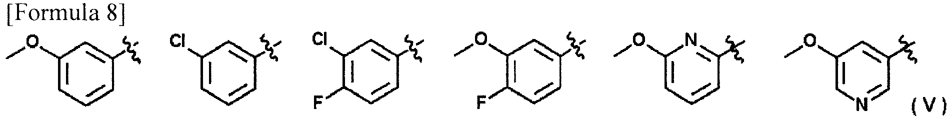

Even more preferably, R² is a group represented by any one of the structures in the following formula group (VI).

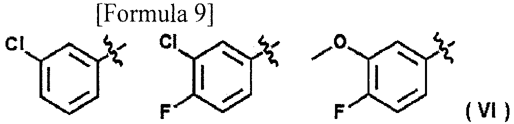

One preferred embodiment of R³ in the present invention is of the structure represented by the following formula (II): 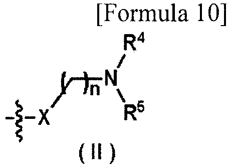 wherein in formula (II),
one preferred embodiment of X is an oxygen atom;
when X is an oxygen atom, n is preferably an integer of 3;
R⁴ and R⁵ are preferably groups that, together with the adjoining nitrogen atom, form a 4- to 8-membered saturated or unsaturated heterocycle optionally containing one or more nitrogen, oxygen or sulfur atoms in the ring in addition to the adjoining nitrogen atom (the 4- to 8-membered saturated or unsaturated heterocycle is optionally substituted by one or two groups selected from the group consisting of hydroxy, C₁₋₅ alkyl (the C₁₋₅ alkyl is optionally substituted by one or two hydroxyl groups), C₁₋₅ alkoxy, halogen atoms, cyano, C₂₋₅ alkanoyl and trifluoromethyl, and the 4- to 8-membered saturated or unsaturated heterocycle optionally has a C₁₋₅ alkylene group crosslinking two different carbon atoms in the ring), 2-oxa-6-azaspiro[3.3]hept-6-yl, or 2-oxa-7-azaspiro[3.5]non-7-yl.

More preferably, R⁴ and R³ are groups that, together with the adjoining nitrogen atom, form a 5- to 7-membered saturated heterocycle optionally containing one or more oxygen atoms in the ring in addition to the adjoining nitrogen atom (the 5- to 7-membered saturated heterocycle is optionally substituted by one or two groups selected from the group consisting of hydroxy and C₁₋₅ alkyl, and the 5- to 7-membered saturated heterocycle optionally has a C₁₋₅ alkylene group crosslinking two different carbon atoms in the ring), 2-oxa-6-azaspiro[3.3]hept-6-yl, or 2-oxa-7-azaspiro[3.5]non-7-yl.

Even more preferably, R⁴ and R⁵ are groups that, together with the adjoining nitrogen atom, form piperidino (the piperidino is optionally substituted by hydroxy), morpholin-4-yl , 2-oxa-6-azaspiro[3.3]hept-6-yl, or 3-oxa-8-azabicyclo[3.2.1]oct-8-yl.

In the above formula (II), another preferred embodiment of X is phenylene (the phenylene is optionally substituted by one halogen atom), with 1,3-phenylene (which 1,3-phenylene is optionally substituted by one halogen atom) being more preferred.

When X is phenylene, n is preferably an integer of 1; preferred R⁴ and R⁵, which may be the same or different, are C₁₋₅ alkyl;
more preferably, R⁴ and R⁵ are the same and each represent methyl.

Another preferred structure of R³ in the present invention is the following formula (III):

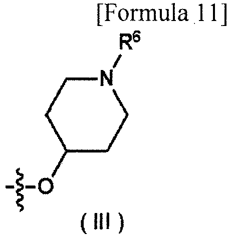

In the formula (III), R⁶ is preferably C₃₋₇ cycloalkyl.

Yet another preferred structure of R³ in the present invention is the following formula (IIIa):

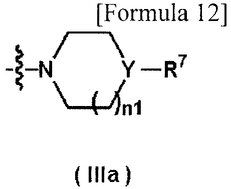

In the formula (IIIa), when Y is a nitrogen atom, R⁷ is preferably C₁₋₃ alkyl and n1 is 2; when Y is the formula CH, R⁷ is preferably 1-pyrrolidinyl and n1 is 1.

A preferred embodiment of ring A in the present invention is a group represented by any one of the structures in the following formula group (VII):

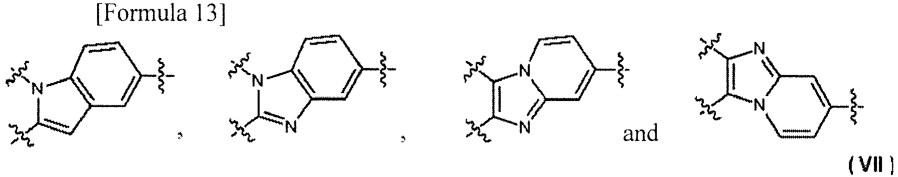

More preferably, ring A is a group represented by any one of the structures in the following formula group (VIII):

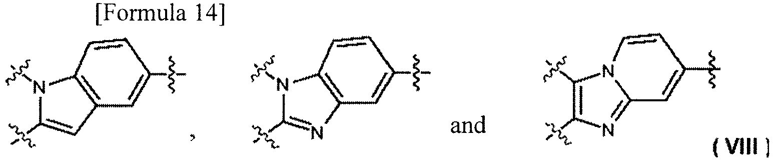

Even more preferably, ring A is a group represented by any one of the structures in the following formula group (IX):

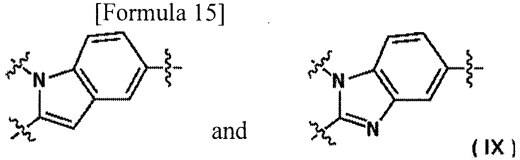

The term "pharmaceutically acceptable salt" encompasses salts with inorganic acids such as sulfuric acid, hydrochloric acid, hydrobromic acid, phosphoric acid, and nitric acid; salts with organic acids such as formic acid, trifluoroacetic acid, acetic acid, oxalic acid, lactic acid, tartaric acid, fumaric acid, maleic acid, citric acid, benzenesulfonic acid, methanesulfonic acid, p-toluenesulfonic acid, benzoic acid, camphorsulfonic acid, ethanesulfonic acid, glucoheptonic acid, gluconic acid, glutamic acid, glycolic acid, malic acid, malonic acid, mandelic acid, galactaric acid, and naphthalene-2-sulfonic acid; salts with one or more metal ions such as lithium, sodium, potassium, calcium, magnesium, zinc, and aluminum ions; and salts with amines such as ammonia, arginine, lysine, piperazine, choline, diethylamine, 4-phenylcyclohexylamine, 2-aminoethanol, and benzathine.

The compounds of the present invention can also occur as a variety of solvates. From the aspect of applicability as medicines, the compounds may also occur in the form of hydrates.

The compounds of the present invention encompass all possible forms including their enantiomers, diastereomers, equilibrium compounds, mixtures thereof at any proportions, and racemates.

The compounds of the present invention can be formulated into pharmaceutical preparations together with one or more pharmaceutically acceptable carriers, excipients, or diluents. Examples of such carriers, excipients, and diluents include water, lactose, dextrose, fructose, sucrose, sorbitol, mannitol, polyethylene glycol, propylene glycol, starch, gum, gelatin, alginate, calcium silicate, calcium phosphate, cellulose, water syrup, methylcellulose, polyvinylpyrrolidone, alkyl parahydroxybenzosorbate, talc, magnesium stearate, stearic acid, glycerin, and a variety of oils such as sesame oil, olive oil, and soybean oil.

The above-mentioned carriers, excipients, or diluents are optionally mixed with commonly used additives such as a bulking agent, a binder, a disintegrant, a pH adjuster, or a solubilizer, and can be prepared in the form of oral or parenteral medicines, such as tablets, pills, capsules, granules, powders, liquids, emulsions, suspensions, ointments, injections, or skin plasters, by common formulation technology. The compounds of the present invention can be orally or parenterally administered to adult patients in a unit dosage of 0.001 to 500 mg once or several times a day. The dosage can be appropriately adjusted depending on, for example, the type of the disease to be treated and the age, weight, and symptoms of the patient.

The compounds of the present invention may include those in which one or more hydrogen, fluorine, carbon, nitrogen, oxygen, or sulfur atoms are replaced with radioisotopes or stable isotopes thereof. These labeled compounds are useful, for example, in metabolic or pharmacokinetic studies or as ligands of receptors in biological analysis.

The compounds of the present invention can, for example, be produced in accordance with the methods shown below.

The compounds represented by Formula (I) and pharmaceutically acceptable salts thereof can be produced by various organic synthesis techniques known to those skilled in the art. For example, they can be produced by the following synthetic processes to which the present invention is by no means limited.

The term "inert solvents" as used herein covers, for example, aromatic solvents such as benzene, toluene, xylene, and pyridine; hydrocarbon solvents such as hexane, pentane, and cyclohexane; halogenated hydrocarbon solvents such as dichloromethane, chloroform, 1,2-dichloroethane, and carbon tetrachloride; ether solvents such as tetrahydrofuran, diethyl ether, 1,2-dimethoxyethane, and 1,4-dioxane; ester solvents such as ethyl acetate and ethyl formate; alcoholic solvents such as methanol, ethanol, isopropyl alcohol, tert-butyl alcohol, and ethylene glycol; ketonic solvents such as acetone and methyl ethyl ketone; amide solvents such as N,N-dimethylformamide, N-methylpyrrolidone, and N,N-dimethylactamide; sulfoxide solvents such as dimethyl sulfoxide; nitrile solvents such as acetonitrile and propionitrile; water; as well as homogeneous and heterogeneous mixed solvents thereof. These inert solvents may be chosen as appropriate depending on various reaction conditions known to those skilled in the art.

The term "bases" as used herein covers, for example, hydrides of alkali metals or alkaline earth metals such as lithium hydride, sodium hydride, potassium hydride, and calcium hydride; amides of alkali metals or alkaline earth metals such as lithium amide, sodium amide, lithium diisoproylamide, lithium dicyclohexylamide, lithium hexamethyldisilazide, sodium hexamethyldisilazide, and potassium hexamethyldisilazide; lower alkoxides of alkali metals or alkaline earth metals such as sodium methoxide, sodium ethoxide, and potassium tert-butoxide; alkyl lithium compounds such as butyl lithium, sec-butyl lithium, tert-butyl lithium, and methyl lithium; hydroxides of alkali metals or alkaline earth metals such as sodium hydroxide, potassium hydroxide, lithium hydroxide, and barium hydroxide; carbonates of alkali metals or alkaline earth metals such as sodium carbonate, potassium carbonate, and cesium carbonate; hydrogencarbonates of alkali metals or alkaline earth metals such as sodium hydrogencarbonate and potassium hydrogencarbonate; amines such as triethylamine, N-methylmorpholine, N,N-diisopropylethylamine, 1,8-diazabicyclo[5.4.0]undec-7-ene (DBU), 1,5-diazabicyclo[4.3.0]non-5-ene (DBN), and N,N-dimethylaniline; and basic heterocyclic compounds such as pyridine, imidazole, and 2,6-lutidine. These bases may be chosen as appropriate depending on various reaction conditions known to those skilled in the art.

The term "acids" as used herein covers, for example, inorganic acids such as hydrochloric acid, hydrobromic acid, sulfuric acid, nitric acid, and phosphoric acid, and organic acids such as p-toluenesulfonic acid, methanesulfonic acid, trifluoroacetic acid, formic acid, and acetic acid. These acids may be chosen as appropriate depending on various reaction conditions known to those skilled in the art.

The compounds of the present invention can, for example, be produced in accordance with the methods shown below.

Among the compounds represented by Formula (I), a compound represented by Formula (1-c) can be produced by the synthetic process shown in Scheme 1.

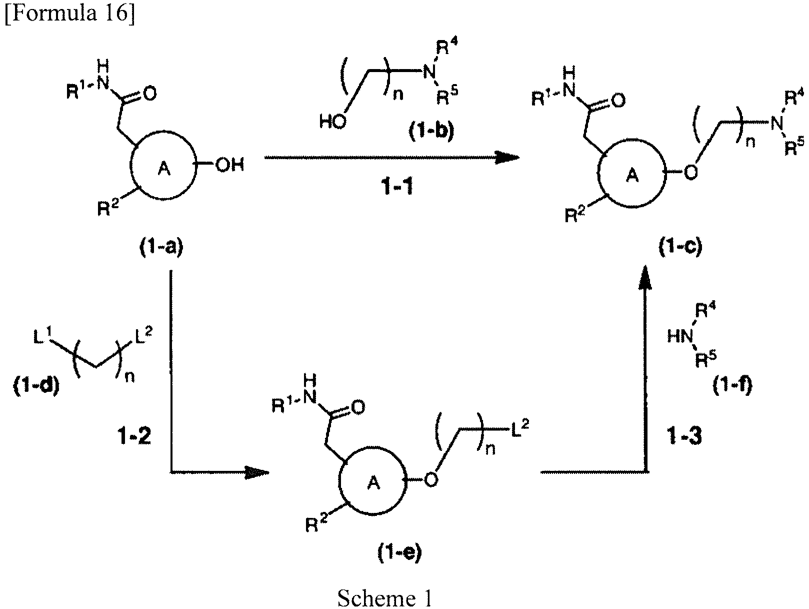

(wherein, R¹, R², R⁴, R⁵, A and n are the same as above; L¹ and L² each represent a leaving group; the term "leaving group" means a p-toluenesulfonyloxy group, a methanesulfonyloxy group, a halogen atom, etc.)

The compound represented by Formula (1-c) can be obtained from the compound represented by Formula (1-a) and the compound represented by Formula (1-b) through reaction under the conditions of the Mitsunobu reaction (Step 1-1). The comprehensive overview of the Mitsunobu reaction is found in Synthesis. 1981, 1-28.; Chem. Asian J. 2007, 2, 1340-1355.; Chem. Pharm. Bull. 2003, 51(4), 474-476.

The compound represented by Formula (1-e) can be obtained from the compound represented by Formula (1-a) and the compound represented by Formula (1-d) through reaction under a basic condition such as potassium carbonate, cesium carbonate, etc. (Step 1-2). The reaction in Step 1-2 proceeds in a solvent such as N,N-dimethylformamide, dimethyl sulfoxide, tetrahydrofuran, acetonitrile, ethanol or isopropyl alcohol or in a mixed solvent thereof in the presence of an inorganic base such as potassium carbonate or cesium carbonate or an organic base such as triethylamine or diisopropylethylamine under the temperature condition of from near 0°C to near the boiling point of the solvent.

The compound represented by Formula (1-c) can also be obtained from the compound represented by Formula (1-e) and the amine compound represented by Formula (1-f) through reaction in the presence or absence of a base (Step 1-3). The reaction in Step 1-3 proceeds in the absence of a solvent, or in a solvent such as tetrahydrofuran, acetonitrile, N,N-dimethylformamide, dimethyl sulfoxide, ethanol, isopropyl alcohol, or a mixed solvent thereof under the temperature condition of from room temperature to near the boiling point of the solvent. The reaction will proceed more smoothly in the presence of sodium iodide or potassium iodide if this is used in addition to an inorganic base such as potassium carbonate or cesium carbonate, or an organic base such as triethylamine or diisopropylethylamine.

Among the compounds represented by Formula (I), a compound represented by Formula (1-c) can also be produced by the synthetic process shown in Scheme 2.

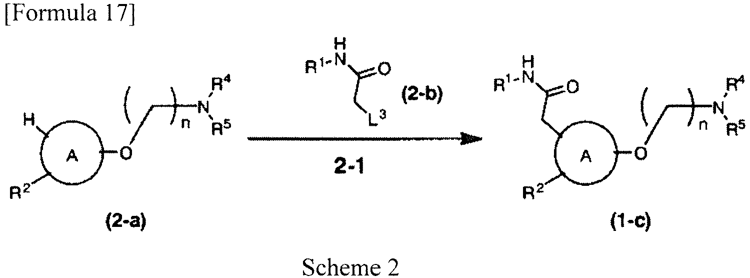

(wherein, R¹, R², R⁴, R⁵, A and n are the same as above; L³ represents a chlorine atom, a bromine atom, or an iodine atom.)

The compound represented by Formula (1-c) can also be obtained from a compound represented by Formula (2-a) and a compound represented by Formula (2-b) through alkylation reaction in the presence of a base such as sodium hydride or potassium carbonate (Step 2-1).

Among the compounds represented by Formula (I), a compound represented by Formula (1-c) can also be produced by the synthetic process shown in Scheme 3.

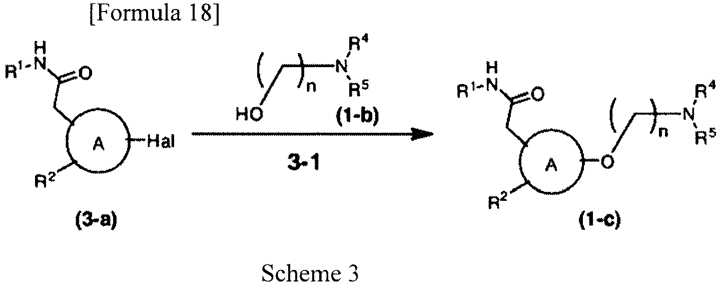

(wherein, R¹, R², R⁴, R⁵, A and n are the same as above; Hal represents a halogen atom such as a chlorine atom, a bromine atom, or an iodine atom.)

The compound represented by Formula (1-c) can also be obtained from a compound represented by Formula (3-a) and a compound represented by Formula (1-b) through reaction under the condition of etherization reaction using a palladium catalyst (Step 3-1). The comprehensive overview of etherization reaction using a palladium catalyst can be found in M. Paulucki, J. P. Wolfe, S. L. Buchwald, J. Am. Chem. Soc., 1996, 118, 10333.; G. Mann, J. F. Hartwig, J. Am. Chem. Soc. 1996, 118, 13109.; M. Watanabe, M. Nishiyama, Y. Koie, Tetrahedron Lett. 1999, 40, 8837.; Q. Shelby, N. Kataoka, G. Mann, J. F. Hartwig, J. Am. Chem. Soc. 2000, 122, 10718.; K. E. Torraca, S. Kuwabe, S. L. Buchwald, J. Am. Chem. Soc. 2000, 122, 12907.; C. A. Parrish, S. L. Buchwald, J. Org. Chem. 2001, 66, 2498.; P. M. Karen, E. Torraca, X. Huang, C. A. Parrish, and S. L. Buchwald, J. Am. Chem. Soc, 2001, 10770-10771.; Andrei V/Vorogushin, Xiaohua Huang, and Stephen L. Buchwald J. Am. Chem. Soc., 2005, 8146 -8149.

Among the compounds represented by Formula (I), a compound represented by Formula (4-b) can be produced by the synthetic process shown in Scheme 4.

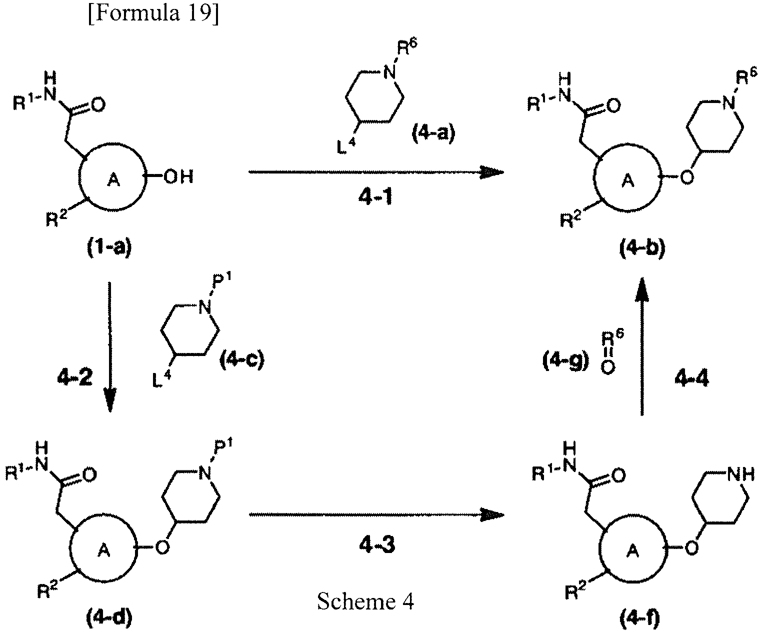

(wherein, R¹, R², R⁶, and A are the same as above; L⁴ represents a leaving group or a hydroxyl group; P¹ represents an amino protecting group such as tert-butoxycarbonyl [see Protective Groups in Organic Synthesis, 4th edition, John Wiley & Sons, INC.]

The compound represented by Formula (4-b) can be obtained by reaction between a compound represented by Formula (1-a) and a compound represented by Formula (4-a) (Step 4-1). When L⁴ is a leaving group, the reaction in Step 4-1 proceeds in an inert solvent in the presence of either an inorganic base such as potassium carbonate or cesium carbonate or an organic base such as triethylamine or diisopropylethylamine. When L⁴ is a hydroxyl group, the reaction in Step 4-1 proceeds under the same conditions of the Mitsunobu reaction as in Step 1-1.

The compound represented by Formula (4-d) can be obtained by the same method as in Step 4-1 from a compound represented by Formula (1-a) and a compound represented by Formula (4-c) (Step 4-2).

The protective group (P¹) in the resulting compound represented by Formula (4-d) can be deprotected by a common technique [see Protective Groups in Organic Synthesis, 4th edition, John Wiley & Sons, INC.] to derive a compound represented by Formula (4-f) (Step 4-3) which is then subjected to a common reductive amination reaction with a corresponding ketone (4-g), to yield a compound represented by Formula (4-b) (Step 4-4). The reductive amination reaction is accomplished by the following procedure: the ketone (4-g) and a compound represented by Formula (4-f) are reacted to generate iminium cations, which are reduced with a reducing agent such as sodium triacetoxyborohydride, borohydride, sodium borohydride, or sodium cyanoborohydride. This reaction proceeds in an inert solvent such as methanol, ethanol, tetrahydrofuran, dichloromethane, or chloroform or in a mixed solvent thereof in the presence or absence of an acid catalyst under the temperature condition of from -70°C to room temperature. If desired, this reaction may be carried out by reduction using a catalyst such as palladium on carbon and hydrogen gas.

Among the compounds represented by Formula (I), a compound represented by Formula (5-e) can be produced by the synthetic process shown in Scheme 5. 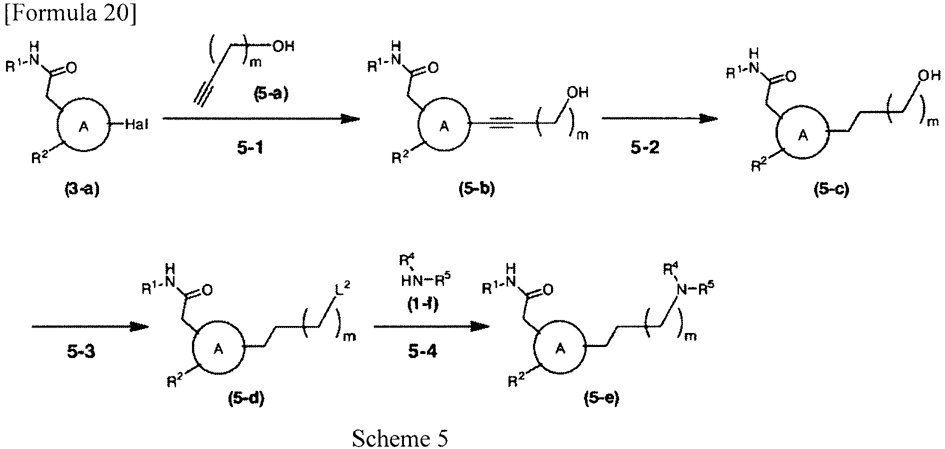 (wherein, R¹, R², R⁴, R⁵, A, L², and Hal are the same as above; m represents an integer of 1 to 3.)

A compound represented by Formula (5-b) can be obtained by reacting a compound of Formula (3-a) with a compound of Formula (5-a) under the conditions of the Sonogashira-Hagihara crosscoupling reaction (Step 5-1). The comprehensive overview of the Sonogashira-Hagihara crosscoupling reaction is found in Tetrahedron lett. 1975, 50, 4467. and Comprehensive Organic Synthesis 1991, 3, 521.

The resulting compound represented by Formula (5-b) can be subjected to reductive reaction based on a common method of hydrogenation such as one using a catalyst such as palladium on carbon and hydrogen gas, to yield a compound represented by Formula (5-c) (Step 5-2).

The hydroxyl group in the resulting compound represented by Formula (5-c) can be converted to a common leaving group (Step 5-3) and the compound is then reacted with a corresponding amine (1-f), to yield a compound represented by Formula (5-e) (Step 5-4). The reaction in Step 5-3 (convertion to a leaving group) may be exemplified by chlorination, bromination, iodination, methanesulfonylation, p-toluenesulfonylation, etc.

Examples of chlorination reaction include, for example, a method using carbon tetrachloride and triphenylphosphine, a method using thionyl chloride or phosphorus oxychloride, and a method in which p-toluenesulfonyl chloride or the like is used to give a leaving group, followed by substitution with lithium chloride or the like. These reactions may employ solvents such as tetrahydrofuran, dioxane, dichloromethane, chloroform, N,N-dimethylformamide, or mixed solvents thereof. These reactions may be performed at -50 to 100°C.

Examples of bromination reaction include, for example, a method using carbon tetrabromide and triphenylphosphine. This reaction may be performed in solvents such as tetrahydrofuran, dioxane, dichloromethane, chloroform and N,N-dimethylformamide or mixed solvents thereof at -50 to 50°C.

Examples of iodination reaction include, for example, a method using iodine, triphenyphosphine and imidazole. This reaction may employ solvents such as tetrahydrofuran, dioxane, dichloromethane, chloroform, and N,N- dimethylformamide or mixed solvnts thereof. These reactions may be performed under the temperature condition of -50 to 100°C.

Methanesulfonylation or p-toluenesulfonylation may be performed using, for example, methanesulfonyl chloride or p-toluenesulfonyl chloride, respectively. In this case, a suitable base may be added. Examples of the base to be added include organic amines such as triethylamine and diisopropylethylamine or inorganic bases such as potassium carbonate. Reaction solvents include solvents such as N,N-dimethylformamide, tetrahydrofuran, dioxane, dichloromethane, chloroform and 1,2-dichloroethane or mixed solvents thereof; both reactions may be performed under the temperature condition of -50 to 50°C.

The reaction in Step 5-4 may be performed as in Step 1-3.

Among the compounds represented by Formula (I), a compound represented by Formula (6-c) can be produced by the synthetic process shown in Scheme 6. 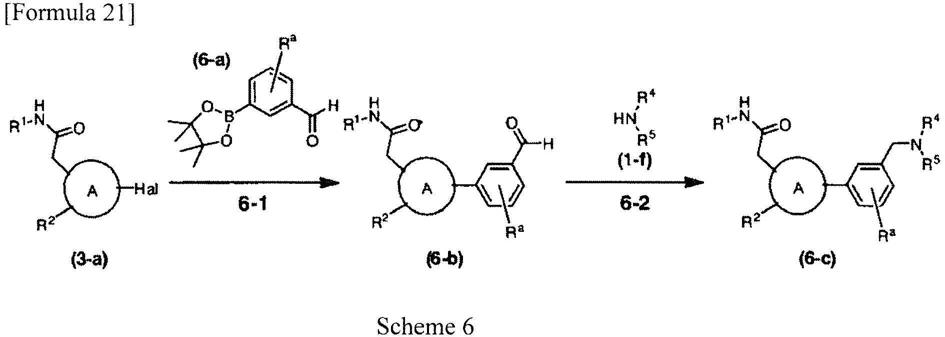 (wherein, R¹, R², R⁴, R⁵, A, and Hal are the same as above; R^{a} represents a hydrogen a tom or a halogen atom.)

The compound represented by Formula (6-b) can be obtained from the compound represented by Formula (3-a) and the boronic acid ester derivative (6-a) through reaction under the conditions of the Suzuki-Miyaura crosscoupling reaction (Step 6-1). The comprehensive overview of the Suzuki-Miyaura cross coupling reaction is found in Angew. Chem. Int., Ed. 2001, 40, 4544.

The resulting compound represented by Formula (6-b) and the amine compound represented by (1-f) can be subjected to reductive amination reacion, to yield a compound represented by Formula (6-c) (Step 6-2). The reaction in Step 6-2 can be performed by the same method under the same conditions as in Step 4-4 described above.

The compound represented by Formula (1-a) in Scheme 1 can be produced by the synthetic process shown in Scheme 7. 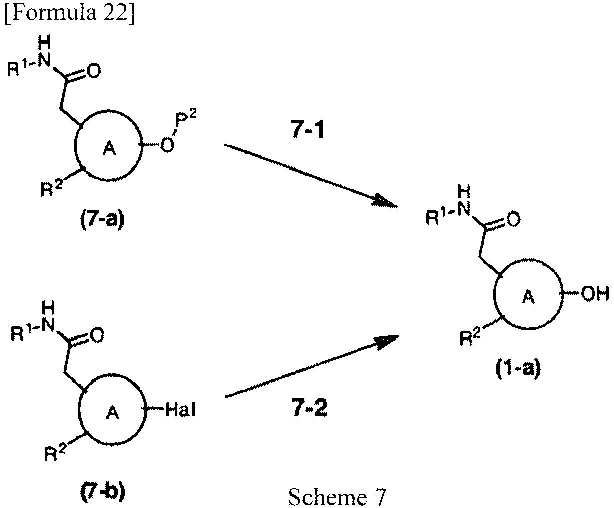 (wherein, R¹, R², A, and Hal are the same as above; P² represents a phenolic hydroxyl protecting group [see Protective Groups in Organic Synthesis, 4th edition, John Wiley & Sons, INC.]

The protective group (P²) in the compound represented by Formula (7-a) can be deprotected by a common procedure [see Protective Groups in Organic Synthesis, 4th edition, John Wiley & Sons, INC.], to yield the compound represented by Formula (1-a) (Step 7-1).

Alternatively, the compound represented by Formula (1-a) can also be obtained by converting the compound of Formula (7-b) to a boronic acid derivative and then hydroxylating the same using hydrogen peroxide (Step 7-2). This step can also be implemented by a method using hydrogen peroxide or the like in the presence of a base such as sodium hydroxide, sodium carbonate or sodium hydrogencarbonate or in accordance with the method described in WO2006/021886.

Among the compounds represented by Formula (2-a), a compound represented by Formula (8-f) can be produced by the synthetic process shown in Scheme 8. 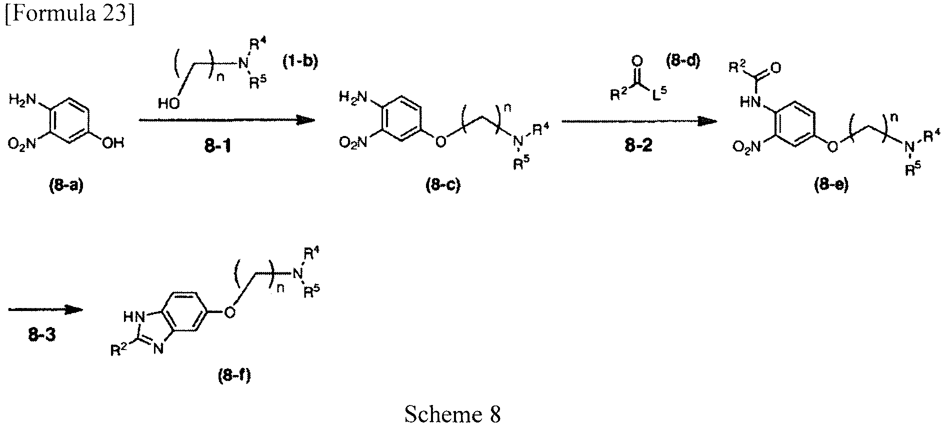 (wherein, R², R⁴, R⁵, and n are the same as above; L⁵ represents a chlorine atom, a bromine atom or a hydroxyl group.)

The compound represented by Formula (8-c) can be obtained under the conditions of the Mitsunobu reaction between the compound represented by Formula (8-a) and the compound represented by Formula (1-b) (Step 8-1). The Mitsunobu reaction here mentioned may be exemplified by the same reaction as in Step 1-1.

The compound represented by Formula (8-e) can be obtained through amidation reaction between the compound represented by Formula (8-c) and the compound represented by Formula (8-d) (Step 8-2). In the case where L⁵ is a chlorine atom or a bromine atom, the intended compound can be produced in an inert solvent in the presence or absence of a base. In the case where L⁵ is a hydroxyl group, the intended compound can be produced by various known ways of amidation reaction. The amidation reaction here mentioned may be exemplified by a method using a dehydration-condensation agent. Dehydration-condensation agents include 1-ethyl-3-(3-dimethylaminopropyl)carbodiimide hydrochloride, dicyclohexylcarbodiimide, diphenylphosphonylazide, carbonyldiimidazole, etc., with an activator such as 1-hydroxybenzotriazole or hydroxysuccinimide being optionally used depending on the need. Reaction solvents include dichloromethane, chloroform, 1,2-dichloroethane, N,N-dimethylformamide, tetrahydrofuran, dioxane, toluene, and ethyl acetate, as well as mixed solvents thereof. In this case, a base may be employed and exemplary bases include organic amines such as triethylamine and diisopropylethylamine, organic acid salts such as sodium 2-ethylhexanoate and potassium 2-ethylhexanoate, and inorganic bases such as potassium carbonate. The reaction may be performed at from -50°C to a temperature near the boiling point of the reaction solvent.

The compound represented by Formula (8-f) can be obtained from the compound represented by Formula (8-e) through intramolecular cyclization reaction in an acetic acid solvent in the presence of iron (Step 8-3).

Among the compounds represented by Formula (7-a) or the compounds represented by Formula (7-b), a compound represented by Formula (9-d) can be produced by the synthetic process shown in Scheme 9. 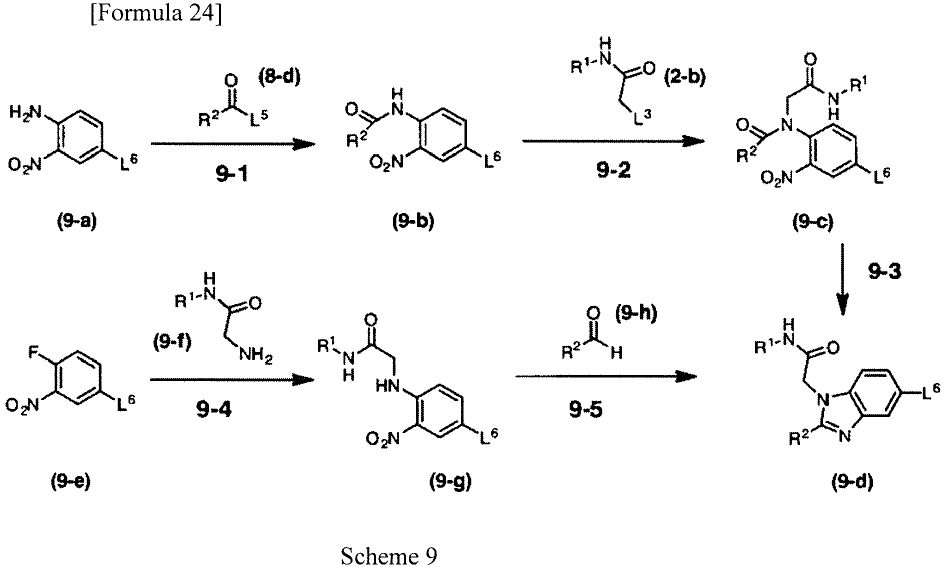 (wherein, R¹, R², L³ and L⁵ are the same as above; L⁶ represents a halogen atom or a phenolic hydroxyl group protected with a protective group; the protective group refers to conventional protective groups described in Protective Groups in Organic Chemistry by J. F. W. McOmie and Protective Groups in Organic Synthesis by T. W. Greene and P. G. M. Wuts, and these can be used to effect protection or deprotection.)

A compound represented by Formula (9-b) can be obtained by the same procedure as in Step 8-2 from the compound represented by Formula (9-a) and the compound represented by Formula (8-d) (Step 9-1).

A compound represented by Formula (9-c) can be obtained by the same procedure as in Step 2-1 from the compound represented by Formula (9-b) and the compound represented by Formula (2-b) (Step 9-2).

A compound represented by Formula (9-d) can be obtained by the same procedure as in Step 8-3 through conversion of the compound represented by Formula (9-c) (Step 9-3).

A compound represented by Formula (9-g) can be obtained by the same procedure as in Step 2-1 from the compound represented by Formula (9-e) and the compound represented by Formula (9-f) (Step 9-4).

The compound represented by Formula (9-d) can be obtained through cyclization reaction of the compound represented by Formula (9-g) and a compound represented by Formula (9-h) in an inert solvent in the presence of sodium dithionite (Step 9-5).

Among the compounds represented by Formula (7-a) or the compounds represented by Formula (7-b), compounds represented by Formulae (10-b) and (10-c) can be produced by the synthetic process shown in Scheme 10. 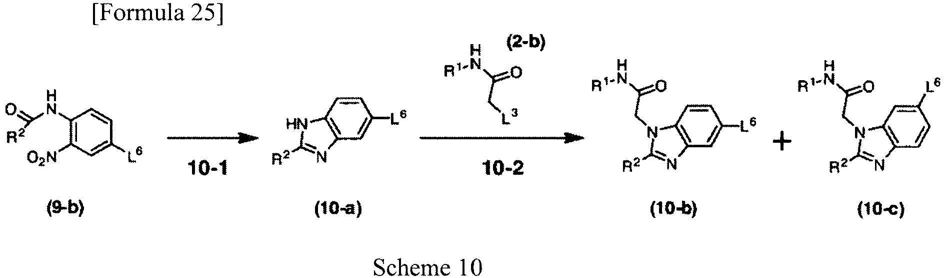 (wherein, R¹, R², L³, and L⁶ are the same as above.)

A compound represented by Formula (10-a) can be obtained by the same procedure as in Step 8-3 through conversion of the compound represented by Formula (9-b) (Step 10-1).

The compounds represented by Formulae (10-b) and (10-c) can be obtained by the same procedure as in Step 2-1 from the compound represented by Formula (10-a) and the compound represented by Formula (2-b) (Step 10-2).

Among the compounds represented by Formula (7-a), a compound represented by Formula (11-f) can be produced by the synthetic process shown in Scheme 11. 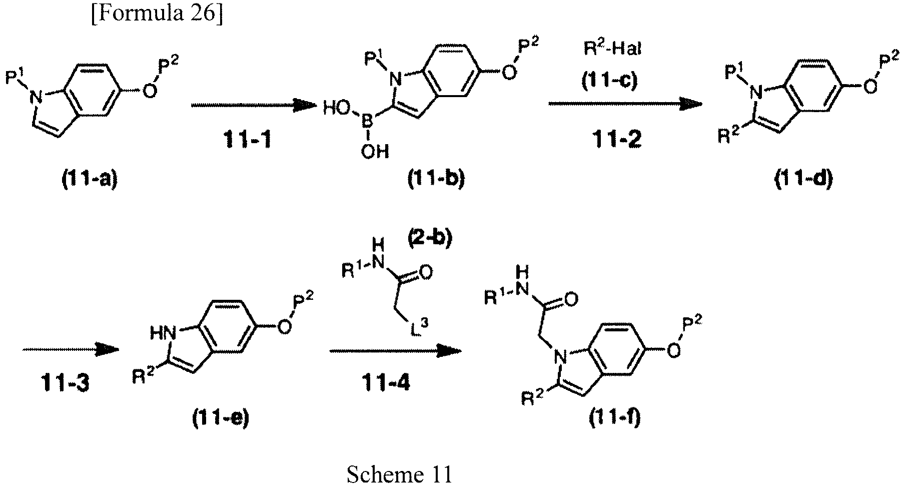 (wherein, R¹, R², P¹, P², Hal, and L³ are the same as above.)

The compound represented by Formula (11-b) can be obtained by a process that comprises first converting the compound of Formula (11-a) to a Grignard reagent, an organolithium reagent or the like in an inert solvent using various organic synthesis techniques known to skilled artisans and thereafter treating such reagents with trimethyl borate, triethyl borate, triisopropyl borate or the like (Step 11-1).

The compound represented by Formula (11-d) can be obtained through the Suzuki-Miyaura crosscoupling reaction between the compound represented by Formula (11-b) and the compound represented by Formula (11-c) (Step 11-2). The comprehensive overview of the Suzuki-Miyaura crosscoupling reaction is found in Chem. Rev. 1995, 95, 2457-2483.

The compound represented by Formula (11-e) can be produced by removing the protective group P¹ in the compound (11-d) using various organic synthesis techniques known to skilled artisans [see Protective Groups in Organic Synthesis, 4th edition, John Wiley & Sons, INC.] (Step 11-3).

The compound represented by Formula (11-f) can be obtained by the same procedure as in Step 2-1 from the compound represented by Formula (11-e) and the compound represented by Formula (2-b) (Step 11-4).

Among the compounds represented by Formula (2-a), the compound represented by Formula (12-e) can be produced by the synthetic process shown in Scheme 12. 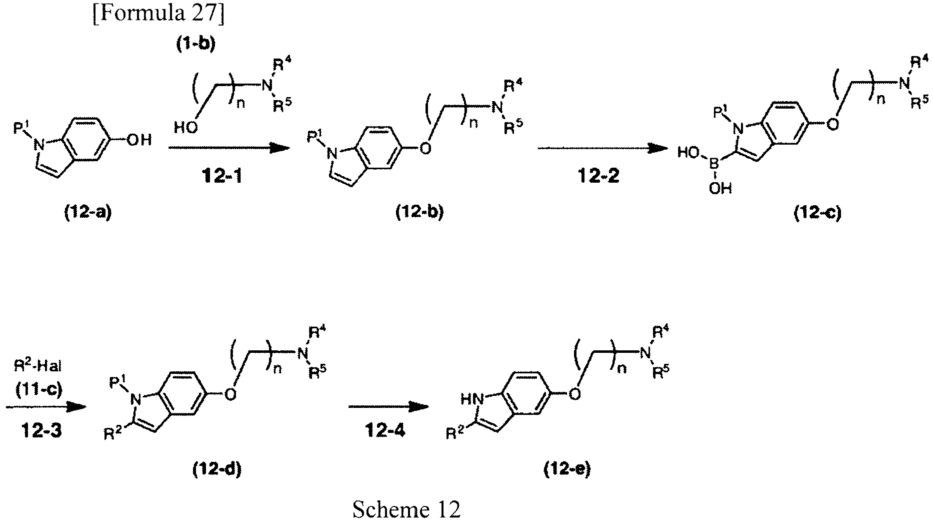 (wherein R², R⁴, R⁵, n, P¹, and Hal are the same as above.)

The compound represented by Formula (12-b) can be obtained under the conditions of the Mitsunobu reaction between the compound represented by Formula (12-a) and the compound represented by Formula (1-b) (Step 12-1). The Mitsunobu reaction here mentioned may be exemplified by the same reaction as in Step 1-1.

The compound represented by Formula (12-c) can be obtained by converting the compound of Formula (12-b) using the same procedure as in Step 11-1 (Step 12-2).

The compound represented by Formula (12-d) can be obtained by the same procedure as in Step 11-2 from the compound represented by Formula (12-c) and the compound represented by Formula (11-c) (Step 12-3).

The compound represented by Formula (12-e) can be obtained by converting the compound of Formula (12-d) using the same procedure as in Step 11-3 (Step 12-4).

Among the compounds represented by Formula (7-b), the compound represented by Formula (13-e) can be produced by the synthetic process shown in Scheme 13. 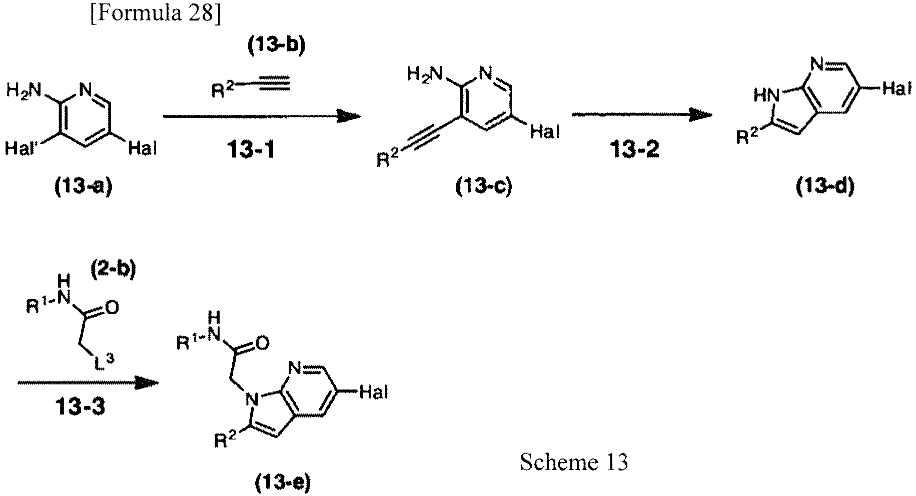 (wherein R¹, R², L³, and Hal are the same as above; Hal' represents a halogen atom.)

The compound represented by Formula (13-c) can be obtained by the same procedure as in Step 5-1 through the Sonogashira-Hagihara crosscoupling reaction between the compound represented by Formula (13-a) and the compound represented by Formula (13-b) (Step 13-1).

The compound represented by Formula (13-d) can be obtained from the compound of Formula (13-c) in an inert solvent such as N-methylpiperidone (NMP), DMF, THF, DMSO, etc. in the presence of an alkali metal compound base such as potassium tert-butoxide or sodium ethoxide (Step 13-2). The reaction can be performed in the range from room temperature to a temperature near the boiling point of the solvent.

The compound represented by Formula (13-e) can be obtained from the compound represented by Formula (13-d) and the compound represented by Formula (2-b) using the same procedure as in Step 2-1 (Step 13-3).

Among the compounds represented by Formula (7-b), the compound represented by Formula (14-e) can be produced by the synthetic process shown in Scheme 14. 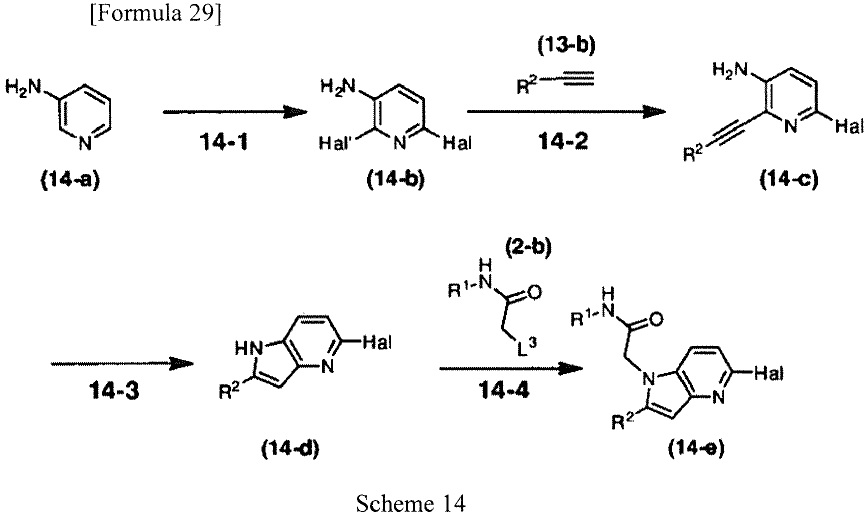 (wherein R¹, R², L³, Hal, and Hal' are the same as above.)

The compound represented by Formula (14-b) can be obtained from the compound of Formula (14-a) by subjecting it to an electrophilic substitution reaction with a halogenating agent (Step 14-1). Here the electrophilic substitution reaction proceeds in an inert solvent or in a solventless manner in the presence or absence of an acid and in the presence of a halogenating agent such as chlorine, bromine, iodine or N-chlorosuccinimide, N-bromosuccinimide or N-iodosuccinimide under the temperature condition of from near 0°C to near the boiling point of the solvent. The comprehensive overview of the electrophilic substitution reaction can be found in Comprehensive Organic Transformations, Second Edition, 1999, John Wiley & Sons, INC.

The compound represented by Formula (14-c) can be obtained by the same procedure as in Step 5-1 through the Sonogashira-Hagihara crosscoupling reaction between the compound represented by Formula (14-b) and the compound represented by Formula (13-b) (Step 14-2).

The compound represented by Formula (14-d) can be obtained from the compound represented by Formula (14-c) using the same procedure as in Step 13-2 (Step 14-3).

The compound represented by Formula (14-e) can be obtained from the compound represented by Formula (14-d) and the compound represented by Formula (2-b) using the same procedure as in Step 2-1 (Step 14-4).

Among the compounds represented by Formula (7-b), the compound represented by Formula (15-f) can be produced by the synthetic process shown in Scheme 15. 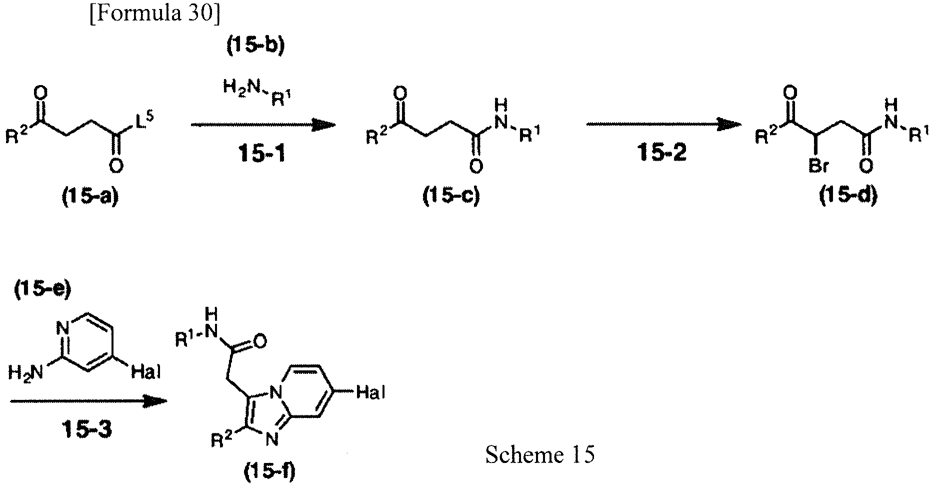 (wherein R¹, R², L⁵, and Hal are the same as above.)

The compound represented by Formula (15-c) can be obtained from the compound of Formula (15-a) and a corresponding amine (15-b) using the same procedure as in Step 8-2 (Step 15-1).

The compound represented by Formula (15-d) can be obtained by reacting the compound of Formula (15-c) with bromine in an inert solvent such as carbon tetrachloride, chloroform, dichloromethane, or the like (Step 15-2).

The compound represented by Formula (15-f) can be obtained by reaction between the compound represented by Formula (15-d) and the compound represented by Formula (15-e) in an inert solvent such as acetonitrile, ethanol, DMF or the like (Step 15-3).

Among the compounds represented by Formula (7-a), the compound represented by Formula (16-e) can be produced by the synthetic process shown in Scheme 16. 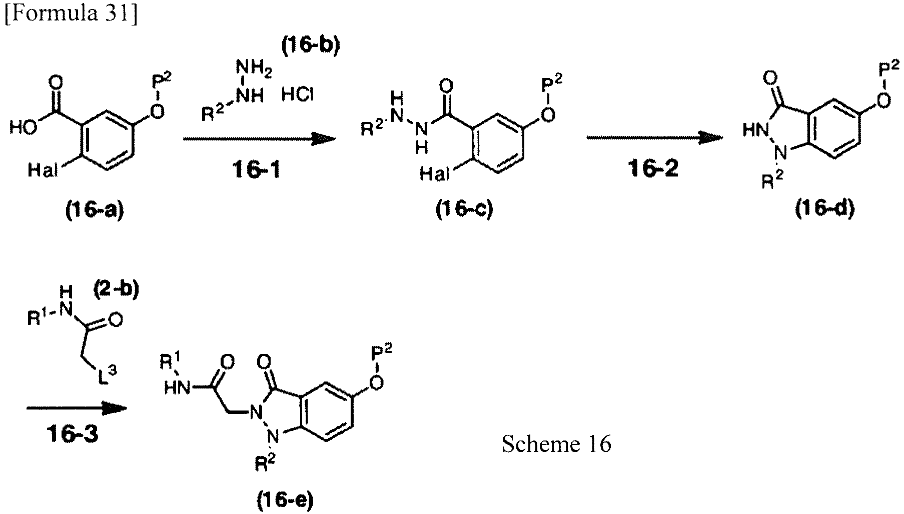 (wherein R¹, R², L³, P², and Hal are the same as above.)

The compound represented by Formula (16-c) can be obtained by a process which comprises reacting the compound of Formula (16-a) with DMF and oxalyl chloride in chloroform or thionyl chloride in toluene so as to convert it to an acid halide derivative and then subjecting the same to amidation reaction with the hydrazine compound of Formula (16-b) (Step 16-1).

The compound represented by Formula (16-d) can be obtained by cyclization through the Ullmann reaction within the molecule of the compound represented by Formula (16-c) (Step 16-2). The comprehensive overview of the Ullmann reaction can be found in Ley, S. V.; Thomas, A. W. Angew. Chem., Int. Ed. 2003, 42, 5400-5449.

The compound represented by Formula (16-d) can be obtained by the same procedure as in Step 2-1 from the compound represented by Formula (16-d) and the compound represented by Formula (2-b) (Step 16-3).

Among the compounds represented by Formula (7-a), the compound represented by Formula (17-g) can be produced by the synthetic process shown in Scheme 17. 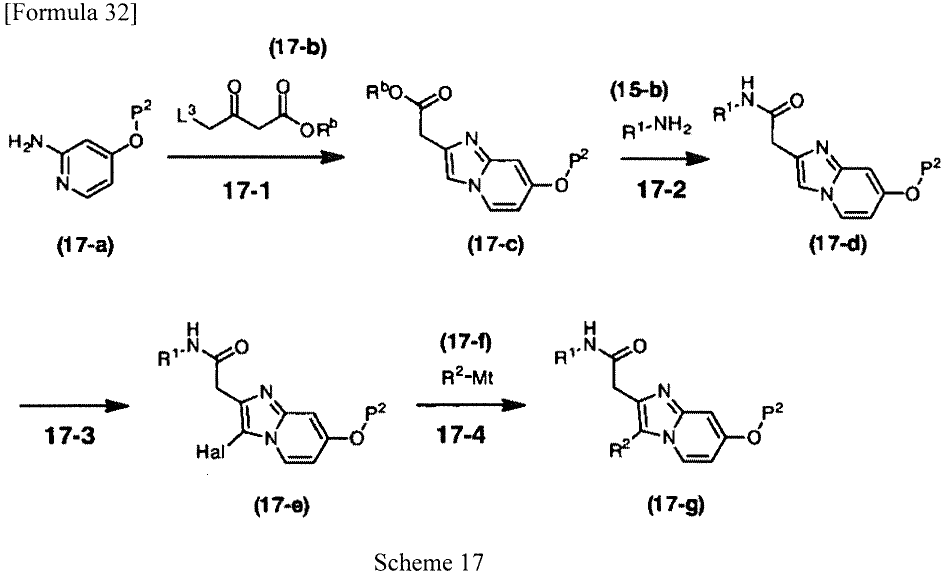 (wherein R¹, R², L³, P², and Hal are the same as above; R^{b} represents C₁₋₅ alkyl; Mt represents a metal atom or a metal atomic group that are used in the coupling reaction and may be exemplified by a magnesium reagent, a zinc reagent, a boron reagent having boric acid or a boric acid ester bound thereto, and a tin reagent.)

The compound represented by Formula (17-c) can be obtained by reaction between the compound of Formula (17-a) and the compound of Formula (17-b) in an inert solvent such as 1,2-dimethoxyethane, ethanol, THF, or DMSO at an appropriate temperature such as room temperature or reflux (Step 17-1).

The compound represented by Formula (17-d) can be obtained by amidation reaction between the compound of Formula (17-c) and the compound of Formula (15-b) in an inert solvent in the presence of diethylaluminum chloride (Step 17-2).

The compound represented by Formula (17-e) can be obtained by subjecting the compound represented by Formula (17-d) to electrophilic substitution reaction with a halogenating agent (Step 17-3). Exemplary halogenating agents include chlorine, bromine, iodine, N-chlorosuccinimide, N-bromosuccinimide, and N-iodosuccinimide.

The compound represented by Formula (17-g) can be obtained from the compound represented by Formula (17-e) and the compound represented by Formula (17-f) through the Migita-Kosugi-Stille crosscoupling reaction or the Suzuki-Miyaura crosscoupling reaction (Step 17-4). The comprehensive overview of the Migita-Kosugi-Stille crosscoupling reaction is found in Angew. Chem. Int., Ed. 2004, 43, 4704-4734.

Among the compounds represented by Formula (I), the compound represented by Formula (18-g) can be produced by the synthetic process shown in Scheme 18. 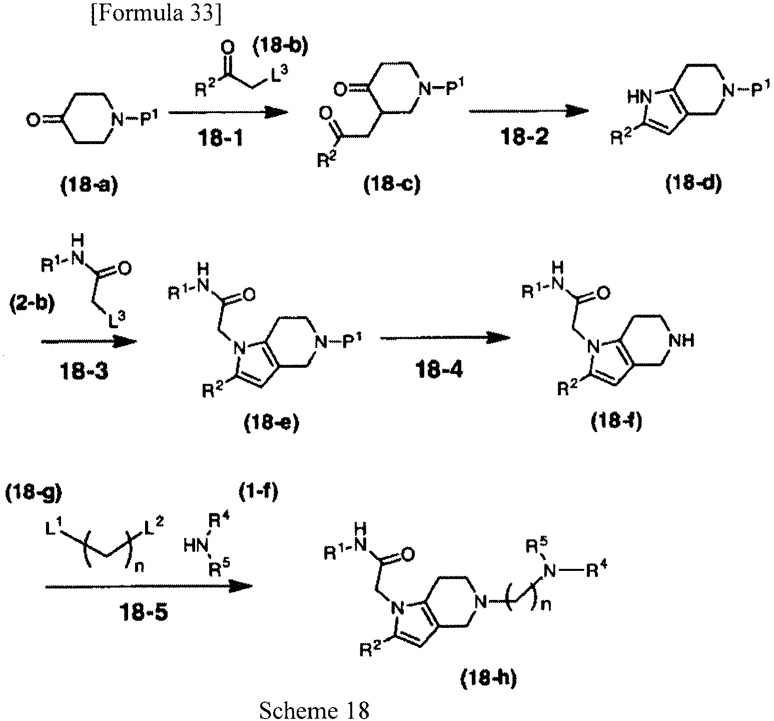 (wherein R¹, R², R⁴, R⁵, n, L¹, L², L³, and P¹ are the same as above.)

The compound represented by Formula (18-c) can be obtained by a process comprising adding pyrrolidine to the compound represented by Formula (18-a) so that the ketone is activated at α-position and then adding the compound represented by Formula (18-b) in the presence of a base such as N,N-diisopropylethylamine (Step 18-1).

The compound represented by Formula (18-d) can be obtained by cyclization reaction of the compound of Formula (18-c) in an inert solvent in the presence of ammonium acetate (Step 18-2).

The compound represented by Formula (18-e) can be obtained by the same procedure as in Step 2-1 from the compound represented by Formula (18-d) and the compound represented by Formula (2-b) (Step 18-3).

The compound represented by Formula (18-f) can be obtained by the same procedure as in Step 11-3 through deprotection of the compound represented by Formula (18-e) (Step 18-4).

The compound represented by Formula (18-h) can be obtained by a process comprising alkylating the compound of Formula (18-f) with the compound of Formula (18-g) in an inert solvent in the presence of a base such as N,N-diisopropylethylamine and then adding a corresponding amine compound represented by Formula (1-f) (Step 18-5).

The compound represented by Formula (9-d) can be produced by the synehtic process shown in Scheme 19.

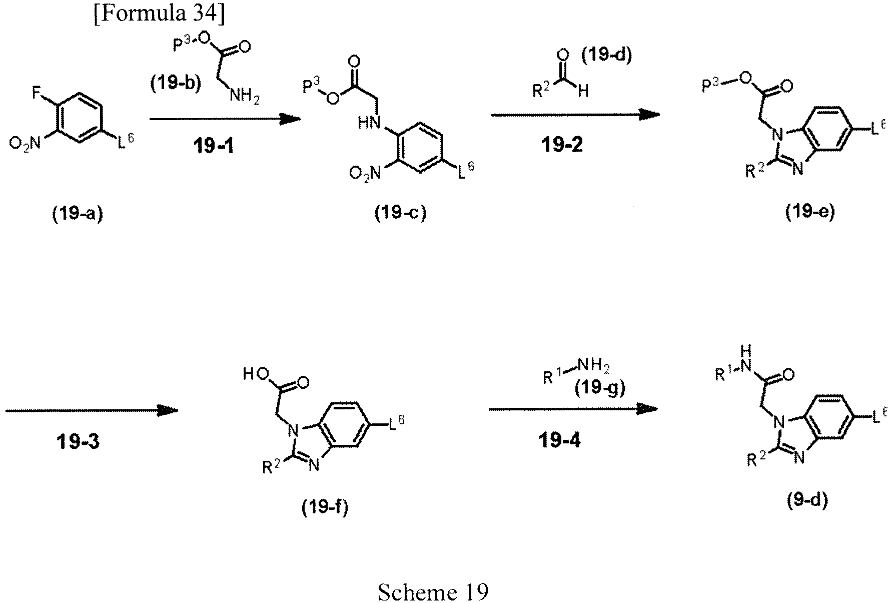

(wherein R¹, R², and L⁶ are the same as above; P³ represents a carboxyl protecting group; the protective group refers to conventional protective groups described in Protective Groups in Organic Chemistry by J. F. W. McOmie and Protective Groups in Organic Synthesis by T. W. Greene and P. G. M. Wuts; these may be used to perform protection or deprotection.)

The compound represented by Formula (19-c) is obtained by the same procedure as in Step 9-4 from the compound represented by Formula (19-a) and the compound represented by Formula (19-b) (Step 19-1).

The compound represented by Formula (19-e) is obtained by the same procedure as in Step 9-5 from the compound represented by Formula (19-c) and the compound represented by Formula (19-d) (Step 19-2).

The compound represented by Formula (19-f) is obtained by deprotection of the protective group P³ in the compound represented by Formula (19-e) (Step 19-3).

The compound represented by Formula (9-d) is obtained by the same procedure as in Step 8-2 from the compound represented by Formula (19-f) and the compound represented by Formula (19-g) (Step 19-4).

Among the compounds represented by Formula (I), the compound represented by Formula (20-b) can be produced by the synthetic process shown in Scheme 20. 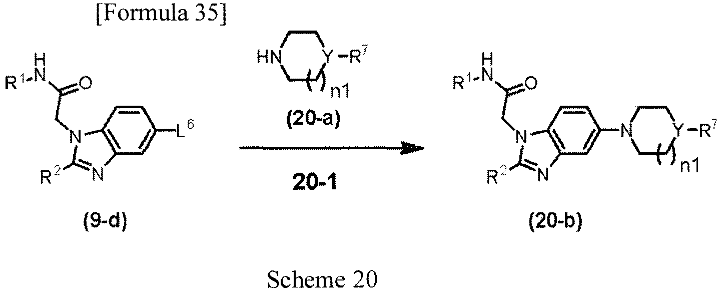 (wherein R¹, R², R⁷, L⁶, Y, and n1 are the same as above.)

The compound represented by Formula (20-b) can be obtained from the compound of Formula (9-d) and the compound of Formula (20-a) by amination reaction using a transition-metal catalyst (Step 20-1). The comprehensive concept of the amination reaction using transition-metal catalysts may be found in Angewandte Chemie, International Edition (2008), 47(34), 6338-6361.

### EXAMPLES

The present invention will now be described in more detail by Reference Examples, Examples, and Test Examples, which are by no means intended to limit the present invention and may be modified without departing from the scope of the present invention.

In Reference Examples and Examples, the "phase separator" in post-treatment is an ISOLUTE (registered trademark) Phase Separator of Biotage Inc. In purification by column chromatography, SNAP Cartridge KP-NH of Biotage Inc. was used for "SNAP Cartridge KP-NH", SNAP Cartridge KP-Sil of Biotage Inc. for "SNAP Cartridge KP-Sil", SNAP Cartridge HP-Sil of Biotage Inc. for "SNAP Cartridge HP-Sil", and Chromatorex (registered trademark) NH of Fuji Silysia Chemical Ltd. for "Chromatorex NH"; also performed was thin-layer chromatography (TLC) using TLC plate NH (product of Fuji Silysia Ltd.) and the Silica Gel 60 and Silica Gel 60N used were silica gels commercially available from Kanto Chemical Co., Ltd. In purification by preparative thin-layer chromatography (PTLC), Silica Gel 60F₂₅₄, 20 cm × 20 cm, of Merck was used. In purification by "reverse-phase column chromatography", Waters SunFire prep C18 OBD, 5.0 µm, φ 30 × 50 mm or YMC-Actus Triart C18, 5.0 µm, φ 30 × 50 mm was used.

The data described in the Reference Examples and Examples below were obtained by measurement with the following instruments:
NMR spectrometer: JNM-ECA 600 (600 MHz, JEOL Ltd.), JNM-ECA 500 (500 MHz, JEOL Ltd.), UNITY NOVA 300 (300 MHz, Varian, Inc.), or GEMINI 2000/200 (200 MHz, Varian, Inc.);
MS spectrometer: LCMS-2010EV (Shimadzu Corporation) or Platform LC (Micromass, Ltd.).
In the Reference Examples and Examples below, high-performance liquid chromatography-mass spectrometry (LCMS) was performed under the following conditions of measurement:

### Condition 1

Instrument: Platform LC (Micromass, Ltd.) and Agilent 1100 (Agilent Technologies, Inc.);
Column: SunFire C18, 2.5 µm, φ 4.6 × 50 mm (Waters Corporation);
Solvent: Solution A, water containing 0.1% trifluoroacetic acid; Solution B, acetonitrile containing 0.1% trifluoroacetic acid;
Gradient: 0 min (Solution A/Solution B = 90/10), 0.5 min (Solution A/Solution B = 90/10), 5.5 min (Solution A/Solution B = 20/80), 6.0 min (Solution A/Solution B = 1/99), and 6.3 min (Solution A/Solution B = 1/99);
Flow rate: 1 mL/min, Detection: 254 nm; and
Ionization: electron spray ionization (ESI).

### Condition 2-1

Instrument: Agilent 2900 and Agilent 6150;
Column: Waters Acquity CSH C18, 1.7 µm, φ 2.1 × 50 mm;
Solvent: Solution A, water containing 0.1% formic acid; Solution B, acetonitrile containing 0.1% formic acid;
Gradient: 0 min (Solution A/Solution B = 80/20), 1.2 to 1.4 min (Solution A/Solution B = 1/99); and
Flow rate: 0.8 mL/min, Detection: 254 nm.

### Condition 2-2

Instrument, column, and solvent are the same as those in Condition 2-1;
Gradient and flow rate: 0.8 mL/min, 0 min (Solution A/Solution B = 95/5), 1.20 min (Solution A/Solution B = 50/50), and 1.0 mL/min, 1.38 min (Solution A/Solution B = 3/97); and

### Detection: 254 nm.

In the Reference Examples and Examples below, compounds were named using ACD/Name (ACD/Labs 12.01, Advanced Chemistry Development Inc.)

Terms and reagent names in the Examples are denoted by the following abbreviations:
Brine (saturated brine), MeOH (methanol), MgSO₄ (anhydrous magnesium sulfate), K₂CO₃ (potassium carbonate), Na₂CO₃ (sodium carbonate), Na₂SO₄ (anhydrous sodium sulfate), NaHCO₃ (sodium hydrogencarbonate), NaOH (sodium hydroxide), KOH (potassium hydroxide), HCl (hydrogen chloride), IPE (diisopropyl ether), THF (tetrahydrofuran), DMF (N,N-dimethylformamide), Et₂O (diethyl ether), EtOH (ethanol), NH₄OH (25 to 28% aqueous ammonia), EtOAc (ethyl acetate), CHCl₃ (chloroform), DMSO (dimethyl sulfoxide), MeCN (acetonitrile), n-Hexane (n-hexane), Et₃N (triethylamine), iPr₂NEt (diisopropylethylamine), Pd(PPh₃)₄ [tetrakistriphenylphosphine palladium(0)], HATU [O-(7-azabenzotriazol-1-yl)-N,N,N',N'-tetramethyluronium hexafluorophosphate], DPPA (diphenylphosphoryl azide), BH₃·THF (borane-tetrahydrofuran complex), NaBO₃·4H₂O (sodium perborate tetrahydrate), 9-BBN (9-borabicyclo[3.3.1]nonane), IBX (1-hydroxy-1,2-benziodoxol-3(1H)-one 1-oxide), BBr₃ (boron tribromide), MsCl (methanesulfonyl chloride), TMSCH₂N₂ (trimethylsilyl diazomethane), n-BuLi (n-butyllithium), EDC·HCl [1-ethyl-3-(3-dimethylaminopropyl)carbodiimide hydrochloride], HOBt·H₂O (1-hydroxybenzotriazole monohydrate), Cs₂CO₃ (cesium carbonate), PdCl₂(PPh₃)₂ [bis(triphenylphosphine)palladium(II) dichloride], NaBH₄ (sodium borohydride), Na₂SO₃ (sodium sulfite), PdCl₂(dppf)·CH₂Cl₂ {[1,1'-bis(diphenylphosphino)ferrocene]palladium(II) dichloride/dichloromethane complex (1:1)}, AcOK (potassium acetate), TFA (trifluoroacetic acid), DEAD (diethyl azodicarboxylate), Na₂S₂O₄ (sodium dithionite), AcOH (acetic acid), IPA (isopropyl alcohol), Boc (tert-butoxycarbonyl), CuI (copper(I) iodide), KOtBu (tert-butoxypotassium), NaBH₃CN (sodium cyanoborohydride), and CMBP (cyanomethylenetributylphosphorane).

### • Reference Example P-A1: Synthesis of 2-nitro-4-[3-(piperidin-1-yl)propoxy]aniline

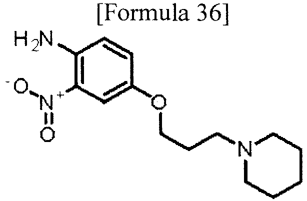

The compound 4-amino-3-nitrophenol (2.15 g) was dissolved in THF (40 mL) and after adding 1-piperidine propanol (2.00 g), triphenylphosphine (5.49 g) and DEAD (2.2 mol/L toluene sol., 9.52 mL), the mixture was stirred at room temperature for 16 hours. The reaction mixture was concentrated under reduced pressure and the resulting residue was purified by silica gel column chromatography (Chromatorex NH; mobile phase: n-hexane/EtOAc = 80/20-65/35; v/v) to give the titled compound (3.41 g as reddish brown oil). MS (ESI pos.) m/z: 280 ([M+H]⁺)

### • Reference Example P-A2: Synthesis of 3-chloro-N-{2-nitro-4-[3-(piperidin-1-yl)propoxy]phenyl}benzamide

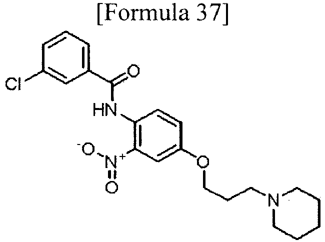

To a solution in CHCl₃ (40 mL) of the compound (3.40 g) obtained in Reference Example P-A1 and pyridine (4.92 mL), 3-chlorobenzoyl chloride (1.87 mL) was added and the mixture was stirred at room temperature for 1.5 hours. The reaction mixture was washed with a saturated solution of sodium hydrogencarbonate and saturated brine consecutively. After drying the organic layer with Na₂SO₄, the desiccant was separated by filtration and the solvent was distilled off under reduced pressure to give the titled compound (4.60 g as reddish orange oil).
MS (ESI pos.) m/z: 418([M+H]⁺)

### • Reference Example P-A3: Synthesis of 2-(3-chlorophenyl)-5-[3-(piperidin-1-yl)propoxy]-1 H-benzimidazole

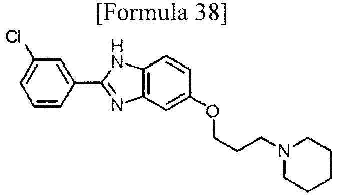

To the compound (3.00 g) obtained in Reference Example P-A2 and iron (2.23 g), AcOH (30 mL) was added and the mixture was refluxed under heating for an hour. After leaving the reaction mixture to cool, the insoluble matter was separated by filtration through Celite (registered trademark) and the filtrate was concentrated under reduced pressure. After adding EtOAc (100 mL) and a saturated solution of sodium hydrogencarbonate (100 mL), the resulting solids were recovered by filtration and washed (with water and EtOAc) to give the titled compound (1.00 g as pale brown solids).
MS (ESI pos.) m/z: 370([M+H]⁺)

### • Reference Example P-A4: Synthesis of 2-[(4-bromo-2-nitrophenyl)amino]-N-isopropyl acetamide

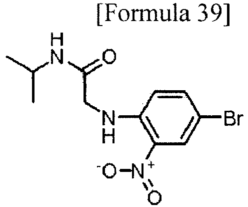

A suspension in acetonitrile (400 mL) of 5-bromo-2-fluoronitrobenzene (50.0 g), 2-amino-N-isopropylacetamide( 29.0 g) and potassium carbonate (62.8 g) was stirred at an external temperature of 80°C for 2 hours. After leaving the reaction mixture to cool, water (600 mL) and n-hexane (200 mL) were added and the resulting solids were recovered by filtration, washed (with water and n-hexane) and dried to give the titled compound (57.9 g as orange solids).
MS (ESI pos.) m/z: 316([M+H]⁺)

### • Reference Example P-A5: Synthesis of 2-[5-bromo-2-(3-chlorophenyl)-1H-benzimidazol-1-yl]-N-isopropyl acetamide

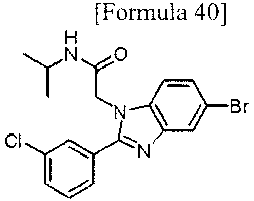

The compound (2.00 g) obtained in Reference Example P-A4 and 3-chlorobenzaldehyde (0.978 g) were suspended in a mixed solvent of DMSO (10 mL) and EtOH (10 mL) and after adding Na₂S₂O₄ (3.30 g), the mixture was stirred at an external temperature of 95°C for 18 hours. After leaving the reaction mixture to cool, water (100 mL) and IPE (20 mL) were added, followed by extraction with ethyl acetate (100 mL) and washing with saturated brine (100 mL). The aqueous layer was extracted with ethyl acetate (100 mL). The organic layers were combined and dried with Na₂SO₄; thereafter, the desiccant was separated by filtration and the filtrate was concentrated under reduced pressure. The resulting residue was purified by silica gel column chromatography (SNAP Cartridge HP-Sil; mobile phase: CHCl₃/MeOH = 100/0-95/5; v/v) to give the titled compound (1.62 g as colorless solids).
MS (ESI pos.) m/z: 406([M+H]⁺)

### • Reference Example P-A6: Synthesis of 2-[2-(3-chlorophenyl)-5-(4,4,5,5-tetramethyl-1,3,2-dioxaborolan-2-yl)-1H-benzimidazol-1-yl]-N-isopropyl acetamide

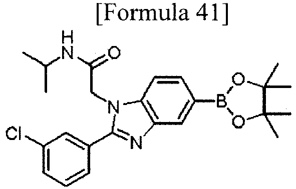

The compound (1.43 g) obtained in Reference Example P-A5, bis(pinacolato)diboron (1.79 g), PdCl₂ (dppf)·CH₂Cl₂ (0.144 g) and AcOK (1.04 g) were suspended in DMSO (15 mL) and the suspension was stirred at an external temperature of 100°C for 2 hours. After leaving the suspension to cool, water (100 mL) and IPE (20 mL) were added and the mixture was stirred for 15 minutes under cooling with ice. The resulting solids were recovered by filtration and dried to give the titled compound (1.82 g as reddish brown solids).
MS (ESI pos.) m/z: 454([M+H]⁺)

### • Reference Example P-A7: Synthesis of 2-[2-(3-chlorophenyl)-5-hydroxy-1H-benzimidazol-1-yl]-N-isopropyl acetamide

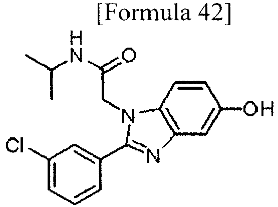

The compound (1.43 g) obtained in Reference Example P-A6 was dissolved in a mixed solvent of THF (7.5 mL) and EtOH (7.5 mL); after adding a solution of NaHCO₃ (0.591 g) in water (4.5 mL), 30% aqueous hydrogen peroxide (1.20 mL) was added dropwise and the mixture was stirred for 2 hours under cooling with ice. A solution of Na₂SO₃ (1.33 g) in water (50 mL) was added under cooling with ice and the mixture was stirred for 15 minutes. The resulting solids were recovered by filtration and dried to give the titled compound (1.48 g as pale brown solids).
MS (ESI pos.) m/z: 344([M+H]⁺)

### • Reference Example P-A8: Synthesis of 2-[2-(3-chlorophenyl)-5-(3-chloropropoxy)-1H-benzimidazol-1-yl]-N-isopropyl acetamide

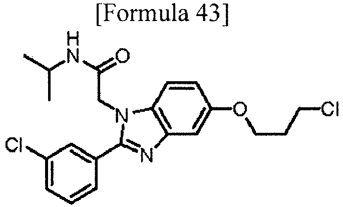

The compound (1.00 g) obtained in Reference Example P-A7, 1-bromo-3-chloropropane (0.864 mL) and potassium carbonate (2.01 g) were placed in DMF (15 mL) and the mixture was stirred at an external temperature 95°C for 2 hours. Further, 1-bromo-3-chloropropane (0.576 mL) was added and the mixture was stirred at an external temperature 95°C for 2 hours. After leaving the reaction mixture to cool, ethyl acetate (100 mL) and water (100 mL) were added and phase separation was effected; then, the organic layer was washed with saturated brine (100 mL). The aqueous layer was extracted with ethyl acetate (100 mL) and the combined organic layers were dried with Na₂SO₄; thereafter, the dried layers were separated by filtration and the filtrate was concentrated under reduced pressure. The resulting residue was purified by silica gel column chromatography (SNAP Cartridge KP-NH; mobile phase: n-hexane/CHCl₃ = 60/40-0/100; v/v) to give the titled compound (0.583 g as pale brown solids).
MS (ESI pos.) m/z: 420([M+H]⁺)

### • Reference Example P-A9: Synthesis of N-(4-bromo-2-nitrophenyl)-4-fluoro-3-methoxybenzamide

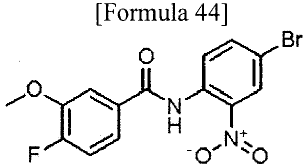

To a solution in chloroform(100 mL) of 4-bromo-2-nitroaniline (5.14 g) and pyridine (5.74 mL), 4-fluoro-3-methoxybenzoyl chloride (5.60 g) was added under cooling with ice and the mixture was stirred at room temperature for 2 hours. The resulting solids were recovered by filtration and washed to give the titled compound (1.77 g as yellow solids).
MS (ESI pos.) m/z: 369([M+H]⁺)

### • Reference Example P-A10: Synthesis of N-(4-bromo-2-nitrophenyl)-4-fluoro-N-[2-(isopropylamino)-2-oxoethyl]-3-methoxybenzamide

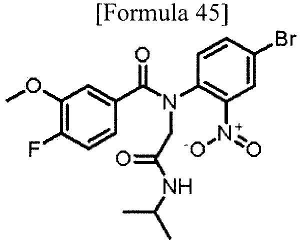

The compound (1.00 g) obtained in Reference Example P-A9, 2-bromo-N-isopropyl acetamide (537 mg) and potassium carbonate (749 mg) were placed in DMF (15 mL) and the mixture was stirred at an external temperature of 80°C for 3 hours. After leaving the reaction mixture to cool, water (50 mL) and ethyl acetate (50 mL) were added and phase separation was effected, followed by washing with saturated brine (50 mL). The aqueous layer was extracted with ethyl acetate (50 mL) twice and the organic layers were combined, dried with Na₂SO₄ and separated by filtration; the filtrate was concentrated under reduced pressure. The resulting residue was purified by silica gel column chromatography (SNAP Cartridge KP-Sil; mobile phase: n-hexane/ethyl acetate = 88/12-0/100; v/v) to give the titled compound (0.94 g as yellow solids).
MS (ESI pos.) m/z: 468([M+H]⁺)

### • Reference Example P-A11: Synthesis of 2-[5-bromo-2-(4-fluoro-3-methoxyphenyl)-1H-benzimidazol-1-yl]-N-isopropyl acetamide

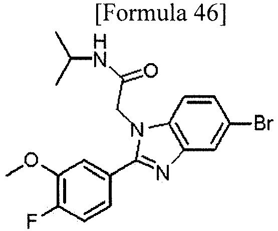

The compound (0.88 g) obtained in Reference Example P-A10 and an iron powder (583 mg) were placed in AcOH (12 mL) and the mixture was stirred at an external temperature of 100°C for 2 hours. After leaving the reaction mixture to cool, the insoluble matter was separated by filtration through Celite (registered trademark) and the filtrate was concentrated under reduced pressure. Chloroform (50 mL) and an aqueous solution of NaOH (0.5 mol/L, 50 mL) were added and phase separation was effected, followed by washing with water (50 mL) and saturated brine (50 mL). The organic layer was dried with Na₂SO₄ and then separated by filtration; the filtrate was concentrated under reduced pressure. The resulting residue was purified by silica gel column chromatography (SNAP Cartridge KP-Sil; mobile phase: chloroform/methanol = 99/1-90/10; v/v) to give the titled compound (194 mg as yellow solids).
MS (ESI pos.) m/z: 420([M+H]⁺)

### · Reference Example P-A12: Synthesis of 2-[2-(4-fluoro-3-methoxyphenyl)-5-hydroxy-1H-benzimidazol-1-yl]-N-isopropyl acetamide

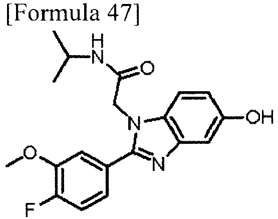

### Step P-A12-1

The compound (120 mg) obtained in Reference ExampleP-A11 was treated by the same procedure as in Reference Example P-A6, yielding a crude product (266 mg as blackish brown oil).

### Step P-A 12-2

The crude product obtained in Step P-A 12-1 was treated by the same procedure as in Reference Example P-A7, yielding the titled compound (105 mg as gray solids).
MS (ESI pos.) m/z: 358([M+H]⁺)

### • Reference Example P-A13: Synthesis of 2-[5-(3-chloropropoxy)-2-(4-fluoro-3-methoxyphenyl)-1H-benzimidazol-1-yl]-N-isopropyl acetamide

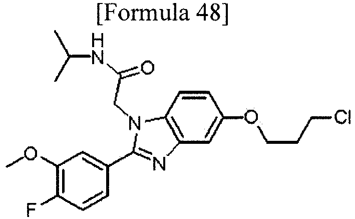

The compound (103 mg) obtained in Reference Example P-A12 was treated by the same procedure as in Reference Example P-A8, yielding the titled compound (78.8 mg as pale yellow solids).
MS (ESI pos.) m/z: 434([M+H]⁺)

### • Reference Example P-A 14: Synthesis of 5-bromo-2-(4-fluoro-3-methoxyphenyl)-1H-benzimidazole

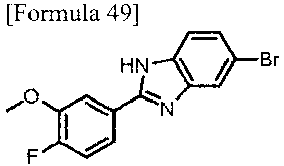

The compound (3.0 g) obtained in Reference Example P-A9 was treated by the same procedure as in Reference Example P-A11, yielding the titled compound (2.87 g as pale yellow amorphous mass).
MS (ESI pos.) m/z: 321([M+H]⁺)

### • Reference Example P-A15: Synthesis of 2-[5-bromo-2-(4-fluoro-3-methoxyphenyl)-1H-benzimidazol-1-yl]-N-isopropyl acetamide and 2-[6-bromo-2-(4-fluoro-3-methoxyphenyl)-1H-benzimidazol-1-yl]-N-isopropyl acetamide in admixture

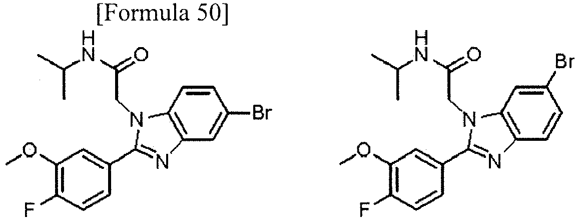

The compound (0.45 g) obtained in Reference Example P-A14 was treated by the same procedure as in Reference Example P-A10, yielding the titled compound (0.33 g as colorless solids).
MS (ESI pos.) m/z: 420([M+H]⁺)

### • Reference Example P-A16: Synthesis of 2-[5-(3-chloro-5-formylphenyl)-2-(4-fluoro-3-methoxyphenyl)-1H-benzimidazol-1-yl]-N-isopropyl acetamide and 2-[6-(3-chloro-5-formylphenyl)-2-(4-fluoro-3-methoxyphenyl)-1H-benzimidazol-1-yl]-N-isopropyl acetamide in admixture

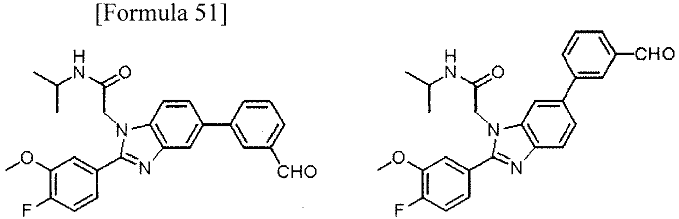

The compound (150 mg) obtained in Reference Example P-A15, 3-chloro-5-(4,4,5,5-tetramethyl-1,3,2-dioxaborolan-2-yl)benzaldehyde (124 mg), Pd(PPh₃)₄ (41.3 mg), an aqueous solution of Na₂CO₃ (2 mol/L, 1.5 mL), toluene (0.75 mL) and EtOH (1.5 mL) were stirred at an external temperature of 100°C for 2 hours. After leaving the reaction mixture to cool, water and IPE were added; the resulting solids were recovered by filtration and dried to give the titled compound (90 mg as pale brown solids).
MS (ESI pos.) m/z: 480([M+H]⁺)

### •Reference Example P-A17: Synthesis of 2-[5-bromo-2-(3-chloro-4-fluorophenyl)-1H-benzimidazol-1-yl]-N-isopropyl acetamide

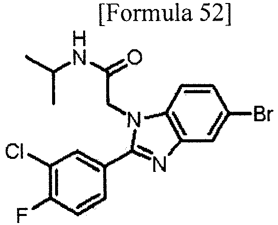

The compound (57.9 g) obtained in Reference Example P-A4 was treated by the same procedure as in Reference Example P-A5, yielding the titled compound (61.4 g as colorless solids).
MS (ESI pos.) m/z: 424([M+H]⁺)

### • Reference Example P-A18: Synthesis of 2-[2-(3-chloro-4-fluorophenyl)-5-(4,4,5,5-tetramethyl-1,3,2-dioxaborolan-2-yl)-1H-benzimidazol-1-yl]-N-isopropyl acetamide

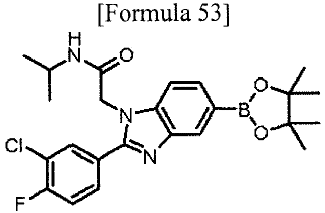

The compound (30.0 g) obtained in Reference Example P-A17 was treated by the same procedure as in Reference Example P-A6, yielding the titled compound (26.4 g as pale brown solids).
MS (ESI pos.) m/z: 472([M+H]⁺)

### •Reference Example P-A19: Synthesis of 2-[2-(3-chloro-4-fluorophenyl)-5-hydroxy-1H-benzimidazol-1-yl]-N-isopropyl acetamide

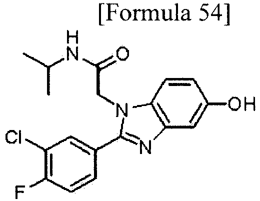

The compound (26.2 g) obtained in Reference Example P-A18 was treated by the same procedure as in Reference Example P-A7, yielding the titled compound (14.0 g as pale brown solids).
MS (ESI pos.) m/z: 362([M+H]⁺)

### •Reference Example P-A20: Synthesis ofN-(4-bromo-2-nitrophenyl)-3-chloro-4-fluorobenzamide

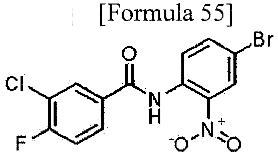

The compound 3-chloro-4-fluorobenzoyl chloride (6.7 mL) was treated by the same procedure as in Reference Example P-A10, yielding the titled compound (13.9 g as yellow solids).
MS (ESI neg.) m/z: 371([M-H]⁻)

### •Reference Example P-A21: Synthesis of 5-bromo-2-(3-chloro-4-fluorophenyl)-1H-benzimidazole

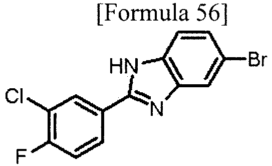

The compound (13.9 g) obtained in Reference Example P-A20 was treated by the same procedure as in Reference Example P-A11, yielding the titled compound (9.47 g as colorless solids).
MS (ESI pos.) m/z: 325([M+H]⁺)

### •Reference Example P-A22: Synthesis of 2-[5-bromo-2-(3-chloro-4-fluorophenyl)-1H-benzimidazol-1-yl]-N-isopropyl acetamide and 2-[6-bromo-2-(3-chloro-4-fluorophenyl)-1H-benzimidazol-1-yl]-N-isopropyl acetamide in admixture

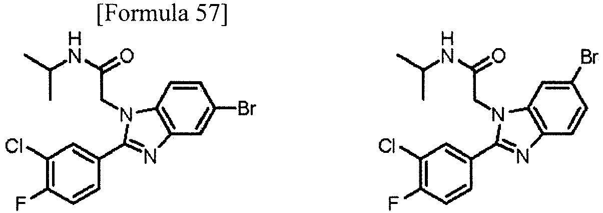

The compound (5.00 g) obtained in Reference Example P-A21 was treated by the same procedure as in Reference Example P-A10, yielding the titled compound (6.49 g as colorless solids).
MS (ESI pos.) m/z: 424([M+H]⁺)

### •Reference Example P-A23: Synthesis of 2-[2-(3-chloro-4-fluorophenyl)-5-hydroxy-1H-benzimidazol-1-yl]-N-isopropyl acetamide and 2-[2-(3-chloro-4-fluorophenyl)-6-hydroxy-1H-benzimidazol-1-yl]-N-isopropyl acetamide in admixture

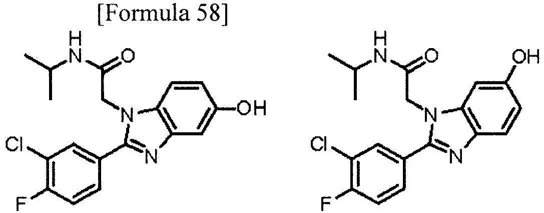

### Step P-A23-1

The compound (3.50 g) obtained in Reference Example P-A22 was treated by the same procedure as in Reference Example P-A6, yielding a crude product (10.0 g as black solids).

### Step P-A23-2

The compound (10.0 g) obtained in Step P-A23-1 was treated by the same procedure as in Reference Example P-A7, yielding the titled compound (831 mg as gray solids). MS (ESI pos.) m/z: 362([M+H]⁺)

### •Reference Example P-A24: Synthesis of 2-[2-(3-chloro-4-fluorophenyl)-5-(3-chloropropoxy)-1H-benzimidazol-1-yl]-N-isopropyl acetamide and 2-[2-(3-chloro-4-fluorophenyl)-6-(3-chloropropoxy)-1H-benzimidazol-1-yl]-N-isopropyl acetamide in admixture

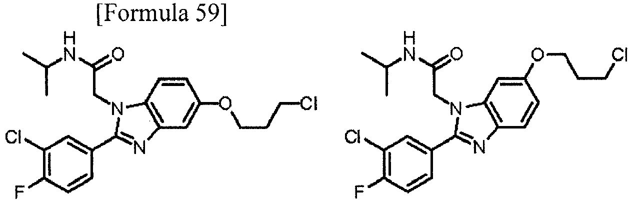

The compound (500 mg) obtained in Reference Example P-A23 was treated by the same procedure as in Reference ExampleP-A8, yielding the titled compound (363 mg as colorless solids).
MS (ESI pos.) m/z: 438([M+H]⁺)

### •Reference Example P-A25: Synthesis of 2-[2-(3-chloro-4-fluorophenyl)-5-(3-chloropropoxy)-1H-benzimidazol-1-yl]-N-isopropyl acetamide

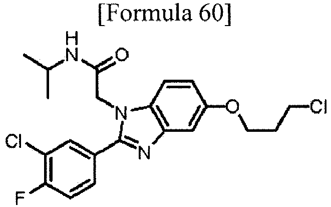

The compound (1.50 g) obtained in Reference Example P-A19 was treated by the same procedure as in Reference Example P-A8, yielding the titled compound (0.74 g as pale brown solids).
MS (ESI pos.) m/z: 438([M+H]⁺)

### •Reference Example P-A26: Synthesis of 2-[5-(4-chlorobutoxy)-2-(3-chloro-4-fluorophenyl)-1H-benzimidazol-1-yl]-N-isopropyl acetamide

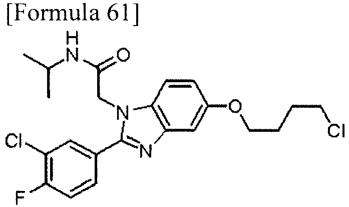

The compound (0.300 g) obtained in Reference Example P-A19 and 1-bromo-4-chlorobutane (0.286 mL) were treated by the same procedure as in Reference Example P-A8, yielding the titled compound (0.294 g as pale brown solids).
MS (ESI pos.) m/z: 452([M+H]⁺)

### •Reference Example P-A27: Synthesis of 2-[5-bromo-2-(6-methoxypyridin-2-yl)-1H-benzimidazol-1-yl]-N-isopropyl acetamide

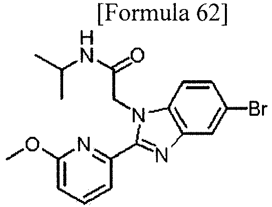

The compound (316 mg) obtained in Reference Example P-A4 and 6-methoxypicolinaldehyde (132 µL) were treated by the same procedure as in Reference Example P-A5, yielding the titled compound (204 mg as pale brown solids).
MS (ESI pos.) m/z: 403([M+H]⁺)

### •Reference Example P-A28: Synthesis of N-isopropyl-2-[2-(6-methoxypyridin-2-yl)-5-(4,4,5,5-tetramethyl-1,3,2-dioxaborolan-2-yl)-1H-benzimidazol-1-yl]acetamide

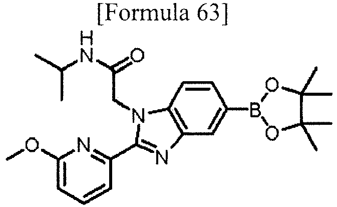

The compound (204 mg) obtained in Reference Example P-A27 was treated by the same procedure as in Reference Example P-A6, yielding the titled compound (228 mg as gray solids).
MS (ESI pos.) m/z: 451([M+H]⁺)]

### •Reference Example P-A29: Synthesis of 2-[5-hydroxy-2-(6-methoxypyridin-2-yl)-1H-benzimidazol-1-yl]-N-isopropyl acetamide

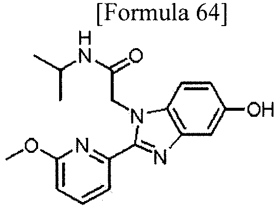

The compound (228 mg) obtained in Reference Example P-A28 was treated by the same procedure as in Reference Example P-A7, yielding the titled compound (144 mg as pale brown solids).
MS (ESI pos.) m/z: 341([M+H]⁺)

### •Reference Example P-A30: Synthesis of tert-butyl [(4-bromo-2-nitrophenyl)amino]acetate

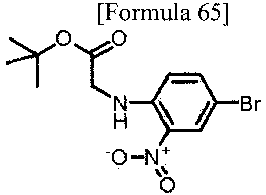

The compound tert-butyl aminoacetate hydrochloride (9.14 g) was treated by the same procedure as in Reference Example P-A4, yielding the titled compound (7.98 g as orange solids).
¹H-NMR (200 MHz, DMSO-d₆) δ (ppm); 1.50 (9 H, s), 4.16 (2 H, d, J=5.7 Hz), 6.90 (1 H, d, J=9.6 Hz), 7.67 (1 H, dd, J=9.2, 2.6 Hz), 8.19 (1 H, d, J=2.6 Hz), 8.33 - 8.42 (1 H, m).

### •Reference Example P-A31: Synthesis of tert-butyl [5-bromo-2-(4-fluoro-3-methoxyphenyl)-1H-benzimidazol-1-yl] acetate

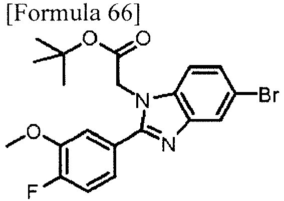

The compound (4.00 g) obtained in Reference Example P-A30 was treated by the same procedure as in Reference Example P-A5, yielding the titled compound (3.00 g as pale yellow solids).
MS (ESI pos.) m/z: 435([M+H]⁺)

### •Reference Example P-A32: Synthesis of [5-bromo-2-(4-fluoro-3-methoxyphenyl)-1H-benzimidazol-1-yl]acetic acid

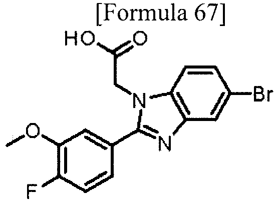

To the compound (3.00 g) obtained in Reference Example P-A31, TFA (21 ml) was added under cooling with ice and the mixture was stirred at room temperature for 8 hours. The reaction solvent was concentrated under reduced pressure and the resulting solids were washed with hexane and recovered by filtration to give the titled compound (2.11 g as colorless solids).
MS (ESI pos.) m/z: 379([M+H]⁺)

### •Reference Example P-A33: Synthesis of 2-[5-bromo-2-(4-fluoro-3-methoxyphenyl)-1H-benzimidazol-1-yl]-N-tert-butyl acetamide

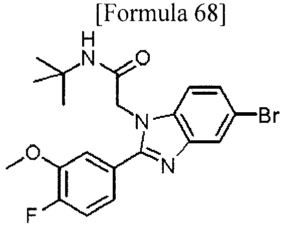

The compound (1.11 g) obtained in Reference Example P-A32 and DIEA (0.996 ml) were dissolved in DMF (11 ml) and under cooling with ice, HATU (1.67 g) and t-butylamine (3.10 ml) were added consecutively and the mixture was stirred at room temperature for 16 hours. To the reaction mixture, water (50 ml) was added, followed by extraction with ethyl acetate (50 ml) and washing with saturated brine (50 ml). The aqueous layer was extracted with ethyl acetate (50 ml) and the organic layers were combined, dried with Na₂SO₄ and separated by filtration; the filtrate was then concentrated under reduced pressure. The resulting residue was purified by silica gel column chromatography (SNAP Cartridge HP-Sphere; mobile phase: chloroform/methanol =100/0-95/5; v/v) to give the titled compound (1.12 g as pale brown solids).
MS (ESI pos.) m/z: 434([M+H]⁺)

### •Reference Example P-A34: Synthesis of tert-butyl [5-bromo-2-(3-chloro-4-fluorophenyl)-1H-benzimidazol-1-yl]acetate

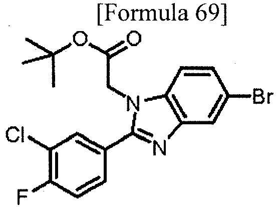

The compound (5.00 g) obtained in Reference Example P-A30 was treated by the same procedure as in Reference Example P-A5, yielding the titled compound (3.38 g as yellow solids).
MS (ESI pos.) m/z: 439([M+H]⁺)

### •Reference Example P-A35: Synthesis of [5-bromo-2-(3-chloro-4-fluorophenyl)-1H-benzimidazol-1-yl]acetic acid

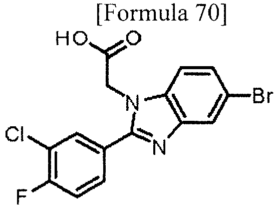

The compound (2.90 g) obtained in Reference Example P-A30 was treated by the same procedure as in Reference Example P-A32, yielding the titled compound (3.17 g as pale yellow solids).
MS (ESI pos.) m/z: 383([M+H]⁺)

### •Reference Example P-A36: Synthesis of 2-[5-bromo-2-(3-chloro-4-fluorophenyl)-1H-benzimidazol-1-yl]-N-tert-butyl acetamide

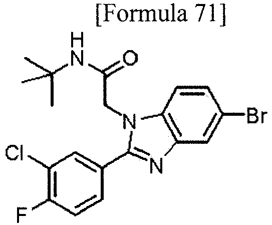

The compound (3.00 g) obtained in Reference Example P-A35 was treated by the same procedure as in Reference Example P-A33, yielding the titled compound (2.52 g as colorless solids).
MS (ESI pos.) m/z: 438([M+H]⁺)

### •Reference Example P-B1: Synthesis of 2-(3-chlorophenyl)-5-methoxy-1H-indole

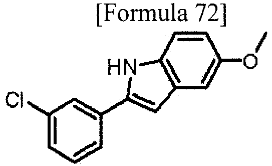

### Step P-B1-1

To a THF solution (50 mL) of diisopropylamine (9.02 g), a n-butyl lithium/n-Hexane solution (2.73 M, 32.7 mL) was added dropwise over 5 minutes in a nitrogen stream under cooling with ice. The mixture was stirred for an hour under the same conditions. A separate reactor containing a THF solution (50 mL) of N-Boc-5-methoxyindole (17.5 g) and triisopropyl borate (20.23 g) was charged with the previously prepared lithium diisopropylamide solution which was added dropwise over 5 minutes in a nitrogen stream under cooling with ice. The mixture was stirred for an hour under the same conditions. To the reaction mixture, 2 M hydrochloric acid was added until pH = 5; thereafter, EtOAc (100 mL) was added and phase separation was effected, followed by extraction of the aqueous layer with EtOAc (30 mL × 2). The combined organic layers were dried with MgSO₄ and, thereafter, the desiccant was separated by filtration and the filtrate was concentrated under reduced pressure to give a crude product (11.27 g as pale yellow solids).

### Step P-B 1-2

To an EtOH/Toluene (1/3; v/v) solution (160 mL) of the obtained crude product (4.15 g), 1-chloro-3-iodobenzene (4.08 g) and Pd(PPh₃)₄ (1.65 g), an aqueous solution of Na₂CO₃ (2 M, 40 mL) was added and the mixture was stirred at 100°C for an hour. To the reaction mixture, water was added and extraction was conducted with CHCl₃. After drying the organic layer with MgSO₄, the desiccant was separated by filtration and the filtrate was concentrated under reduced pressure to give a crude product (7.60 g as brown oil).

### Step P-B 1-3

To a CHCl₃ solution (100 mL) of the obtained crude product (5.10 g), trifluoroacetic acid (40.0 mL) was added and the mixture was stirred at room temperature for 65 hours. The reaction mixture was concentrated under reduced pressure and the residue was dissolved in EtOAc and washed with a saturated aqueous solution of NaHCO₃, water, and Brine. After drying the organic layer with MgSO₄, the desiccant was separated by filtration and the filtrate was concentrated under reduced pressure. The resulting residue was purified by silica gel column chromatography (Silica Gel 60; n-hexane/EtOAc = 90/10-75/25; v/v) to give the titled compound (2.67 g as pale yellow solids).
MS (ESI pos.) m/z: 258([M+H]⁺).

### •Reference Example P-B2: Synthesis of 2-[2-(3-chlorophenyl)-5-methoxy-1H-indol-1-yl]-N-isopropyl acetamide

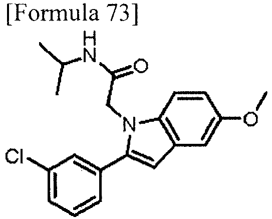

To a DMF solution (40.0mL) of the compound (2.67 g) obtained in Reference Example P-B1, 60% sodium hydride (497.3 mg) was added and the mixture was stirred at room temperature for an hour. To the reaction mixture, 2-bromo-N-isopropyl acetamide (2.80 g) was added and the mixture was stirred at room temperature for 17 hours. To the reaction mixture, water was added and after extraction with EtOAc, the organic layer was washed with Brine. After drying the organic layer with MgSO₄, the desiccant was separated by filtration and the filtrate was concentrated under reduced pressure. The resulting residue was purified by neutral OH-form silica gel column chromatography (CHCl₃/MeOH = 99/1-95/5; v/v) to give the titled compound (2.18 g as pale yellow solids).
MS (ESI pos.) m/z: 357([M+H]⁺)

### •Reference Example P-B3: Synthesis of 2-[2-(3-chlorophenyl)-5-hydroxy-1H-indol-1-yl]-N-isopropyl acetamide

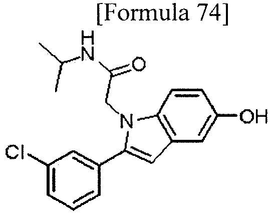

To a CHCl₃ solution (40.0 mL) of the compound (2.18 g) obtained in Reference Example P-B2, BBr₃/n-Hexane (1 M, 30.6 mL) was added under cooling with ice and the mixture was reverted to room temperature before it was stirred for 17 hours. The mixture was rendered basic by adding a saturated aqueous solution of NaHCO₃ under cooling with ice and then the organic layer was washed and phase separation was effected. Further, the organic layer was washed with water and Brine and then dried with MgSO₄; thereafter, the desiccant was separated by filtration and the filtrate was concentrated under reduced pressure. The resulting residue was purified by neutral OH-form silica gel column chromatography (SNAP Cartridge KP-Sil; mobile phase: CHCl₃/MeOH = 99/1-95/5; v/v). The resulting solids were washed with IPE in suspension to give the titled compound (1.91 g as pale yellow solids).
MS (ESI pos.) m/z: 343([M+H]⁺).

### •Reference Example P-B4: Synthesis of 2-[2-(3-chlorophenyl)-5-(3-chloropropoxy)-1H-indol-1-yl]-N-isopropyl acetamide

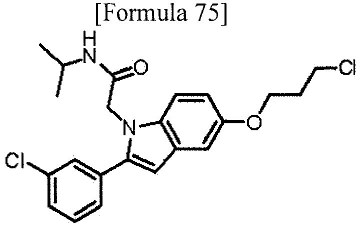

A DMF suspension (20.0 mL) of the compound (1.00 g) obtained in Reference Example P-B3, 1-bromo-3-chloropropane (0.867 mL) and K₂CO₃ (2.02 g) was stirred at 60°C for 6 hours. The reaction mixture was diluted with EtOAc and washed with water and Brine. After the organic layer was dried with MgSO₄, the desiccant was separated by filtration and the filtrate was concentrated under reduced pressure. The resulting residue was purified by neutral OH-form silica gel column chromatography (SNAP Cartridge KP-Sil; mobile phase: CHCl₃/EtOAc = 90/10; v/v) to give the titled compound (977 mg as pale yellow powder).
MS (ESI pos.) m/z: 441([M+Na]⁺)

### •Reference Example P-B5: Synthesis of 2-(3-chloro-4-fluorophenyl)-5-methoxy-1H-indole

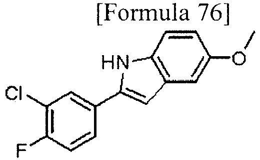

The crude compound (5.00 g) obtained in Step P-B1-1 and 4-bromo-2-chloro-1-fluorobenzene (4.32 g) were treated by the same procedure as in Reference Example P-B1, yielding the titled compound (3.57 g as colorless solids).
MS (ESI neg.) m/z: 274([M-H]⁻)

### •Reference Example P-B6: Synthesis of 2-[2-(3-chloro-4-fluorophenyl)-5-methoxy-1H-indol-1-yl]-N-isopropyl acetamide

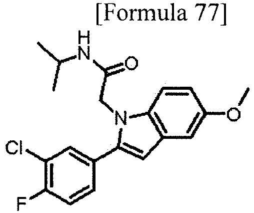

The compound (3.55 g) obtained in Reference Example P-B5 was treated by the same procedure as in Reference Example P-B2, yielding the titled compound (4.25 g as pale yellow solids).
MS (ESI pos.) m/z: 375([M+H]⁺)

### •Reference Example P-B7: Synthesis of 2-[2-(3-chloro-4-fluorophenyl)-5-hydroxy-1H-indol-1-yl]-N-isopropyl acetamide

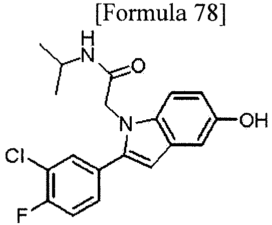

The compound (4.20 g) obtained in Reference Example P-B6 was treated by the same procedure as in Reference Example P-B3, yielding the titled compound (4.55 g as green solids).
MS (ESI pos.) m/z: 361([M+H]⁺)

### •Reference Example P-B8: Synthesis of 2-[2-(3-chloro-4-fluorophenyl)-5-(3-chloropropoxy)-1H-indol-1-yl]-N-isopropyl acetamide

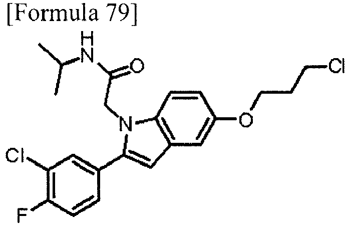

The compound (2.00 g) obtained in Reference Example P-B7 was treated by the same procedure as in Reference Example P-B4, yielding the titled compound (1.29 g as pale brown solids).
MS (ESI neg.) m/z: 435([M-H]⁻)

### •Reference Example P-B9: Synthesis of 5-(benzyloxy)-2-(4-fluoro-3-methoxyphenyl)-1H-indole

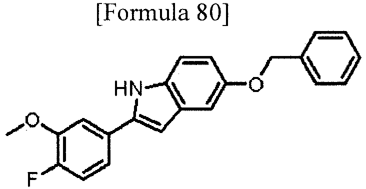

The compound tert-butyl ester of 5-benzyloxy-indole-1-carboxylic acid (6.23 g) and 5-bromo-2-fluoroanisole (2.01 g) were treated by the same procedure as in Reference Example P-B1, yielding the titled compound (1.25 g as pale yellow powder).
MS (ESI pos.) m/z: 348([M+H]⁺)

### •Reference Example P-B10: Synthesis of 2-[5-(benzyloxy)-2-(4-fluoro-3-methoxyphenyl)-1H-indol-1-yl]-N-isopropyl acetamide

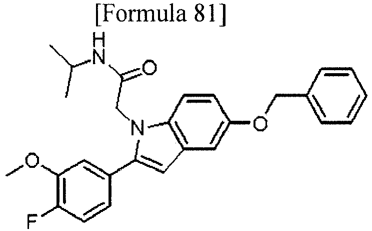

The compound (1.25 g) obtained in Reference Example P-B9 was treated by the same procedure as in Reference Example P-B2, yielding the titled compound (1.01 g as pale yellow solids).
MS (ESI pos.) m/z: 447([M+H]⁺)

### •Reference Example P-B11: Synthesis of 2-[2-(4-fluoro-3-methoxyphenyl)-5-hydroxy-1H-indol-1-yl]-N-isopropyl acetamide

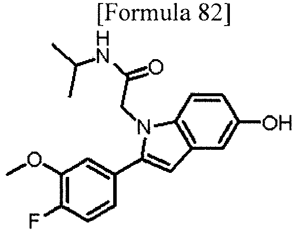

To a THF solution (23.0 mL) of the compound (1.01 g) obtained in Reference Example P-B10, palladium hydroxide (100 mg) was added and the mixture was stirred at room temperature for 2 hours in a hydrogen atmosphere. The reaction mixture was filtered through Celite (registered trademark) and the filtrate was concentrated under reduced pressure to give the titled compound (867 mg as brown solids).
MS (ESI pos.) m/z: 357([M+H]⁺)

### •Reference Example P-B12: Synthesis of 2-[5-(3-chloropropoxy)-2-(4-fluoro-3-methoxyphenyl)-1H-indol-1-yl]-N-isopropyl acetamide

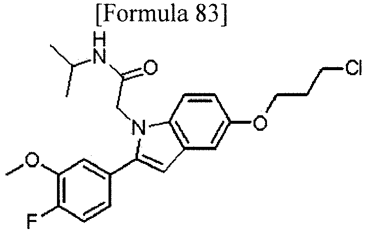

The compound (500 mg) obtained in Reference Example P-B11 was treated by the same procedure as in Reference Example P-B4, yielding the titled compound (444 mg as pale yellow powder).
MS (ESI pos.) m/z: 433([M+H]⁺)

### •Reference Example P-B13: Synthesis of tert-butyl 4-({2-(3-chloro-4-fluorophenyl)-1-[2-(isopropylamino)-2-oxoethyl]-1H-indol-5-yl}oxy)piperidine-1-carboxylate

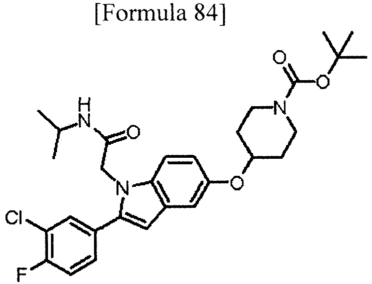

A MeCN suspension (20.0 mL) of the compound (1.00 g) obtained in Reference Example P-B7, 1-Boc-4-methanesulfonyloxypiperidine (1.55 g) and Cs₂CO₃ (1.81 g) was stirred under heating at 100°C for 8 hours in a nitrogen stream and then left to cool. To the reaction mixture were added CHCl₃ (50 mL) and water (50 mL) and phase separation was effected, followed by extraction of the aqueous layer with CHCl₃. The combined organic layers were washed with Brine and dried with MgSO₄; thereafter, the desiccant was separated by filtration and the filtrate was concentrated under reduced pressure to give the titled compound (1.45 g as pale brown solids).
MS (ESI pos.) m/z: 566([M+Na]⁺)

### •Reference Example P-B 14: Synthesis of 2-[2-(3-chloro-4-fluorophenyl)-5-(piperidin-4-yloxy)-1H-indol-1-yl]-N-isopropyl acetamide

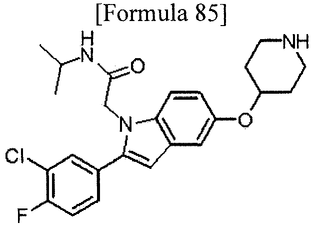

To a solution in CHCl₃ (28.0 mL) of the compound (1.40 g) obtained in Reference Example P-B7, trifluoroacetic acid (14.0 mL) was added in a nitrogen stream and the mixture was stirred overnight at room temperature. The reaction mixture was concentrated under reduced pressure and to the resulting residue, a HCl/IPA solution (2 M, 15 mL) was added, followed by stirring at room temperature for 4 hours. The reaction mixture was concentrated under reduced pressure and to the residue, CHCl₃ (100 mL) and a saturated aqueous solution of NaHCO₃ (150 mL) were added and phase separation was effected, followed by extracting the aqueous layer with CHCl₃. The organic layers were combined and dried with anhydrous magnesium sulfate; thereafter, the desiccant was separated by filtration and the filtrate was concentrated under reduced pressure. The resulting residue was purified by silica gel column chromatography (Silica Gel 60; mobile phase: CHCl₃/MeOH/NH₄OH = 98/2/0.2-90/10/1; v/v/v) to give the titled compound (184 mg as brown amorphous mass).
MS (ESI pos.) m/z: 444([M+H]⁺)

### •Reference Example P-B15: Synthesis of 2-(3-chlorophenyl)-1-[2-(isopropylamino)-2-oxoethyl]-1H-indol-5-yl trifluoromethanesulfonate

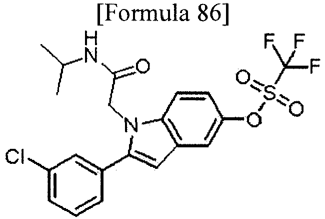

A mixture of the compound (500 mg) obtained in Reference Example P-B3, N-phenylbis(trifluoromethanesulfonimide) (625 mg), Et₃N (177 mg) and CHCl₃ (5.0 mL) was stirred at room temperature for 3 days. After adding water, the mixture was extracted with CHCl₃ and the organic layer was washed with Brine. After drying the organic layer with MgSO₄, the desiccant was separated by filtration and the filtrate was concentrated under reduced pressure. The resulting residue was purified by silica gel column chromatography (SNAP Cartridge KP-Sil, 50 g) to give the titled compound (774 mg as colorless solids).
¹H-NMR (600 MHz, CDCl₃) δ (ppm); 1.08 (6 H, d, J=6.9 Hz), 4.10 - 4.19 (1 H, m), 4.69 (2 H, s), 5.26 (1 H, d, J=8.3 Hz), 6.73 (1 H, s), 7.21 (1 H, dd, J=8.7, 2.3 Hz), 7.28 - 7.34 (3 H, m), 7.44 - 7.47 (2 H, m), 7.60 (1 H, d, J=2.3 Hz).

### •Reference Example P-B16: Synthesis of 2-[2-(3-chlorophenyl)-5-(4-hydroxybut-1-yn-1-yl)-1H-indol-1-yl]-N-isopropyl acetamide

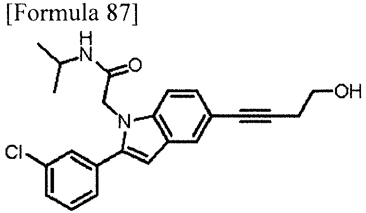

A mixture of the compound (500 mg) obtained in Reference Example P-B15, 3-butyn-1-ol (0.10 mL), PdCl₂(PPh₃)₂ (37 mg), CuI (20 mg), Et₃N (1.75 mL) and DMF (9.2 mL) was stirred at room temperature for 2 days in a nitrogen atmosphere. After further adding 3-butyn-1-ol (0.10 mL) and PdCl₂(PPh₃)₂ (37 mg), the mixture was stirred at room temperature for a day. After adding water and EtOAc to the reaction mixture for effecting phase separation, washing was conducted with Brine. After drying the organic layer with Na₂SO₄, the desiccant was separated by filtration and the filtrate was concentrated under reduced pressure. The resulting residue was purified by silica gel column chromatography (SNAP Cartridge KP-Sil, 50 g) to give the titled compound (321 mg as colorless solids). ¹H-NMR (600 MHz, CDCl₃) δ (ppm); 1.04 - 1.08 (6 H, m), 1.68 - 1.74 (2 H, m), 2.74 - 2.81 (2 H, m), 4.12 - 4.19 (1 H, m), 4.66 (2 H, s), 5.40 - 5.46 (1 H, m), 6.64 (1 H, s), 7.13 (1 H, d, J=1.4 Hz), 7.19 (1 H, d, J=8.3 Hz), 7.30 (1 H, s), 7.38 - 7.41 (2 H, m), 7.46 (2 H, d, J=10.1 Hz).

### •Reference Example P-B17: Synthesis of 4-{2-(3-chlorophenyl)-1-[2-(isopropylamino)-2-oxoethyl]-1H-indol-5-yl}butyl methanesulfonate

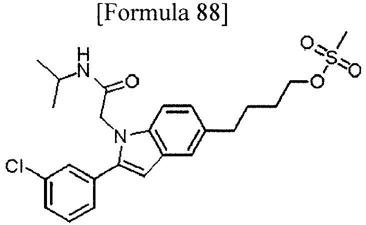

A mixture of the compound (300 mg) obtained in Reference Example P-B16, palladium/carbon (5%) (300 mg) and EtOH (3.0 mL) was purged with hydrogen and then stirred overnight at room temperature in a hydrogen atmosphere. The reaction mixture was filtered through Celite (registered trademark) and the filtrate was concentrated under reduced pressure.

To a CHCl₃ suspension (5.0 mL) of the resulting residue, Et₃N (0.212 mL) and MsCl (0.088 mL) were added at 0°C and the mixture was immediately subjected to stirring that continued overnight. To the reaction mixture, water was added and phase separation was effected; subsequently, the organic layer was washed with a saturated aqueous solution of NaHCO₃ and Brine and dried with Na₂SO₄; thereafter, the desiccant was separated by filtration and the filtrate was concentrated under reduced pressure. The resulting residue was purified by silica gel column chromatography (SNAP Cartridge KP-Sil, 50 g) to give the titled compound (157 mg as pale yellow solids).
MS (ESI pos.) m/z: 477([M+H]⁺)

### •Reference Example P-B18: Synthesis of tert-butyl 5-[3-(piperidin-1-yl)propoxy]-1H-indole-1-carboxylate

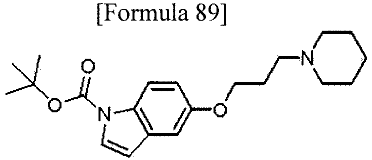

To a mixture of N-Boc-5-hydroxyindole (5.0 g), 1-piperidinepropanol (4.95 mL), Toluene (100 mL) and THF (25 mL), cyanomethylenetributylphosphorane (7.3 mL) was added and the mixture was stirred first at 85°C for 3 hours, then at 100°C for 3 hours, and finally at room temperature overnight. The reaction mixture was concentrated under reduced pressure and the resulting residue was purified by silica gel column chromatography (SNAP Cartridge KP-Sil, 100 g) to give the titled compound (3.97 g).
MS (ESI pos.) m/z: 359([M+H]⁺)

### •Reference Example P-B 19: Synthesis of 2-(6-methoxypyridin-2-yl)-5-[3-(piperidin-1-yl)propoxy]-1H-indole

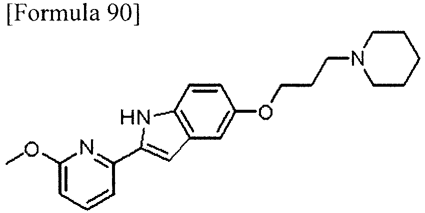

The compound (2.50 g) obtained in Reference Example P-B18 was treated by the same procedure as in Reference Example P-B 1, yielding the titled compound (90 mg as pale brown amorphous mass).
MS (ESI pos.) m/z: 366([M+H]⁺)

### •Reference Example P-B20: Synthesis of 2-(3-methoxyphenyl)-5-[3-(piperidin-1-yl)propoxy]-1H-indole

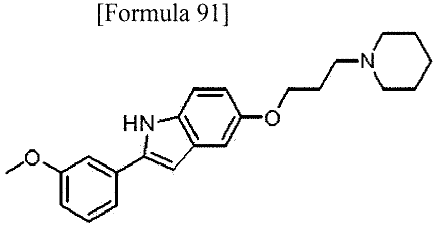

The compound (2.50 g) obtained in Reference Example P-B18 was treated by the same procedure as in Reference Example P-B1, yielding the titled compound (64 mg as pale brown amorphous mass).
MS (ESI pos.) m/z: 365([M+H]⁺)

### •Reference Example P-B21: Synthesis of 5-bromo-3-[(3-chlorophenyl)ethynyl]pyridine-2-amine

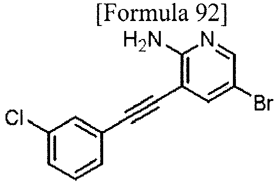

The compound 2-amino-3,5-dibromopyridine (2.00 g), 1-chloro-3-ethynylbenzene (0.986 mL), bis(triphenylphosphine)palladium(II) dichloride (0.281 g), copper(I) iodide (0.152 g) and triethylamine (5.55 mL) were placed in DMF (40 mL) and the mixture was stirred at an external temperature of 110°C for 4 hours. After being left to cool, the reaction mixture was diluted with ethyl acetate (150 mL) and washed with water (150mL) and saturated brine (150 mL) consecutively. The aqueous layer was extracted with EtOAc (150 mL) twice and the combined organic layers were dried with Na₂SO₄; thereafter, the desiccant was concentrated under reduced pressure. The resulting residue was purified by silica gel column chromatography (SNAP Cartridge KP-NH; mobile phase: n-hexane/ethyl acetate = 100/0-33/64; v/v) to give the titled compound (1.27 g as pale brown solids).
MS (ESI pos.) m/z: 307([M+H]⁺)

### •Reference Example P-B22: Synthesis of 5-bromo-2-(3-chlorophenyl)-1H-pyrrolo[2,3-b] pyridine

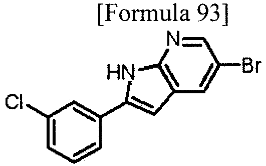

The compound (0.550 g) obtained in Reference Example P-B21 and KOtBu (0.421 g) were placed in a mixed solvent of THF (11 mL) and DMF (5.5 mL) and the resulting mixture was stirred at an external temperature of 85°C for 20 hours. After being left to cool, the reaction mixture was diluted with ethyl acetate (50 mL) and washed with saturated aqueous ammonium chloride (50 mL) and saturated brine (50 mL) consecutively. After drying the organic layer with Na₂SO₄, the desiccant was separated by filtration and the filtrate was concentrated under reduced pressure. The resulting residue was purified by silica gel column chromatography (SNAP Cartridge HP-Sil; mobile phase: n-hexane/ethyl acetate = 88/12-0/100; v/v) and the resulting roughly purified product was washed with IPE under stirring, recovered by filtration and dried to give the titled compound (0.279 g as brown solids).
MS (ESI pos.) m/z: 307([M+H]⁺)

### •Reference Example P-B23: Synthesis of 2-[5-bromo-2-(3-chlorophenyl)-1H-pyrrolo[2,3-b]pyridin-1-yl]-N-isopropyl acetamide

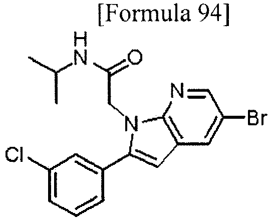

The compound (0.278 g) obtained in Reference Example P-B22, 2-bromo-N-isopropyl acetamide (0.244 g) and potassium carbonate (0.375 g) were placed in DMF (5.56 mL) and the mixture was stirred at an external temperature of 100°C for 2 hours. Further, 2-bromo-N-isopropyl acetamide (0.08 g) was added and the mixture was stirred at an external temperature of 100°C for 30 minutes. After being left to cool, the reaction mixture was diluted with ethyl acetate (50 mL) and washed with saturated aqueous ammonium chloride (50 mL) and saturated brine (50 mL) consecutively. The organic layer was dried with Na₂SO₄ and, thereafter, the desiccant was separated by filtration and the filtrate was concentrated under reduced pressure. The resulting residue was purified by silica gel column chromatography (SNAP Cartridge HP-Sil; mobile phase: n-hexane/ethyl acetate = 88/12-0/100; v/v) to give the titled compound (94 mg as brown solids).
MS (ESI pos.) m/z: 408([M+H]⁺)

### •Reference Example P-B24: Synthesis of 2-[2-(3-chlorophenyl)-5-(4,4,5,5-tetramethyl-1,3,2-dioxaborolan-2-yl)-1H-pyrrolo[2,3-b]pyridin-1-yl]-N-isopropyl acetamide

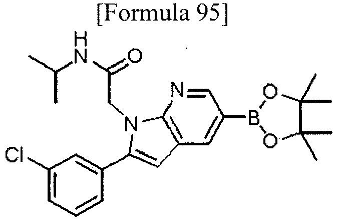

The compound (94 mg) obtained in Reference Example P-B23 was treated by the same procedure as in Reference Example P-A6, yielding the titled compound (275 mg as brown solids).
MS (ESI pos.) m/z: 454([M+H]⁺)

### •Reference Example P-B25: Synthesis of 2-[2-(3-chlorophenyl)-5-hydroxy-1H-pyrrolo[2,3-b]pyridin-1-yl]-N-isopropyl acetamide

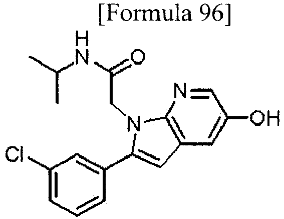

The compound (275 mg) obtained in Reference Example P-B24 was treated by the same procedure as in Reference Example P-A7, yielding the titled compound (53 mg as brown solids).
MS (ESI pos.) m/z: 344([M+H]⁺)

### •Reference Example P-B26: Synthesis of 6-bromo-2-[(3-chlorophenyl)ethynyl]pyridin-3-amine

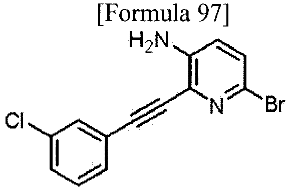

The compound 2,6-dibromopyridine-3-amine (2.17 g) was treated by the same procedure as in Reference Example P-B21, yielding the titled compound (890 mg as brownish red oil).
MS (ESI pos.) m/z: 307([M+H]⁺)

### •Reference Example P-B27: Synthesis of 5-bromo-2-(3-chlorophenyl)-1H-pyrrolo[3,2-b]pyridine

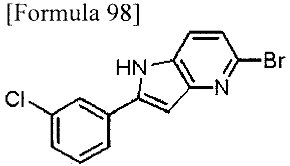

The compound (890 mg) obtained in Reference Example P-B26 was treated by the same procedure as in Reference Example P-B22, yielding the titled compound (326 mg as brownish red solids).
MS (ESI pos.) m/z: 307([M+H]⁺)

### •Reference Example P-B28: Synthesis of 2-[5-bromo-2-(3-chlorophenyl)-1H-pyrrolo[3,2-b]pyridin-1-yl]-N-isopropyl acetamide

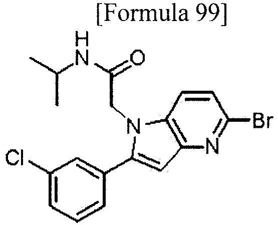

The compound (0.233 g) obtained in Reference Example P-B27 was dissolved in DMF (4.0 mL) and after adding sodium hydride (33 mg) under cooling with ice, the mixture was stirred at room temperature for 30 minutes. To the reaction mixture, 2-bromo-N-isopropyl acetamide (150 mg) was added and the mixture was stirred at room temperature for 16 hours. To the reaction mixture, water (20 mL) and ethyl acetate (20 mL) were added and after phase separation, the organic layer was washed with saturated brine (20 mL). After drying the organic layer with Na₂SO₄, the desiccant was separated by filtration and the filtrate was concentrated under reduced pressure. The resulting residue was purified by silica gel column chromatography (SNAP Cartridge KP-NH; mobile phase: n-hexane/chloroform = 20/80-0/100; v/v) to give the titled compound (172 mg as pale brown solids).
MS (ESI pos.) m/z: 406([M+H]⁺)

### •Reference Example P-C1: Synthesis of 4-(3-chloro-4-fluorophenyl)-N-isopropyl-4-oxobutaneamide

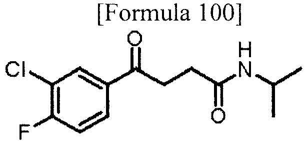

To a DMF solution (10 mL) of 4-(3-chloro-4-fluorophenyl)-4-oxobutyric acid (1.35 g), HOBt·H₂O (1.35 g), EDC·HCl (1.35 g) and isopropylamine (0.6 mL) were added under cooling with ice and the mixture was stirred at room temperature for 3 days. To the reaction mixture, water was added under cooling with ice and the mixture was stirred; the precipitating solids were recovered by filtration to give the titled compound (1.63 g as pale yellow solids).
MS (ESI pos.) m/z: 272([M+H]⁺)

### •Reference Example P-C2: Synthesis of 3-bromo-4-(3-chloro-4-fluorophenyl)-N-isopropyl-4-oxobutaneamide

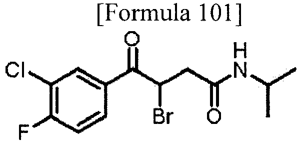

To a carbon tetrachloride suspension (40 mL) of the compound (2.28 g) obtained in Reference Example P-C1, a carbon tetrachloride solution (10 mL) of bromine (0.42 mL) was added dropwise under cooling with ice and the mixture was stirred overnight at room temperature. Water was added to the reaction mixture under cooling with ice and extraction was conducted with CHCl₃; after drying the organic layer with Na₂SO₄, the desiccant was separated by filtration and the filtrate was concentrated under reduced pressure. The resulting residue was washed in suspension with a n-Hexane/EtOAc (6/1; v/v) mixed solvent and the solids were recovered by filtration to give the titled compound (2.56 g as colorless solids).
MS (ESI pos.) m/z: 350, 352([M+H]⁺)

### •Reference Example P-C3: Synthesis of 2-[7-bromo-2-(3-chloro-4-fluorophenyl)imidazo[1,2-a]pyridin-3-yl]-N-isopropyl acetamide

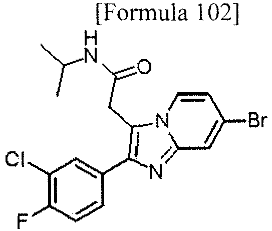

A mixture of the compound (1.5 g) obtained in Reference Example P-C2, 2-amino-4-bromopyridine (740 mg) and MeCN (40 mL) was refluxed at an external temperature of 100°C for 6 hours, then stirred at room temperature for 3 days, and again refluxed at an external temperature of 100°C for 6 hours. After distilling off the solvent under reduced pressure, the resulting residue was washed in suspension with a n-Hexane/EtOAc (4/1; v/v) mixed solvent and the solids were recovered by filtration. The resulting solids were purified by OH silica gel column chromatography (SNAP Cartridge KP-Sil, 50 g; mobile phase: n-Hexane/EtOAc = 75/25-0/100; v/v) to give the titled compound (430 mg as pale yellow solids).
MS (ESI pos.) m/z: 424, 426([M+H]⁺)

### •Reference Example P-C4: Synthesis of 2-[2-(3-chloro-4-fluorophenyl)-7-(4,4,5,5-tetramethyl-1,3,2-dioxaborolan-2-yl)imidazo[1,2-a]pyridin-3-yl]-N-isopropyl acetamide

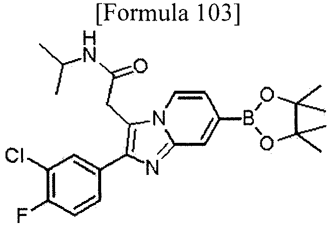

A solution of the compound (150 mg) obtained in Reference Example P-C3, bis(pinacolato)diboron (108 mg), PdCl₂(dppf)·CH₂Cl₂ (30 mg) and potassium acetate (104 mg) in a mixture of 1,4-dioxane (20 mL) and toluene (20 mL) was stirred an external temperature of 100°C for 5 hours in a nitrogen atmosphere. After being left to cool, the mixture was concentrated under reduced pressure; to the resulting residue, n-Hexane (containing 25% IPE) and water were added and the mixture was stirred at room temperature; the precipitating solids were recovered by filtration to give the titled compound (155 mg as gray solids).
MS (ESI pos.) m/z: 472([M+H]⁺)

### •Reference Example P-C5: Synthesis of 2-[2-(3-chloro-4-fluorophenyl)-7-hydroxyimidazo[1,2-a]pyridin-3-yl]-N-isopropyl acetamide

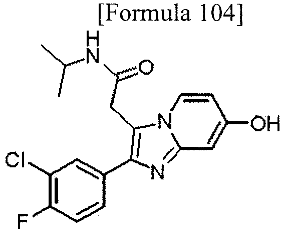

To an EtOH/THF (1/1; v/v) solution (7.0 mL) of the compound (385 mg) obtained in Reference Example P-C4, an aqueous solution (3.5 mL) of NaHCO₃ (554 mg) was added under cooling with ice and, thereafter, a 30% aqueous H₂O₂ solution (0.3 mL) was added dropwise and the mixture was stirred for 3 hours. To the reaction mixture, Na₂SO₃ (350 mg) in aqueous solution (3.5 mL) and water (7.0 mL) were added consecutively under cooling with ice and the mixture was stirred; the precipitating solids were recovered by filtration to give the titled compound (310 mg as gray solids).
MS (ESI pos.) m/z: 362([M+H]⁺)

### •Reference Example P-D1: Synthesis of 2-bromo-N'-(3-chlorophenyl)-5-methoxybenzohydrazide

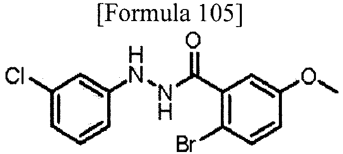

The compound 2-bromo-5-methoxybenzoic acid (6.45 g) and DMF (0.429 mL) were placed in chloroform (77 mL) and after adding oxalyl chloride (4.79 mL), the mixture was stirred at room temperature for 2 hours and the reaction mixture was concentrated under reduced pressure. To the resulting residue, triethylamine (11.6 mL), (3-chlorophenyl)hydrazine hydrochloride (5.00 g) and THF (64 mL) were added and the mixture was stirred at room temperature for 2 days. The reaction mixture was diluted with chloroform (50 mL) and washed with saturated aqueous sodium hydrogencarbonate (150 mL) and saturated brine (150 mL) consecutively. After drying the organic layer with Na₂SO₄, the desiccant was separated by filtration and the filtrate was concentrated under reduced pressure. The resulting residue was purified by silica gel column chromatography (SNAP Cartridge HP-Sil; mobile phase: chloroform/methanol = 100/0-90/10; v/v). The resulting roughly purified product was washed in suspension with IPE and filtered to give the titled compound (5.19 g as colorless solids).
MS (ESI pos.) m/z: 355([M+H]⁺)

### •Reference Example P-D2: Synthesis of 1-(3-chlorophenyl)-5-methoxy-1,2-dihydro-3H-indazol-3-one

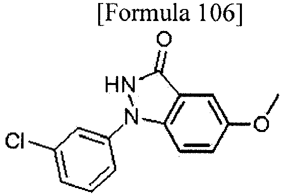

The compound (3.0 g) obtained in Reference Example P-D1, copper iodide(I) (0.161 g), L-proline (0.194 g) and potassium carbonate (2.33 g) were placed in DMSO (84 mL) and the mixture was stirred at room temperature for 16 hours. To the reaction mixture, ethyl acetate (150 mL) was added and the mixture was washed with saturated aqueous ammonium chloride (150 mL) and saturated brine (150 mL) consecutively. After drying the organic layer with Na₂SO₄, the desiccant was separated by filtration and the filtrate was concentrated under reduced pressure. The resulting residue was purified by silica gel column chromatography (SNAP Cartridge HP-Sil; mobile phase: chloroform/methanol = 99/1-92/8; v/v) to give the titled compound (1.41 g).
MS (ESI pos.) m/z: 275([M+H]⁺)

### •Reference Example P-D3: Synthesis of 2-[1-(3-chlorophenyl)-5-methoxy-3-oxo-1,3-dihydro-2H-indazol-2-yl]-N-isopropyl acetamide

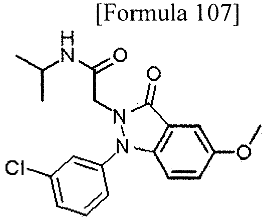

The compound (500 mg) obtained in Reference Example P-D2 was treated by the same procedure as in Reference Example P-B28, yielding the titled compound (139 mg as brown amorphous mass).
MS (ESI pos.) m/z: 374([M+H]⁺)

### •Reference Example P-D4: Synthesis of 2-[1-(3-chlorophenyl)-5-hydroxy-3-oxo-1,3-dihydro-2H-indazol-2-yl]-N-isopropyl acetamide

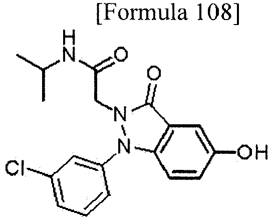

The compound (0.127 g) obtained in Reference Example P-D3 was dissolved in chloroform (2.5 mL) and after adding a BBr₃/hexane solution (1.0 mol/L, 1.0 mL) under cooling with ice, the mixture was stirred at room temperature for 2 hours. After ice-cooling the reaction mixture, water (20 mL) was added and extraction was conducted with chloroform (20 mL). The organic layer was washed with saturated brine and after drying the organic layer with Na₂SO₄, the desiccant was separated by filtration and the filtrate was concentrated under reduced pressure. The resulting residue was purified by silica gel column chromatography (SNAP Cartridge KP-Sil; mobile phase: chloroform/methanol = 99/1-80/20; v/v) to give the titled compound (78.9 mg as brown amorphous mass).
MS (ESI pos.) m/z: 360([M+H]⁺)

### •Reference Example P-E1: Synthesis of tert-butyl 3-[2-(3-chlorophenyl)-2-oxoethyl]-4-oxopiperidine-1-carboxylate

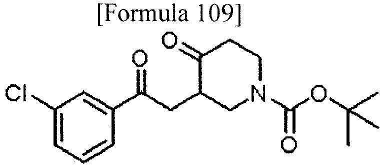

To a toluene solution (43.5 mL) of 1-Boc-4-piperidone (4.37 g), pyrrolidine (1.8 mL) was added and reaction was conducted under microwave irradiation (150°C x 1 hr). The reaction mixture was concentrated under reduced pressure and to a THF solution (90 mL) of the resulting residue, 2-bromo-3-chloroacetophenone (5.1 g) and iPr₂NEt (7.5 mL) were added and the mixture was stirred at an external temperature of 65°C for two days. The reaction mixture was concentrated under reduced pressure and the resulting residue was purified by silica gel column chromatography (SNAP Cartridge KP-Sil; mobile phase: n-Hexane/EtOAc = 100/0-60/40; v/v) to give the titled compound (4.67 g as red oil).
¹H-NMR (600 MHz, CDCl₃) δ (ppm); 1.43 - 1.57 (9 H, m), 2.40 - 4.64 (9 H, m), 7.42 (1 H, t, J=7.8 Hz), 7.52 - 7.57 (1 H, m), 7.83 - 7.87 (1 H, m), 7.92 - 7.96 (1 H, m).

### •Reference Example P-E2: Synthesis of tert-butyl 2-(3-chlorophenyl)-1,4,6,7-tetrahydro-5H-pyrrolo[3,2-c]pyridine-5-carboxylate

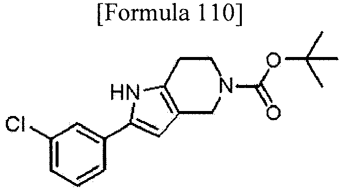

To an EtOH solution (95 mL) of the compound (4.67 g) obtained in Reference Example P-E1, ammonium acetate (5.11 g) was added and the mixture was refluxed for 3 hours. After concentrating the reaction mixture under reduced pressure, CHCl₃ and a saturated aqueous solution of NaHCO₃ were added and extraction was conduced with CHCl₃. The organic layer was concentrated under reduced pressure and the resulting residue was purified by silica gel column chromatography (SNAP Cartridge HP-Sil; mobile phase: n-Hexane/EtOAc = 100/0-75/25; v/v) to give the titled compound (2.56 g as yellow solids).
¹H-NMR (600 MHz, CDCl₃) δ (ppm); 1.50 (9 H, s), 2.69 - 2.77 (2 H, m), 3.69 - 3.81 (2 H, m), 4.44 (2 H, br. s.), 6.31 (1 H, d, J=2.3 Hz), 7.12 - 7.16 (1 H, m), 7.24 - 7.32 (2 H, m), 7.40 (1 H, s), 8.06 - 8.19 (1 H, m).

### •Reference Example P-E3: Synthesis of 2-[2-(3-chlorophenyl)-4,5,6,7-tetrahydro-1H-pyrrolo[3,2-c]pyridin-1-yl]-N-isopropyl acetamide

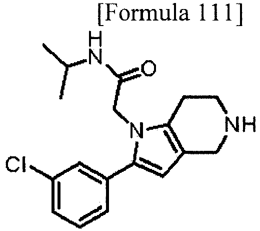

To a DMF solution (51 mL) of the compound (2.56 g) obtained in Reference Example P-E2, 60% sodium hydride (461 mg) and 2-bromo-N-isopropyl acetamide (2.08 g) were added consecutively at 0°C and the mixture was stirred at room temperature for 2 hours. After further adding 60% sodium hydride (307 mg) and 2-bromo-N-isopropyl acetamide (1.39 g), the mixture was stirred overnight at room temperature. To the reaction mixture, MeOH was added and after concentrating the mixture under reduced pressure, water was added and extraction was conducted with CHCl₃; the organic layer was then concentrated under reduced pressure and the resulting residue was purified by silica gel column chromatography (SNAP Cartridge HP-Sil; mobile phase: n-Hexane/EtOAc = 100/0-60/40; v/v) to give a purified product (2.78 g as yellow solids).

To the resulting purified product (2.78 g), a HCl/EtOAc solution (4 M, 28 mL) was added and the mixture was stirred at room temperature for an hour. After concentrating under reduced pressure, water was added and phase separation was conducted with EtOAc. To the resulting aqueous layer, a saturated aqueous solution of NaHCO₃ was added and extraction was conducted with CHCl₃; thereafter, the organic layer was concentrated under reduced pressure to give the titled compound (2.17 g as yellow solids).
¹H-NMR (600 MHz, CDCl₃) δ (ppm); 1.09 (6 H, d, J=6.4 Hz), 2.54 - 2.59 (2 H, m), 3.17 - 3.23 (2 H, m), 3.89 (2 H, s), 4.06 - 4.14 (1 H, m), 4.42 - 4.46 (2 H, m), 5.15 (1 H, d, J=8.3 Hz), 6.12 (1 H, s), 7.13 - 7.18 (1 H, m), 7.28 - 7.33 (3 H, m).

### •Reference Example P-F1: Synthesis of ethyl (7-methoxyimidazo[1,2-a]pyridin-2-yl) acetate

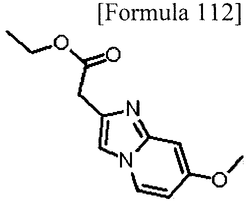

An EtOH solution (60 mL) of 2-amino-4-methoxypyridine (3.00 g) and ethyl 4-chloroacetoacetate (4.93 mL) was stirred at 90°C for 17 hours. The reaction mixture was concentrated under reduced pressure and the resulting residue, after being diluted with EtOAc, was washed with a saturated aqueous solution of NaHCO₃ and Brine. After drying the organic layer with MgSO₄, the desiccant was separated by filtration and the solvents were distilled off under reduced pressure. The resulting residue was purified by silica gel column chromatography (SNAP Cartridge HP-Sil; mobile phase: CHCl₃/MeOH = 99/1-95/5;v/v) to give the titled compound (1.90 g as black oil).
MS (ESI pos.) m/z: 235([M+H]⁺)

### •Reference Example P-F2: Synthesis of N-isopropyl-2-(7-methoxyimidazo[1,2-a]pyridin-2-yl)acetamide

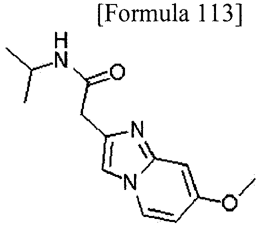

To a CHCl₃ solution (40 mL) of isopropylamine (4.79 g), dimethylaluminum chloride/n-Hexane (0.95 M, 66.2 mL) was added dropwise under cooling with ice and the mixture was stirred for 30 minutes. To the reaction mixture, a CHCl₃ solution (60 mL) of the compound (1.90 g) obtained in Reference Example P-F1 was added dropwise and the mixture was stirred for 17 hours. To the reaction mixture, a saturated aqueous solution of NaHCO₃ was added dropwise under cooling with ice and after stirring for an hour, the mixture was filtered through Celite (registered trademark). The filtrate was washed with water and Brine and dried with MgSO₄; thereafter, the desiccant was separated by filtration and the solvents were distilled off under reduced pressure. The resulting residue was purified by silica gel column chromatography (SNAP Cartridge HP-Sil; mobile phase: CHCl₃/MeOH = 99/1-95/5; v/v) to give the titled compound (1.19 g as brown solids).
MS (ESI pos.) m/z: 248([M+H]⁺)

### •Reference Example P-F3: Synthesis of 2-(3-bromo-7-methoxyimidazo[1,2-a]pyridin-2-yl)-N-isopropyl acetamide

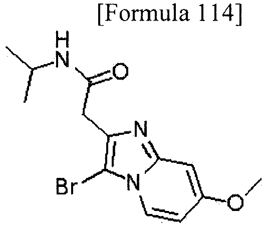

To a DMF solution (12 mL) of the compound (1.19 g) obtained in Reference Example P-F2, N-bromosuccinimide (942 mg) was added under cooling with ice and the mixture was stirred for an hour as the temperature rose to room temperature. The reaction mixture was diluted with EtOAc and washed with water and Brine; after drying the organic layer with MgSO₄, the desiccant was separated by filtration and the solvents were distilled off under reduced pressure. The resulting residue was purified by silica gel column chromatography (SNAP Cartridge HP-Sil; mobile phase: CHCl₃/MeOH = 99/1-95/5; v/v) to give the titled compound (1.10 g as pale yellow solids).
MS (ESI pos.) m/z: 326([M+H]⁺)

### •Reference Example P-F4: Synthesis of 2-[3-(3-chlorophenyl)-7-methoxyimidazo[1,2-a]pyridin-2-yl]-N-isopropyl acetamide

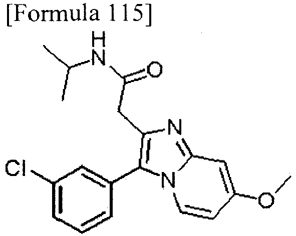

To dimethoxyethane solution (3.30 mL) of the compound (322 mg) obtained in Reference Example P-F3 and 3-chlorophenyboronic acid (201 mg), Pd(PPh₃)₄ (57 mg) and Na₂CO₃ in aqueous solution (2 M, 1.38 mL) were added and the mixture was stirred at 90°C for 4 hours. The reaction mixture was diluted with ethyl acetate and washed with water and Brine. After drying the organic layer with MgSO₄, the desiccant was separated by filtration, the solvents were distilled off under reduced pressure, and the resulting residue was purified by OH silica gel column chromatography (SNAP Cartridge HP-Sil; mobile phase: CHCl₃/MeOH = 99/1-95/5; v/v) and NH silica gel column chromatography (SNAP Cartridge KP-NH; mobile phase: CHCl₃/EtOAc = 80/20-50/50; v/v) to give the titled compound (247 mg as pale yellow oil).
MS (ESI pos.) m/z: 358([M+H]⁺)

### •Reference Example P-F5: Synthesis of 2-[3-(3-chlorophenyl)-7-hydroxyimidazo[1,2-a]pyridin-2-yl]-N-isopropyl acetamide

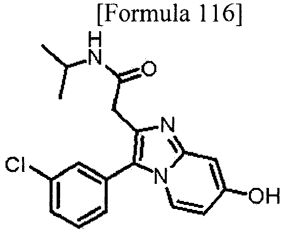

To a DMF solution (3.5 mL) of the compound (247 mg) obtained in Reference Example P-F4, sodium thiomethoxide (194 mg) was added and the mixture was stirred at 120°C for 2 hours. The reaction mixture was diluted with EtOAc and washed with water and Brine; after drying the organic layer with MgSO₄, the desiccant was separated by filtration and the solvents were distilled off under reduced pressure. The resulting residue was washed in suspension with n-Hexane/EtOAc (9/1; v/v) and the solids were recovered by filtration to give the titled compound (90 mg as pale yellow solids).
MS (ESI pos.) m/z: 344([M+H]⁺)

### •Example A-1: Synthesis of 2-{2-(3-chlorophenyl)-5-[3-(piperidin-1-yl)propoxy]-1H-benzimidazol-1-yl}-N-isopropyl acetamide

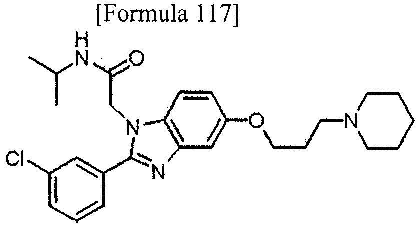

The compound (200 mg) obtained in Reference Example P-A3 was treated by the same procedure as in Reference Example P-B28, yielding the titled compound (40.1 mg as pale brown solids).
MS (ESI pos.) m/z: 469([M+H]⁺)
¹H-NMR (600 MHz, CDCl₃) δ (ppm); 1.10 (6 H, d, J=6.9 Hz), 1.42 - 1.50 (2 H, m), 1.59 - 1.65 (4 H, m), 2.00 - 2.08 (2 H, m), 2.38 - 2.49 (4 H, m), 2.49 - 2.59 (2 H, m), 4.09 (2 H, s), 4.20 (1 H, s), 4.76 (2 H, s), 5.43 - 5.48 (1 H, m), 6.99 - 7.04 (1 H, m), 7.21 (1 H, d, J=9.2 Hz), 7.32 (1 H, d, J=2.3 Hz), 7.46 (1 H, d, J=7.8 Hz), 7.48 - 7.51 (1 H, m), 7.52 - 7.55 (1 H, m), 7.72 - 7.78 (1 H, m).

### •Example A-2: Synthesis of 2-{2-(3-chlorophenyl)-5-[3-(morpholin-4-yl)propoxy]-1H- benzimidazol-1-yl}-N-isopropyl acetamide

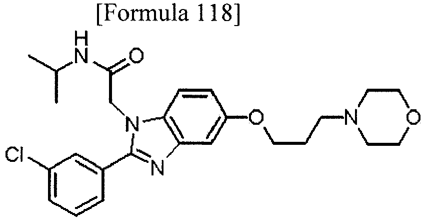

A solution in EtOH (2.0 mL) of the compound (100 mg) obtained in Reference Example P-A8, morpholine (0.62 mL), iPr₂NEt (0.207 mL) and potassium iodide (119 mg) was stirred at an external temperature of 100°C for 10 hours. After leaving the reaction mixture to cool, the solvent was distilled off under reduced pressure. The resulting residue was purified by NH silica gel column chromatography (SNAP Cartridge KP-NH, 28 g; mobile phase: n-Hexane/CHCl₃= 70/30-0/100; v/v) and OH silica gel column chromatography (SNAP Cartridge KP-Sil, 10 g; mobile phase: EtOAc/MeOH = 99/1-85/15; v/v). To the purified product, EtOAc (0.5 mL) and IPE (2.0 mL) were added and the mixture was washed in suspension overnight at room temperature. The solids were recovered by filtration to give the titled compound (59 mg as colorless solids).
MS (ESI pos.) m/z: 471([M+H]⁺)
¹H-NMR (600 MHz, CDCl₃) δ (ppm); 1.11 (6 H, d, J=6.6 Hz), 2.00 - 2.08 (2 H, m), 2.45 - 2.63 (6 H, m), 3.71 - 3.78 (4 H, m), 4.12 (2 H, t, J=6.4 Hz), 4.17 - 4.24 (1 H, m), 4.77 (2 H, s), 5.44 - 5.49 (1 H, m), 7.02 (1 H, dd, J=8.9, 2.3 Hz), 7.22 (1 H, d, J=8.7 Hz), 7.34 (1 H, d, J=2.5 Hz), 7.45 - 7.48 (1 H, m), 7.50 - 7.52 (1 H, m), 7.53 - 7.56 (1 H, m), 7.76 (1 H, t, J=1.9 Hz).

### •Example A-3: Synthesis of 2-{2-(3-chlorophenyl)-5-[3-(2-oxa-6-azaspiro[3.3]hept-6-yl)propoxy]-1H-benzimidazol-1-yl}-N-isopropyl acetamide

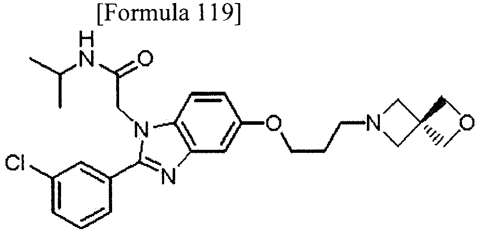

The compound (100 mg) obtained in Reference Example P-A8 and 2-oxa-6-azaspiro[3.3]heptane oxalate (102.9 mg) were treated by the same procedure as in Example A-2, yielding the titled compound (15 mg as colorless amorphous mass).
MS (ESI pos.) m/z: 483([M+H]⁺)
¹H-NMR (600 MHz, CDCl₃) δ (ppm); 1.11 (6 H, d, J=6.6 Hz), 1.83 - 1.90 (2 H, m), 2.60 (2 H, t, J=7.2 Hz), 3.39 (4 H, s), 4.06 (2 H, t, J=6.4 Hz), 4.16 - 4.24 (1 H, m), 4.73 - 4.78 (6 H, m), 5.48 (1 H, d, J=7.8 Hz), 7.00 (1 H, dd, J=8.9, 2.3 Hz), 7.21 (1 H, d, J=8.7 Hz), 7.30 (1 H, d, J=2.5 Hz), 7.45 - 7.49 (1 H, m), 7.49 - 7.52 (1 H, m), 7.52 - 7.56 (1 H, m), 7.74 - 7.76 (1 H, m).

### •Example A-4: Synthesis of 2-[2-(3-chlorophenyl)-5-{3-[(1R,5S)-3-oxa-8-azabicyclo[3.2.1]oct-8-yl]propoxy}-1H-benzimidazol-1-yl]-N-isopropyl acetamide

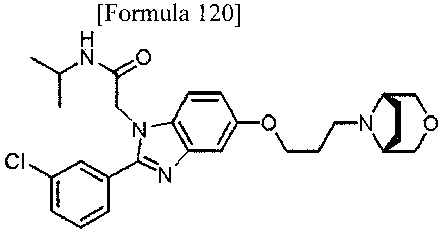

The compound (100 mg) obtained in Reference Example P-A8 and 3-oxa-8-azabicyclo[3.2.1]octane hydrochloride (106.8 mg) were treated by the same procedure as in Example A-2, yielding the titled compound (34 mg as colorless solids).
MS (ESI pos.) m/z: 497([M+H]⁺)
¹H-NMR (600 MHz, CDCl₃) δ (ppm); 1.11 (6 H, d, J=6.6 Hz), 1.83 - 2.06 (6 H, m), 2.45 - 2.55 (2 H, m), 3.04 - 3.13 (2 H, m), 3.53 (2 H, d, J=9.5 Hz), 3.68 - 3.78 (2 H, m), 4.12 - 4.26 (3 H, m), 4.77 (2 H, s), 5.44 - 5.50 (1 H, m), 7.03 (1 H, dd, J=8.7, 2.5 Hz), 7.22 (1 H, d, J=9.1 Hz), 7.35 (1 H, d, J=2.1 Hz), 7.45 - 7.49 (1 H, m), 7.49 - 7.53 (1 H, m), 7.53 - 7.56 (1 H, m), 7.75 - 7.77 (1 H, m).

### •Example A-5: Synthesis of 2-{2-(4-fluoro-3-methoxyphenyl)-5-[3-(piperidin-1-yl)propoxy]-1H-benzimidazol-1-yl}-N-isopropyl acetamide

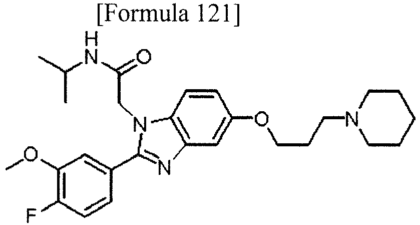

The compound (36.8 mg) obtained in Reference Example P-A13 and piperidine (25.1 µL) were treated by the same procedure as in Example A-2, yielding the titled compound (14.4 mg as pale brown solids).
MS (ESI pos.) m/z: 483([M+H]⁺)
¹H-NMR (600 MHz, DMSO-d₆) δ (ppm); 1.07 (6 H, d, J=6.4 Hz), 1.35 - 1.42 (2 H, m), 1.47 - 1.52 (4 H, m), 1.83 - 1.91 (2 H, m), 2.29 - 2.37 (4 H, m), 2.38 - 2.42 (2 H, m), 3.82 - 3.88 (1 H, m), 3.89 (3 H, s), 4.03 (2 H, t, J=6.4 Hz), 4.81 (2 H, s), 6.91 (1 H, dd, J=8.7, 2.3 Hz), 7.18 - 7.21 (1 H, m), 7.31 - 7.42 (3 H, m), 7.56 - 7.61 (1 H, m), 8.34 (1 H, d, J=7.3 Hz).

### •Example A-6: Synthesis of 2-{2-(4-fluoro-3-methoxyphenyl)-5-[3-(morpholin-4-yl)propoxy]-1H-benzimidazol-1-yl}-N-isopropyl acetamide

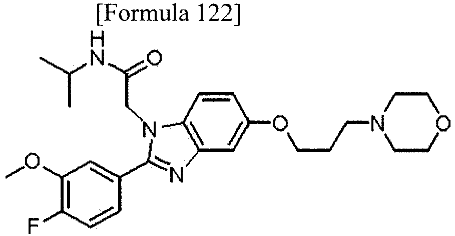

The compound (36.8 mg) obtained in Reference Example P-A13 and morpholine (22.2 µL) were treated by the same procedure as in Example A-2, yielding the titled compound (15.5 mg as pale brown solids).
MS (ESI pos.) m/z: 485([M+H]⁺)
¹H-NMR (600 MHz, DMSO-d₆) δ (ppm); 1.08 (6 H, s), 1.86 - 1.93 (2 H, m), 2.34 - 2.41 (4 H, m), 2.43 - 2.47 (2 H, m), 3.55 - 3.61 (4 H, m), 3.82 - 3.87 (1 H, m), 3.89 (3 H, s), 4.05 (2 H, t, J=6.4 Hz), 4.81 (2 H, s), 6.92 (1 H, dd, J=8.7, 2.3 Hz), 7.21 (1 H, d, J=2.3 Hz), 7.30 - 7.43 (3 H, m), 7.58 (1 H, dd, J=8.3, 1.8 Hz), 8.34 (1 H, d, J=7.3 Hz).

### •Example A-7: Synthesis of 2-{2-(4-fluoro-3-methoxyphenyl)-5-[3-(2-oxa-6-azaspiro[3.3]hept-6-yl)propoxy]-1H-benzimidazol-1-yl}-N-isopropyl acetamide

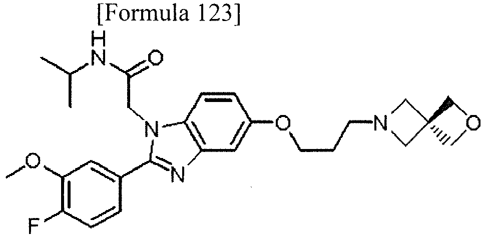

The compound (100 mg) obtained in Reference Example P-A13 and 2-oxa-6-azaspiro[3.3]heptane oxalate (99.7 mg) were treated by the same procedure as in Example A-2, yielding the titled compound (25 mg as colorless amorphous mass).
MS (ESI pos.) m/z: 497([M+H]⁺)
¹H-NMR (600 MHz, CDCl₃) δ (ppm); 1.10 (6 H, d, J=6.6 Hz), 1.80 - 1.91 (2 H, m), 2.55 - 2.62 (2 H, m), 3.38 (4 H, br. s.), 3.96 (3 H, s), 4.06 (2 H, t, J=6.4 Hz), 4.15 - 4.24 (1 H, m), 4.72 - 4.79 (6 H, m), 5.49 (1 H, d, J=7.8 Hz), 7.00 (1 H, dd, J=8.7, 2.1 Hz), 7.14 - 7.17 (1 H, m), 7.19 - 7.24 (2 H, m), 7.32 (1 H, d, J=2.1 Hz), 7.40 (1 H, dd, J=7.8, 2.1 Hz).

### •Example A-8: Synthesis of 2-[2-(4-fluoro-3-methoxyphenyl)-5-{3-[(1R,5S)-3-oxa-8-azabicyclo[3.2.1]oct-8-yl]propoxy}-1H-benzimidazol-1-yl]-N-isopropyl acetamide

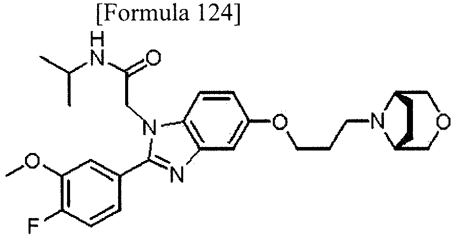

The compound (100 mg) obtained in Reference Example P-A13 and 3-oxa-8-azabicyclo[3.2.1]octane hydrochloride (103.4 mg) were treated by the same procedure as in Example A-2, yielding the titled compound (46 mg as colorless solids).
MS (ESI pos.) m/z: 511([M+H]⁺)
¹H-NMR (600 MHz, CDCl₃) δ (ppm); 1.10 (6 H, d, J=6.6 Hz), 1.85 - 2.04 (6 H, m), 2.46 - 2.54 (2 H, m), 3.04 - 3.11 (2 H, m), 3.50 - 3.56 (2 H, m), 3.68 - 3.76 (2 H, m), 3.96 (3 H, s), 4.14 - 4.24 (3 H, m), 4.77 (2 H, s), 5.47 - 5.51 (1 H, m), 7.02 (1 H, dd, J=8.9, 2.3 Hz), 7.14 - 7.18 (1 H, m), 7.19 - 7.24 (2 H, m), 7.36 (1 H, d, J=2.1 Hz), 7.40 (1 H, dd, J=8.1, 1.9 Hz).

### •Example A-9: Synthesis of 2-[5-{3-chloro-5-[(dimethylamino)methyl]phenyl}-2-(4-fluoro-3-methoxyphenyl)-1H-benzimidazol-1-yl]-N-isopropyl acetamide

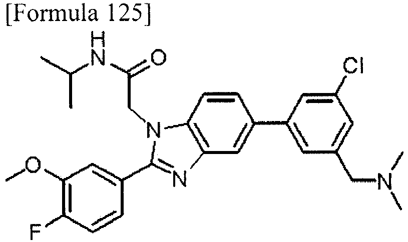

The compound (90 mg) obtained in Reference Example P-A16 was dissolved in a mixed solvent of THF (8 mL) and MeOH (3 mL) and after adding 50% methylamine in aqueous solution (68 µL) and AcOH (65 µL), the mixture was stirred for 30 minutes. Thereafter, NaBH₃CN (18.7 mg) was added and the mixture was stirred at room temperature for 16 hours. The stirred reaction mixture was purified by silica gel column chromatography (Chromatorex NH; mobile phase: chloroform/n-hexane = 80/20; v/v) and preparative pTLC (chloroform/methanol = 8/1; v/v) through two developments, and the roughly purified product of the higher polarity was washed with n-hexane/ethyl acetate (= 4/1; v/v) to give the titled compound (8.2 mg as colorless solids).
MS (ESI pos.) m/z: 509([M+H]⁺)
¹H-NMR (600 MHz, CDCl₃) δ (ppm); 1.11 (6 H, d, J=6.9 Hz), 2.29 (6 H, s), 3.45 - 3.53 (2 H, m), 3.96 (3 H, s), 4.17 - 4.24 (1 H, m), 4.81 (2 H, s), 5.50 (1 H, d, J=7.8 Hz), 7.17 - 7.24 (2 H, m), 7.30 - 7.33 (1 H, m), 7.38 (1 H, s), 7.42 - 7.46 (1 H, m), 7.49 (1 H, br. s.), 7.52 - 7.54 (1 H, m), 7.57 - 7.60 (1 H, m), 8.02 - 8.05 (1 H, m).

### •Example A-10: Synthesis of 2-[6-{3-chloro-5-[(dimethylamino)methyl]phenyl}-2-(4-fluoro-3-methoxyphenyl)-1H-benzimidazol-1-yl]-N-isopropyl acetamide

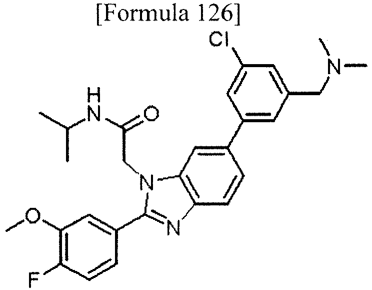

The roughly purified product of the lower polarity as obtained in Example A-9 was washed with n-hexane to give the titled compound (13.7 mg as colorless solids).
MS (ESI pos.) m/z: 509([M+H]⁺)
¹H-NMR (600 MHz, CDCl₃) δ (ppm); 1.10 - 1.16 (6 H, m), 2.24 - 2.38 (6 H, m), 3.44 - 3.55 (2 H, m), 3.97 (3 H, s), 4.18 - 4.26 (1 H, m), 4.85 (2 H, s), 5.51 - 5.64 (1 H, m), 7.21 - 7.26 (2 H, m), 7.30 - 7.33 (1 H, m), 7.44 - 7.55 (4 H, m), 7.58 - 7.62 (1 H, m), 7.90 (1 H, d, J=8.3 Hz).

### •Example A-11: Synthesis of 2-{2-(3-chloro-4-fluorophenyl)-5-[3-(piperidin-1-yl)propoxy]-1H-benzimidazol-1-yl}-N-isopropyl acetamide

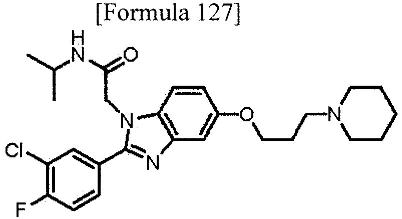

The compound (10 g) obtained in Reference Example P-A19, 1-piperidinepropanol (7.92 g) and CMBP (13.3 g) were dissolved in a mixed solvent of toluene (75 mL) and THF (25 mL) and the solution was stirred at an external temperature of 90°C for 30 minutes. After leaving the mixture to cool, the solvents were distilled off under reduced pressure. The resulting residue was purified by silica gel column chromatography [(neutral silica gel; mobile phase: chloroform/methanol = 100/0→90/10; v/v) and (Chromatorex NH; mobile phase: n-hexane/chloroform = 100/0→0/100; v/v)] and the resulting roughly purified product was washed with ethyl acetate/n-hexane (= 2/1; v/v) twice under stirring, recovered by filtration and dried to give the titled compound (8.80 g as pale pink solids).
MS (ESI pos.) m/z: 487([M+H]⁺)
¹H-NMR (600 MHz, DMSO-d₆) δ (ppm); 1.05 - 1.09 (6 H, m), 1.38 (2 H, d, J=5.5 Hz), 1.45 - 1.53 (4 H, m), 1.84 - 1.91 (2 H, m), 2.29 - 2.37 (4 H, m), 2.38 - 2.42 (2 H, m), 3.81 - 3.88 (1 H, m), 4.01 - 4.06 (2 H, m), 4.82 (2 H, s), 6.91 - 6.95 (1 H, m), 7.19 - 7.22 (1 H, m), 7.38 - 7.42 (1 H, m), 7.59 - 7.64 (1 H, m), 7.79 - 7.83 (1 H, m), 7.94 - 7.98 (1 H, m), 8.37 (1 H, d, J=7.3 Hz).

### •Example A-12: Synthesis of 2-{2-(3-chloro-4-fluorophenyl)-5-[3-(piperidin-1-yl)propoxy]-1H-benzimidazol-1-yl}-N-isopropyl acetamide hydrochloride

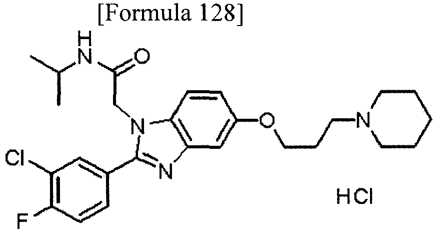

The compound (5.00 g) obtained in Example A-11 was dissolved in EtOH (44.8 mL) and after adding a HCl/ethanol solution (2 mol/L, 6.2 mL), the mixture was stirred at room temperature for an hour. The solvents were distilled off under reduced pressure and the residue was subjected to azeotropic distillation, three times each with EtOH and EtOAc. Following the addition of EtOAc (50 mL), EtOH (7.2 mL) was added under heating. After stirring overnight at room temperature, Et₂O (50 mL) was added and the mixture was recovered by filtration and dried to give the titled compound (4.87 g).
MS (ESI pos.) m/z: 487([M+H]⁺)
¹H-NMR (600 MHz, DMSO-d₆) δ (ppm); 1.07 (6 H, d, J=6.9 Hz), 1.66 - 1.85 (6 H, m), 2.14 - 2.24 (2 H, m), 2.85 - 2.96 (2 H, m), 3.21 (2 H, br. s.), 3.47 (2 H, d, J=10.5 Hz), 3.80 - 3.88 (1 H, m), 4.07 - 4.14 (2 H, m), 4.84 (2 H, s), 6.95 (1 H, dd, J=8.7, 2.3 Hz), 7.24 - 7.29 (1 H, m), 7.44 (1 H, d, J=9.2 Hz), 7.59 - 7.66 (1 H, m), 7.78 - 7.84 (1 H, m), 7.93 - 7.99 (1 H, m), 8.39 - 8.45 (1 H, m), 9.86 (1 H, br. s.).

### •Example A-13: Synthesis of 2-{5-[3-(azepan-1-yl)propoxy]-2-(3-chloro-4-fluorophenyl)-1H-benzimidazol-1-yl}-N-isopropyl acetamide

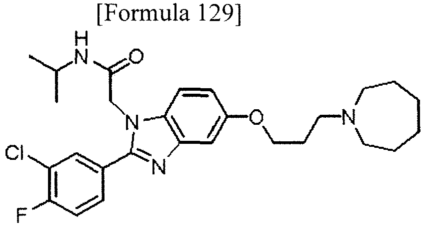

The compound (100 mg) obtained in Reference Example P-A24 and hexamethyleneimine (77.1 µL) were treated by same procedure as in Example A-2, yielding the titled compound (16.9 mg as pale yellow solids).
MS (ESI pos.) m/z: 501 ([M+H]⁺)
¹H-NMR (600 MHz, CDCl₃) δ (ppm); 1.11 (6 H, d, J=6.9 Hz), 1.50 - 1.61 (4 H, m), 1.68 - 1.80 (4 H, m), 2.05 - 2.15 (2 H, m), 2.80 (6 H, br. s.), 4.09 - 4.13 (2 H, m), 4.16 - 4.23 (1 H, m), 4.74 (2 H, s), 5.48 (1 H, d, J=7.8 Hz), 7.01 (1 H, dd, J=8.9, 2.5 Hz), 7.19 - 7.22 (1 H, m), 7.27 - 7.32 (2 H, m), 7.54 - 7.58 (1 H, m), 7.83 (1 H, dd, J=6.9, 2.3 Hz).

### •Example A-14: Synthesis of 2-{2-(3-chloro-4-fluorophenyl)-5-[3-(morpholin-4-yl)propoxy]-1H-benzimidazol-1-yl}-N-isopropyl acetamide

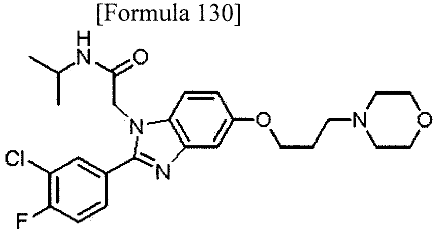

The compound (100 mg) obtained in Reference Example P-A24 and morpholine (59.8 µL) were treated by the same procedure as in Example A-2, yielding the titled compound (18.4 mg as colorless solids).
MS (ESI pos.) m/z: 489([M+H]⁺)
¹H-NMR (600 MHz, DMSO-d₆) δ (ppm); 1.07 (6 H, d, J=6.9 Hz), 1.86 - 1.93 (2 H, m), 2.34 - 2.41 (4 H, m), 2.45 (2 H, t, J=7.1 Hz), 3.58 (4 H, t, J=4.6 Hz), 3.80 - 3.89 (1 H, m), 4.03 - 4.08 (2 H, m), 4.82 (2 H, s), 6.94 (1 H, dd, J=8.7, 2.3 Hz), 7.21 (1 H, d, J=2.3 Hz), 7.38 - 7.43 (1 H, m), 7.59 - 7.64 (1 H, m), 7.79 - 7.84 (1 H, m), 7.96 (1 H, dd, J=7.1, 2.1 Hz), 8.38 (1 H, d, J=7.8 Hz).

### •Example A-15: Synthesis of 2-{2-(3-chloro-4-fluorophenyl)-5-[3-(2-oxa-6-azaspiro[3.3]hept-6-yl)propoxy]-1H-benzimidazol-1-yl}-N-isopropyl acetamide

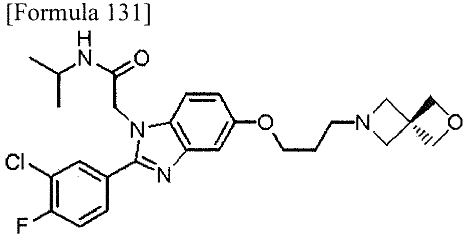

The compound (100 mg) obtained in Reference Example P-A25 and 2-oxa-6-azaspiro[3.3]heptane oxalate (99 mg) were treated by the same procedure as in Example A-2, yielding the titled compound (36 mg as colorless amorphous mass).
MS (ESI pos.) m/z: 501([M+H]⁺)
¹H-NMR (600 MHz, CDCl₃) δ (ppm); 1.11 (6 H, d, J=6.6 Hz), 1.82 - 1.89 (2 H, m), 2.56 - 2.62 (2 H, m), 3.34 - 3.44 (4 H, m), 4.03 - 4.07 (2 H, m), 4.16 - 4.23 (1 H, m), 4.73 (2 H, s), 4.75 (4 H, s), 5.45 (1 H, d, J=7.8 Hz), 7.00 (1 H, dd, J=8.9, 2.3 Hz), 7.18 - 7.22 (1 H, m), 7.27 - 7.32 (2 H, m), 7.53 - 7.57 (1 H, m), 7.83 (1 H, dd, J=6.8, 2.3 Hz).

### •Example A-16: Synthesis of 2-[2-(3-chloro-4-fluorophenyl)-5-{3-[(1R,5S)-3-oxa-8-azabicyclo[3.2.1]oct-8-yl]propoxy}-1H-benzimidazol-1-yl]-N-isopropyl acetamide

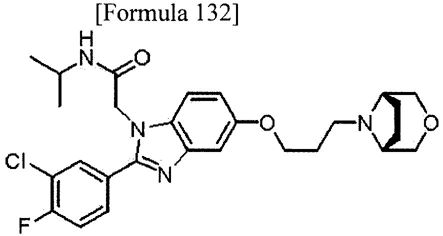

The compound (100 mg) obtained in Reference Example P-A25 and 3-oxa-8-azabicyclo[3.2.1]octane hydrochloride (77 mg) were treated by the same procedure as in Example A-2, yielding the titled compound (69.3 mg as pale brown solids).
MS (ESI pos.) m/z: 515([M+H]⁺)
¹H-NMR (600 MHz, CDCl₃) δ (ppm); 1.11 (6 H, d, J=6.2 Hz), 1.86 - 1.96 (4 H, m), 1.97-2.03 (2 H, m), 2.47 - 2.53 (2 H, m), 3.05 - 3.11 (2 H, m), 3.51 - 3.55 (2 H, m), 3.69 - 3.75 (2 H, m), 4.14 - 4.17 (2 H, m), 4.17 - 4.24 (1 H, m), 4.74 (2 H, s), 5.47 (1 H, d, J=8.3 Hz), 7.01 - 7.04 (1 H, m), 7.21 (1 H, d, J=8.7 Hz), 7.28 - 7.32 (1 H, m), 7.33 (1 H, d, J=2.1 Hz). 7.54 - 7.58 (1 H, m), 7.84 (1 H, dd, J=6.6, 2.1 Hz).

### •Example A-17: Synthesis of 2-{2-(3-chloro-4-fluorophenyl)-5-[4-(piperidin-1-yl)butoxy]-1H-benzimidazol-1-yl}-N-isopropyl acetamide

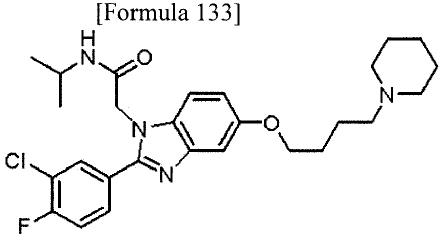

The compound (100 mg) obtained in Reference Example P-A26 and piperidine (82 µL) were treated by the same procedure as in Example A-2, yielding the titled compound (28.6 mg as colorless solids).
MS (ESI pos.) m/z: 501([M+H]⁺)
¹H-NMR (600 MHz, CDCl₃) δ (ppm); 1.10 (6 H, d, J=6.4 Hz), 1.44 (2 H, br. s.), 1.59 - 1.64 (4 H, m), 1.73 (2 H, d, J=7.3 Hz), 1.81 - 1.87 (2 H, m), 2.34 - 2.46 (6 H, m), 4.04 - 4.08 (2 H, m), 4.16 - 4.23 (1 H, m), 4.74 (2 H, s), 5.47 (1 H, d, J=8.3 Hz), 7.00 - 7.02 (1 H, m), 7.18 - 7.21 (1 H, m), 7.27 - 7.32 (2 H, m), 7.54 - 7.57 (1 H, m), 7.83 (1 H, dd, J=6.9, 1.8 Hz).

### •Example A-18: Synthesis of N-isopropyl-2-{2-(6-methoxypyridin-2-yl)-5-[3-(morpholin-4-yl)propoxy]-1H-benzimidazol-1-yl}acetamide

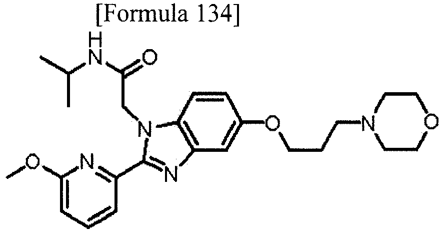

The compound (80 mg) obtained in Reference Example P-A29 was treated by the same procedure as in Example A-11, yielding the titled compound (13.9 mg as colorless solids).
MS (ESI pos.) m/z: 468([M+H]⁺)
¹H-NMR (600 MHz, DMSO-d₆) δ (ppm); 1.01 (6 H, d, J=6.4 Hz), 1.88 - 1.93 (2 H, m), 2.35 - 2.41 (4 H, m), 2.43 - 2.47 (2 H, m), 3.56 - 3.61 (4 H, m), 3.75 - 3.82 (1 H, m), 3.94 (3 H, s), 4.05 - 4.08 (2 H, m), 5.50 (2 H, s), 6.88 - 6.94 (2 H, m), 7.21 - 7.23 (1 H, m), 7.40 (1 H, d, J=8.7 Hz), 7.83 - 7.88 (1 H, m), 7.92 - 7.95 (1 H, m), 8.04 (1 H, d, J=7.3 Hz).

### • Example A-19: Synthesis of N-tert-butyl-2-[2-(4-fluoro-3-methoxyphenyl)-5-(4-methyl-1,4-diazepan-1-yl)-1H-benzimidazol-1-yl]acetamide

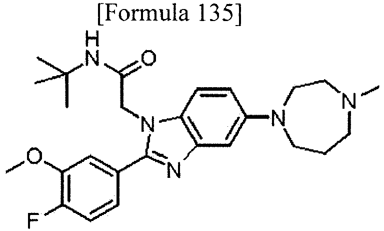

The compound (50 mg) obtained in Reference Example P-A33, 1-methyl-1,4-diazepane (17 µL), Pd₂(dba)₃ (11 mg) and 2'-(dicyclohexylphosphino)-N,N-dimethylbiphenyl-2-amine (9 mg) were suspended in THF (1.0 ml) and after pouring in a THF solution (253 µL) of 1 mol/L-LiHMDS, the mixture was stirred at 65°C for 2 days. The reaction mixture was concentrated under reduced pressure and the resulting residue was purified by silica gel column chromatography [(SNAP Cartridge HP-sphere; mobile phase: CHCl₃/MeOH = 100/0-80/20; v/v) and (SNAP Cartridge KP-NH; mobile phase: CHCl₃/MeOH = 100/0-99/1; v/v) and the resulting roughly purified product was washed with hexane and recovered by filtration to yield the titled compound (10.3 mg as colorless solids).
MS (ESI pos.) m/z: 468([M+H]⁺)
¹H-NMR (600 MHz, CDCl₃) δ (ppm); 1.30 (9 H, s), 2.03 - 2.12 (2 H, m), 2.41 (3 H, br. s.), 2.58 - 2.65 (2 H, m), 2.76 - 2.83 (2 H, m), 3.57 (2 H, t, J=6.2 Hz), 3.63 - 3.68 (2 H, m), 3.95 (3 H, s), 4.66 (2 H, s), 5.52 (1 H, s), 6.85 (1 H, dd, J=9.1, 2.5 Hz), 7.10 - 7.12 (1 H, m), 7.14 - 7.24 (3 H, m), 7.39 (1 H, dd, J=8.1, 1.9 Hz).

### • Example A-20: Synthesis of N-tert-butyl-2-[2-(4-fluoro-3-methoxyphenyl)-5-(4-isopropyl-1,4-diazepan-1-yl)-1H-benzimidazol-1-yl]acetamide

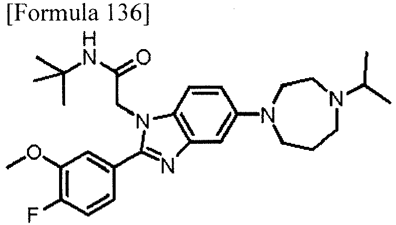

The compound (50 mg) obtained in Reference Example P-A33 was treated by the same procedure as in Example A-19, yielding the titled compound (13.3 mg as colorless solids).
MS (ESI pos.) m/z: 496([M+H]⁺)
¹H-NMR (600 MHz, CDCl₃) δ (ppm); 0.96 - 1.06 (6 H, m), 1.30 (9 H, s), 1.92 - 2.02 (2 H, m), 2.52 - 2.70 (2 H, m), 2.78 - 2.89 (2 H, m), 2.89 - 3.01 (1 H, m), 3.61 (4 H, br. s.), 3.95 (3 H, s), 4.66 (2 H, s), 5.52 (1 H, br. s.), 6.86 (1 H, d, J=8.7 Hz), 7.09 - 7.24 (4 H, m), 7.36 - 7.42 (1 H, m).

### • Example A-21: Synthesis of N-tert-butyl-2-{2-(4-fluoro-3-methoxyphenyl)-5-[4-(pyrrolidin-1-yl)piperidin-1-yl]-1H-benzimidazol-1-yl}acetamide

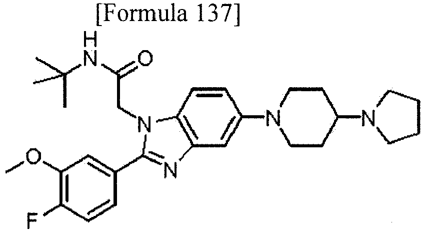

The compound (50 mg) obtained in Reference Example P-A33 was treated by the same procedure as in Example A-19, yielding the titled compound (7.5 mg as colorless solids).
MS (ESI pos.) m/z: 508([M+H]⁺)
¹H-NMR (600 MHz, CDCl₃) δ (ppm); 1.30 (9 H, s), 1.71 - 2.32 (13 H, m), 2.76 - 2.83 (2 H, m), 3.72 (2 H, d, J=11.6 Hz), 3.95 (3 H, s), 4.68 (2 H, s), 5.47 (1 H, s), 7.10 (1 H, d, J=8.7 Hz), 7.23 (3 H, d, J=2.9 Hz), 7.33 - 7.40 (2 H, m).

### • Example A-22: Synthesis of 2-[2-(3-chloro-4-fluorophenyl)-5-(4-methyl-1,4-diazepan-1-yl)-1H-benzimidazol-1-yl]-N-isopropyl acetamide

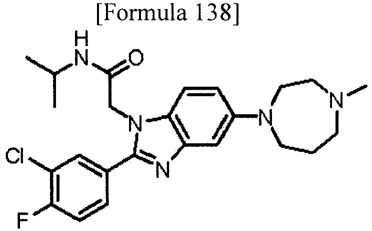

The compound (200 mg) obtained in Reference Example P-A17 was treated by the same procedure as in Example A-19, yielding the titled compound (5.6 mg as colorless solids).
MS (ESI pos.) m/z: 458([M+H]⁺)
¹H-NMR (600 MHz, CDCl₃) δ (ppm); 1.11 (6 H, d, J=6.6 Hz), 2.02 - 2.21 (2 H, m), 2.37 - 2.54 (3 H, m), 2.54 - 2.72 (2 H, m), 2.72 - 2.90 (2 H, m), 3.57 (2 H, t, J=6.2 Hz), 3.69 (2 H, br. s.), 4.16 - 4.25 (1 H, m), 4.71 (2 H, s), 5.52 (1 H, d, J=8.3 Hz), 6.84 - 6.90 (1 H, m), 7.16 (2 H, d, J=9.1 Hz), 7.28 - 7.31 (1 H, m), 7.51 - 7.58 (1 H, m), 7.83 (1 H, dd, J=7.0, 2.1 Hz).

### • Example A-23: Synthesis of N-tert-butyl-2-[2-(3-chloro-4-fluorophenyl)-5-(4-methyl-1,4-diazepan-1-yl)-1H-benzimidazol-1-yl]acetamide

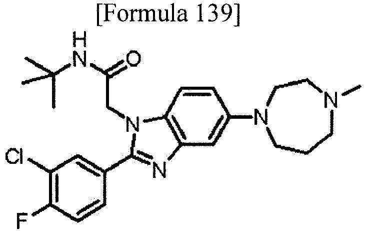

The compound (200 mg) obtained in Reference Example P-A36 was treated by the same procedure as in ExampleA-19, yielding the titled compound (26.2 mg as colorless solids).
MS (ESI pos.) m/z: 472([M+H]⁺)
¹H-NMR (600 MHz, CDCl₃) δ (ppm); 1.32 (9 H, s), 2.02 - 2.18 (2 H, m), 2.38 - 2.47 (3 H, m), 2.57 - 2.70 (2 H, m), 2.75 - 2.85 (2 H, m), 3.57 (2 H, t, J=6.4 Hz), 3.61 - 3.73 (2 H, m), 4.64 (2 H, s), 5.46(1 H, s), 6.87(1 H, dd, J=9.1, 2.5 Hz), 7.07 - 7.11 (1 H, m), 7.17 (1 H, d, J=8.7 Hz), 7.29 (1 H, t, J=8.7 Hz), 7.58 (1 H, ddd, J=8.6, 4.4, 2.3 Hz), 7.83 (1 H, dd, J=6.8, 2.3 Hz).

### • Example B-1: Synthesis of 2-{2-(3-chlorophenyl)-5-[3-(piperidin-1-yl)propoxy]-1H-indol-1-yl}-N-isopropyl acetamide

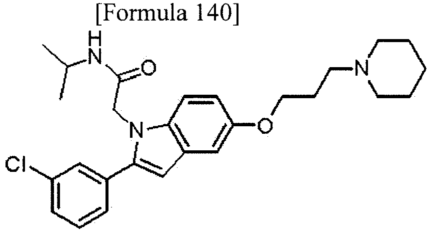

A solution in toluene (7.0 mL) of the compound (200 mg) obtained in Reference Example P-B3, 1-piperidinepropanol (148 mg) and cyanomethylenetributylphosphorane (312 mg) was stirred at 85°C for 4 hours. To the reaction mixture, a saturated aqueous solution of NaHCO₃ was added and extraction was conducted with CHCl₃. The organic layer was washed with Brine and after drying with MgSO₄, the desiccant was separated by filtration and the solvents were distilled off under reduced pressure. The resulting residue was purified by silica gel column chromatography (SNAP Cartridge KP-Sil, 25 g; mobile phase: CHCl₃/MeOH/NH₄OH = 99/1/0.1-95/5/0.5; v/v/v). The purified product was washed in suspension with IPE and the solids were recovered by filtration to give the titled compound (178 mg as colorless solids).
MS (ESI pos.) m/z: 468([M+H]⁺)
¹H-NMR (600 MHz, CDCl₃) δ (ppm); 1.04 (6 H, d, J=6.9 Hz), 1.41 - 1.51 (2 H, m), 1.58 - 1.66 (4 H, m), 1.97 - 2.08 (2 H, m), 2.35 - 2.58 (6 H, m), 4.09 (2 H, t, J=6.4 Hz), 4.11-4.18 (1 H, m), 4.65 (2 H, s), 5.33 - 5.38 (1 H, m), 6.61 (1 H, s), 6.93 - 6.98 (1 H, m), 7.12 - 7.19 (2 H, m), 7.28 - 7.31 (1 H, m), 7.38 - 7.41 (2H, m), 7.44 (1 H, s).

Using the same procedure as in Example B-1, the compounds of Examples B-2 and B-3 were synthesized from Reference Example P-B3.

### • Example B-2: 2-{2-(3-chlorophenyl)-5-[3-(morpholin-4-yl)propoxy]-1H-indol-1-yl}-N-isopropyl acetamide

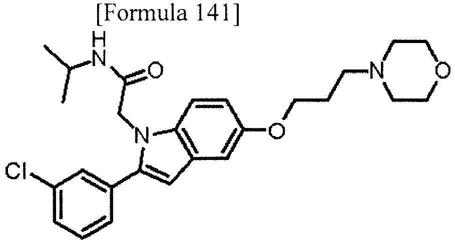

MS (ESI pos.) m/z: 470([M+H]⁺)
¹H-NMR (600 MHz, CDCl₃) δ (ppm); 1.04 (6 H, d, J=6.4 Hz), 1.99 - 2.06 (2 H, m), 2.45 - 2.61 (6 H, m), 3.75 (4 H, t, J=4.6 Hz), 4.08 - 4.18 (3 H, m), 4.65 (2 H, s), 5.33 - 5.38 (1 H, m), 6.61 (1 H, s), 6.96 (1 H, dd, J=8.9, 2.5 Hz), 7.14 (1 H, d, J=2.3 Hz), 7.17 (1 H, d, J=8.7 Hz), 7.28-7.32 (1 H, m), 7.38 - 7.41 (2 H, m), 7.43 - 7.46 (1 H, m).

### • Example B-3: 2-{2-(3-chlorophenyl)-5-[(1-cyclopropylpiperidin-4-yl)oxy]-1H-indol-1-yl}-N-isopropyl acetamide

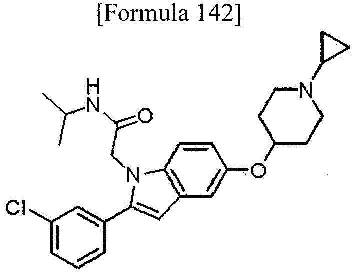

MS (ESI pos.) m/z: 466([M+H]⁺)
¹H-NMR (600 MHz, CDCl₃) δ (ppm); 0.39 - 0.52 (4 H, m), 1.05 (6 H, d, J=6.9 Hz), 1.62 - 1.68 (1 H, m), 1.79 - 1.88 (2 H, m), 1.97 - 2.05 (2 H, m), 2.45 - 2.54 (2 H, m), 2.91 - 2.99 (2 H, m), 4.10 - 4.18 (1 H, m), 4.34 (1 H, br. s.), 4.65 (2 H, s), 5.39 (1 H, d, J=8.3 Hz), 6.61 (1 H, s), 6.98 (1 H, dd, J=8.7, 2.3 Hz), 7.16 - 7.20 (2 H, m), 7.28 - 7.31 (1 H, m), 7.39 - 7.41 (2 H, m), 7.43 - 7.45 (1 H, m).

### • Example B-4: Synthesis of 2-{2-(3-chloro-4-fluorophenyl)-5-[3-(piperidin-1-yl)propoxy]-1H-indol-1-yl}-N-isopropyl acetamide

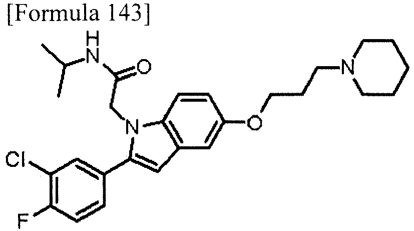

A solution in EtOH (1.4 mL) of the compound (70.0 mg) obtained in Reference Example P-B8, piperidine (40.2 mg), iPr₂NEt (81.3 mg) and potassium iodide (26.1 mg) was stirred overnight at an external temperature of 80°C. After leaving the reaction mixture to cool, water was added and extraction was conducted with CHCl₃. After drying the organic layer with MgSO₄, the desiccant was separated by filtration and the solvents were distilled off under reduced pressure. The resulting residue was purified by reverse-phase silica gel column chromatography (mobile phase: 0.1% TFA MeCN/H₂O = 10/90-90/10; v/v). The fractions were neutralized with a saturated aqueous solution of NaHCO₃ and subjected to extraction with CHCl₃; after drying with MgSO₄, the desiccant was separated by filtration and the solvents were distilled off under reduced pressure to give the titled compound (12.3 mg as brown solids).
MS (ESI pos.) m/z: 486([M+H]⁺)
¹H-NMR (600 MHz, CDCl₃) δ (ppm); 1.05 (6 H, d, J=6.4 Hz), 1.42 - 1.50 (2 H, m), 1.59 - 1.67 (4 H, m), 1.98 - 2.09 (2 H, m), 2.36 - 2.58 (6 H, m), 4.06 - 4.10 (2 H, m), 4.10 - 4.17 (1 H, m), 4.63 (2 H, s), 5.30 - 5.35 (1 H, m), 6.57 (1 H, s), 6.96 (1 H, dd, J=8.9, 2.5 Hz), 7.13 (1 H, d, J=2.3 Hz), 7.16 (1 H, d, J=9.2 Hz), 7.22 - 7.26 (1 H, m), 7.27 - 7.30 (1 H, m), 7.48 - 7.51 (1 H, m).

Using the same procedure as in Example B-4, the compounds of Examples B-5 to B-16 were synthesized from Reference Examples P-B4, P-B8 and P-B12.

### • Example B-5: 2-{2-(3-chloro-4-fluorophenyl)-5-[3-(morpholin-4-yl)propoxy]-1H-indol-1-yl}-N-isopropyl acetamide

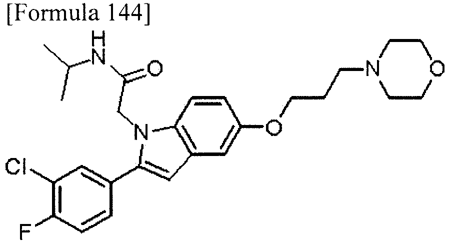

MS (ESI pos.) m/z: 488([M+H]⁺)
¹H-NMR (600 MHz, CDCl₃) δ (ppm); 1.05 (6 H, d, J=6.9 Hz), 1.99 - 2.06 (2 H, m), 2.50 (4 H, br. s.), 2.56 - 2.60 (2 H, m), 3.72 - 3.77 (4 H, m), 4.08 - 4.17 (3 H, m), 4.63 (2 H, s), 5.31 - 5.35 (1 H, m), 6.58 (1 H, s), 6.96 (1 H, dd, J=9.2, 2.3 Hz), 7.14 (1 H, d, J=2.3 Hz), 7.17 (1 H, d, J=9.2 Hz), 7.23 - 7.26 (1 H, m), 7.27 - 7.30 (1 H, m), 7.48 - 7.51 (1 H, m).

### • Example B-6: 2-{2-(3-chlorophenyl)-5-[3-(4-hydroxypiperidin-1-yl)propoxy]-1H-indol-1-yl}-N-isopropyl acetamide

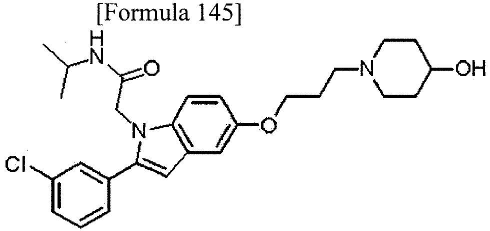

MS (ESI pos.) m/z: 484([M+H]⁺)
¹H-NMR (600 MHz, CDCl₃) δ (ppm); 1.04 (6 H, d, J=6.9 Hz), 1.34 - 1.43 (1 H, m), 1.59 - 1.66 (2 H, m), 1.88 - 1.95 (2 H, m), 1.97 - 2.06 (2 H, m), 2.12 - 2.27 (2 H, m), 2.49 - 2.61 (2 H, m), 2.77 - 2.89 (2 H, m), 3.68 - 3.77 (1 H, m), 4.09 (2 H, t, J=6.2 Hz), 4.14 (1 H, d, J=8.3 Hz), 4.65 (2 H, s), 5.36 (1 H, d, J=8.3 Hz), 6.61 (1 H, s), 6.95 (1 H, dd, J=8.7, 2.3 Hz), 7.14 (1 H, d, J=2.3 Hz), 7.17 (1 H, d, J=8.7 Hz), 7.28 - 7.31 (1 H, m), 7.39 - 7.41 (2 H, m), 7.44 (1 H, br. s).

### • Example B-7: 2-{2-(3-chlorophenyl)-5-[3-(3-hydroxypiperidin-1-yl)propoxy]-1H-indol-1-yl}-N-isopropyl acetamide

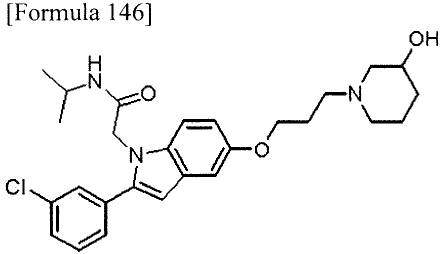

MS (ESI pos.) m/z: 484([M+H]⁺)
¹H-NMR (600 MHz, CDCl₃) δ (ppm); 1.04 (6 H, d, J=6.4 Hz), 1.51 - 1.56 (1 H, m), 1.58 - 1.65 (2 H, m), 1.78 - 1.86 (1 H, m), 1.98 - 2.04 (2 H, m), 2.25 - 2.62 (7 H, m), 3.81 - 3.88 (1 H, m), 4.09 (2 H, t, J=6.2 Hz), 4.11 - 4.18 (1 H, m), 4.65 (2 H, s), 5.37 (1 H, d, J=8.3 Hz), 6.61 (1 H, s), 6.95 (1 H, dd, J=8.7, 2.3 Hz), 7.14 (1 H, d, J=2.3 Hz), 7.17 (1 H, d, J=8.7 Hz), 7.28 - 7.31 (1 H, m), 7.39 - 7.41 (2 H, m), 7.43 - 7.45 (1 H, m).

### • Example B-8: 2-{2-(3-chlorophenyl)-5-[3-(3-hydroxypyrrolidin-1-yl)propoxy]-1H-indol-1-yl}-N-isopropyl acetamide

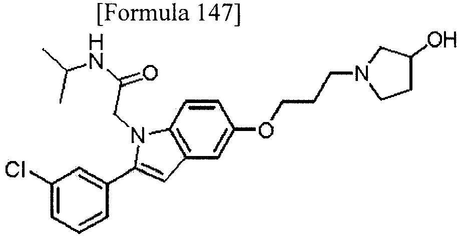

MS (ESI pos.) m/z: 470([M+H]⁺)
¹H-NMR (600 MHz, CDCl₃) δ (ppm); 1.04 (6 H, d, J=6.4 Hz), 1.73 - 1.80 (1 H, m), 1.85 - 1.95 (1 H, m), 2.01 - 2.08 (2 H, m), 2.17 - 2.24 (1 H, m), 2.30 - 2.35 (1 H, m), 2.55 (1 H, dd, J=10.1, 5.0 Hz), 2.69 (2 H, t, J=7.3 Hz), 2.74 (1 H, d, J=10.1 Hz), 2.92 - 2.97 (1 H, m), 4.09 - 4.18 (3 H, m), 4.36 (1 H, br. s.), 4.65 (2 H, s), 5.37 (1 H, d, J=8.3 Hz), 6.61 (1 H, s), 6.96 (1 H, dd, J=8.9, 2.5 Hz), 7.14 (1 H, d, J=2.3 Hz), 7.17 (1 H, d, J=8.7 Hz), 7.28 - 7.31 (1 H, m), 7.38 - 7.41 (2 H, m), 7.43 - 7.45 (1 H, m).

### • Example B-9: 2-{2-(3-ehlorophenyl)-5-[3-(2-oxa-6-azaspiro[3.3]hept-6-yl)propoxy]-1H-indol-1-yl}-N-isopropyl acetamide

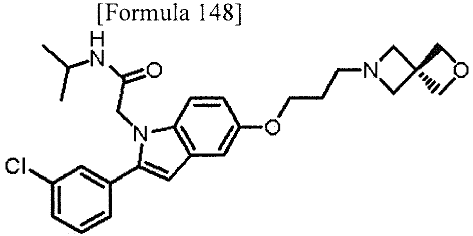

MS (ESI pos.) m/z: 482([M+H]⁺)
¹H-NMR (600 MHz, CDCl₃) δ (ppm); 1.03 - 1.06 (6 H, m), 1.83 - 1.88 (2 H, m), 2.59 (2 H, t, J=7.1 Hz), 3.38 (4 H, s), 4.05 (2 H, t, J=6.4 Hz), 4.10 - 4.17 (1 H, m), 4.65 (2 H, s), 4.76 (4 H, s), 5.33 - 5.37 (1 H, m), 6.59 - 6.61 (1 H, m), 6.92 - 6.95 (1 H, m), 7.12 (1 H, d, J=2.3 Hz), 7.17 (1 H, d, J=8.7 Hz), 7.28 - 7.31 (1 H, m), 7.39 - 7.41 (2 H, m), 7.43 - 7.45 (1 H, m).

### • Example B-10: 2-{2-(3-chlorophenyl)-5-[3-(2-oxa-7-azaspiro[3.5]non-7-yl)propoxy]-1H-indol-1-yl}-N-isopropyl acetamide

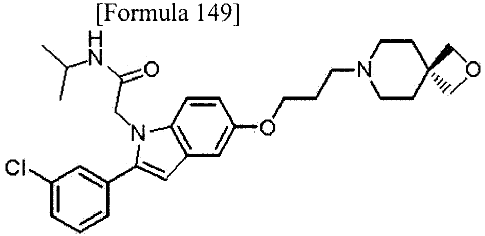

MS (ESI pos.) m/z: 510([M+H]⁺)
¹H-NMR (600 MHz, CDCl₃) δ (ppm); 1.04 (6 H, d, J=6.4 Hz), 1.83 - 2.05 (6 H, m), 2.26 - 2.55 (6 H, m), 4.08 (2 H, t, J=6.4 Hz), 4.11 - 4.17 (1 H, m), 4.43 (4 H, s), 4.65 (2 H, s), 5.35 (1 H, d, J=8.3 Hz), 6.60 (1 H, s), 6.93 - 6.96 (1 H, m), 7.13 (1 H, d, J=2.3 Hz), 7.17 (1 H, d, J=8.7 Hz), 7.28 - 7.31 (1 H, m), 7.39 - 7.41 (2 H, m), 7.43 - 7.45 (1 H, m).

### • Example B-11: 2-{2-(4-fluoro-3-methoxyphenyl)-5-[3-(piperidin-1-yl)propoxy]-1H-indol-1-yl}-N-isopropyl acetamide

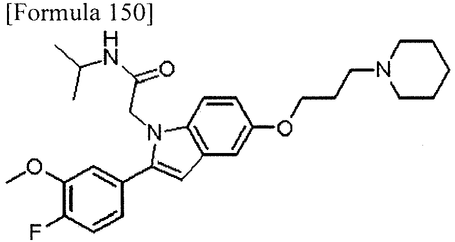

MS (ESI pos.) m/z: 482([M+H]⁺)
¹H-NMR (600 MHz, CDCl₃) δ (ppm); 1.04 (6 H, d, J=6.4 Hz), 1.43 - 1.50 (2 H, m), 1.60 - 1.66 (4 H, m), 2.00 - 2.07 (2 H, m), 2.38 - 2.58 (6 H, m), 3.91 (3 H, s), 4.08 (2 H, t, J=6.4 Hz), 4.10 - 4.17 (1 H, m), 4.65 (2 H, s), 5.39 - 5.43 (1 H, m), 6.57 (1 H, d, J=0.9 Hz), 6.93 - 6.97 (2 H, m), 7.01 (1 H, dd, J=8.0, 2.1 Hz), 7.13 - 7.19 (3 H, m).

### • Example B-12: 2-{2-(4-fluoro-3-methoxyphenyl)-5-[3-(4-hydroxypiperidin-1-yl)propoxy]-1H-indol-1-yl}-N-isopropyl acetamide

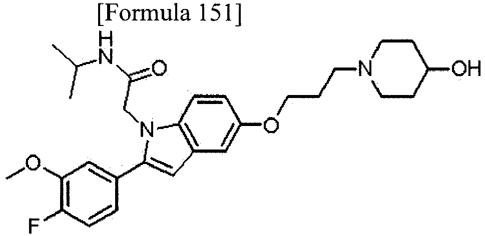

MS (ESI pos.) m/z: 498([M+H]⁺)
¹H-NMR (600 MHz, CDCl₃) δ (ppm); 1.04 (6 H, d, J=6.9 Hz), 1.36 - 1.42 (1 H, m), 1.59 - 1.67 (2 H, m), 1.89 - 1.97 (2 H, m), 1.99 - 2.06 (2 H, m), 2.14 - 2.24 (2 H, m), 2.53 - 2.60 (2 H, m), 2.79 - 2.87 (2 H, m), 3.69 - 3.77 (1 H, m), 3.91 (3 H, s), 4.09 (2 H, t, J=6.2 Hz), 4.10 - 4.17 (1 H, m), 4.65 (2 H, s), 5.41 (1 H, d, J=8.3 Hz), 6.57 (1 H, s), 6.92 - 6.97 (2 H, m), 7.01 (1 H, dd, J=7.8, 1.8 Hz), 7.12 - 7.19 (3 H, m).

### • Example B-13: 2-{2-(4-fluoro-3-methoxyphenyl)-5-[3-(3-hydroxypiperidin-1-yl)propoxy]-1H-indol-1-yl)-N-isopropyl acetamide

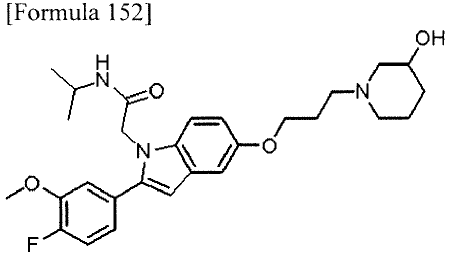

MS (ESI pos.) m/z: 498([M+H]⁺)
¹H-NMR (600 MHz, CDCl₃) δ (ppm); 1.04 (6 H, d, J=6.4 Hz), 1.51 - 1.65 (3 H, m), 1.78-1.88 (1 H, m), 1.98 - 2.05 (2 H, m), 2.25 - 2.63 (7 H, m), 3.85 (1 H, br. s.), 3.91 (3 H, s), 4.09 (2 H, t, J=6.2 Hz), 4.11 - 4.17 (1 H, m), 4.65 (2 H, s), 5.42 (1 H, d, J=8.3 Hz), 6.58 (1 H, s), 6.92 - 6.97 (2 H, m), 7.01 (1 H, dd, J=8.0, 2.1 Hz), 7.12 - 7.19 (3 H, m).

### • Example B-14: 2-{2-(4-fluoro-3-methoxyphenyl)-5-[3-(3-hydroxypyrrolidin-1-yl)propoxy]-1H-indol-1-yl}-N-isopropyl acetamide

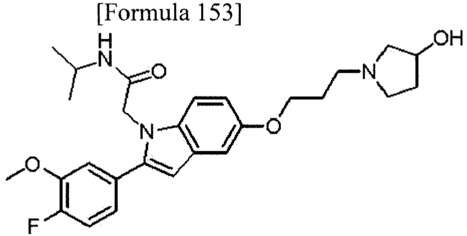

MS (ESI pos.) m/z: 484([M+H]⁺)
¹H-NMR (600 MHz, CDCl₃) δ (ppm); 1.04 (6 H, d, J=6.4 Hz), 1.74 - 1.81 (1 H, m), 1.86-1.96 (1 H, m), 2.02 - 2.08 (2 H, m), 2.17 - 2.25 (1 H, m), 2.30 - 2.37 (1 H, m), 2.52 - 2.59 (1 H, m), 2.68 - 2.72 (2 H, m), 2.74 - 2.77 (1 H, m), 2.92 - 2.98 (1 H, m), 3.91 (3 H, s), 4.09-4.17 (3 H, m), 4.33 - 4.39 (1 H, m), 4.65 (2 H, s), 5.41 (1 H, d, J=8.3 Hz), 6.57 (1 H, s), 6.93 - 6.97 (2 H, m), 7.01 (1 H, dd, J=8.0, 2.1 Hz), 7.13 - 7.19 (3 H, m).

### • Example B-15: 2-{2-(4-fluoro-3-methoxyphenyl)-5-[3-(2-oxa-6-azaspiro[3.3]hept-6-yl)propoxy]-1H-indol-1-yl}-N-isopropyl acetamide

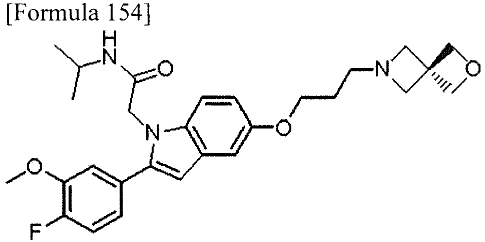

MS (ESI pos.) m/z: 496([M+H]⁺)
¹H-NMR (600 MHz, CDCl₃) δ (ppm); 1.04 (6 H, d, J=6.4 Hz), 1.82 - 1.89 (2 H, m), 2.56 - 2.61 (2 H, m), 3.38 (4 H, s), 3.91 (3 H, s), 4.05 (2 H, t, J=6.4 Hz), 4.09 - 4.17 (1 H, m), 4.65 (2 H, s), 4.76 (4 H, s), 5.38 - 5.42 (1 H, m), 6.56 (1 H, s), 6.91 - 6.97 (2 H, m), 6.99 - 7.02 (1 H, m), 7.12 (1 H, d, J=2.3 Hz), 7.14 - 7.19 (2 H, m).

### • Example B-16: 2-[2-(4-fluoro-3-methoxyphenyl)-5-{3-[(2R)-2-methylpyrrolidin-1-yl]propoxy}-1H-indol-1-yl]-N-isopropyl acetamide

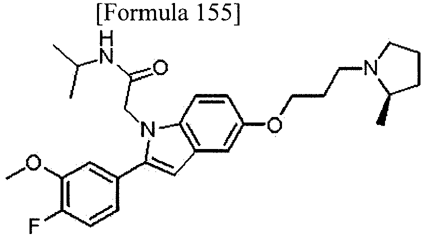

MS (ESI pos.) m/z: 482([M+H]⁺)
¹H-NMR (600 MHz, CDCl₃) δ (ppm); 1.04 (6 H, d, J=6.4 Hz), 1.09 - 1.16 (3 H, m), 1.38-1.49 (1 H, m), 1.64 - 2.37 (8 H, m), 2.99 - 3.08 (1 H, m), 3.16 - 3.26 (1 H, m), 3.92 (3 H, s), 4.06 - 4.18 (3 H, m), 4.66 (2 H, s), 5.38 - 5.43 (1 H, m), 6.57 (1 H, s), 6.93 - 6.97 (2 H, m), 6.99 - 7.02 (1 H, m), 7.13 - 7.19 (3 H, m).

### • Example B-17: Synthesis of 2-{2-(3-chloro-4-fluorophenyl)-5-[(1-cyclopentylpiperidin-4-yl)oxy]-1H-indol-1-yl}-N-isopropyl acetamide

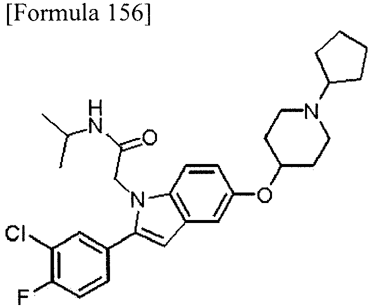

To a solution in CHCl₃ (3.5 mL) of the compound (70.0 mg) obtained in Reference Example P-B 14 and cyclopentanone (26.5 mg), acetic acid (47.3 mg) was added and the mixture was stirred at room temperature for an hour. Subsequently, triacetoxyborohydride (73.5 mg) was added and the mixture was stirred at room temperature for 3 hours. After adding water to the reaction mixture, extraction was conducted with CHCl₃. After drying the organic layer with MgSO₄, the desiccant was separated by filtration and the solvents were distilled off under reduced pressure. The resulting residue was purified by silica gel column chromatography (Biotage SNAP Cartridge KP-Sil, 10 g; mobile phase: CHCl₃/MeOH/NH₄OH = 99/1/0.1-95/5/0.5; v/v/v). The purified product was washed in suspension with IPE and the solids were recovered by filtration to give the titled compound (33.8 mg as brown solids).
MS (ESI pos.) m/z: 512([M+H]⁺)
¹H-NMR (600 MHz, CDCl₃) δ (ppm); 1.05 (6 H, d, J=6.4 Hz), 1.40 - 1.48 (2 H, m), 1.49-1.62 (2 H, m), 1.66 - 1.76 (2 H, m), 1.84 - 1.95 (4 H, m), 2.01 - 2.10 (2 H, m), 2.29 - 2.41 (2 H, m), 2.50 - 2.58 (1 H, m), 2.81 - 2.91 (2H,m),4.11 -4.18(1 H, m), 4.30 - 4.37 (1 H, m), 4.62 (2 H, s), 5.33 - 5.37 (1 H, m), 6.57 (1 H, s), 6.96 - 6.99 (1 H, m), 7.15 - 7.19 (2 H, m), 7.22 - 7.26 (1 H, m), 7.28 - 7.30 (1 H, m), 7.48 - 7.51 (1 H, m).

### • Example B-18: Synthesis of 2-{2-(3-chlorophenyl)-5-[4-(piperidin-1-yl)butyl]-1H-indol-1-yl}-N-isopropyl acetamide

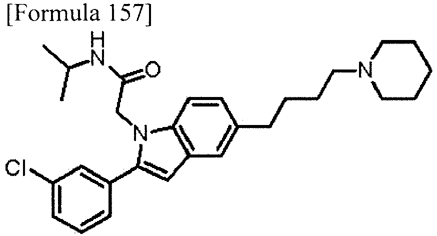

To a MeCN suspension (1.5 mL) of the compound (150 mg) obtained in Reference Example P-B17, K₂CO₃ (217 mg) and piperidine (0.093 mL) were added and the mixture was stirred at 80°C for 5 hours. After leaving the mixture to cool, water was added and extraction was conducted with CHCl₃. The organic layer was washed with Brine and dried with Na₂SO₄; thereafter, the desiccant was separated by filtration and the solvents were distilled off under reduced pressure. The resulting residue was purified by reverse-phase silica gel column chromatography (mobile phase: 0.1% TFA MeCN/H₂O = 10/90-90/10; v/v). The fractions were neutralized with a saturated aqueous solution of NaHCO₃ and extraction was conducted with CHCl₃; after drying with MgSO₄, the desiccant was separated by filtration and the solvents were distilled off under reduced pressure to give the titled compound (47 mg as colorless solids).
¹H-NMR (600 MHz, CDCl₃) δ (ppm); 1.04 - 1.07 (6 H, m), 1.41 - 2.72 (16 H, m), 2.74 - 2.80 (2 H, m), 4.12 - 4.19 (1 H, m), 4.66 (2 H, s), 5.41 - 5.46 (1 H, m), 6.64 (1 H, s), 7.11 - 7.14 (1 H, m), 7.18 - 7.21 (1 H, m), 7.29 - 7.32 (1 H, m), 7.38 - 7.41 (2 H, m), 7.44 - 7.48 (2 H, m).

### • Example B-19: Synthesis of N-isopropyl-2-{2-(5-methoxypyridin-3-yl)-5-[3-(piperidin-1-yl)propoxy]-1H-indol-1-yl}acetamide

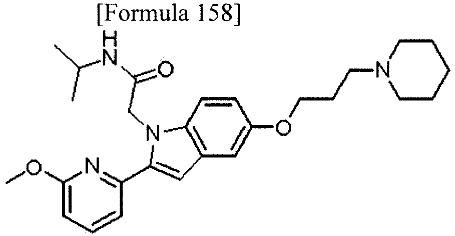

The compound (90 mg) obtained in Reference Example P-B19 was treated by the same procedure as in Reference Example P-B2, yielding the titled compound (22 mg as colorless amorphous mass).
MS (ESI pos.) m/z: 465([M+H]⁺)
¹H-NMR (600 MHz, CDCl₃) δ (ppm); 1.04 (6 H, d, J=6.6 Hz), 1.35 - 3.12 (14 H, m), 3.91 (3 H, s), 4.08 - 4.17 (3 H, m), 4.66 (2 H, s), 5.35 - 5.39 (1 H, m), 6.67 (1 H, s), 6.95 (1 H, dd, J=8.9, 2.3 Hz), 7.14 (1 H, d, J=2.1 Hz), 7.18 (1 H, d, J=9.1 Hz), 7.22 (1 H, dd, J=2.7, 1.9 Hz), 8.33 (1 H, d, J=1.7 Hz), 8.36 (1 H, d, J=2.9 Hz).

### • Example B-20: Synthesis of N-isopropyl-2-{2-(3-methoxyphenyl)-5-[3-(piperidin-1-yl)propoxy]-1H-indol-1-yl}acetamide

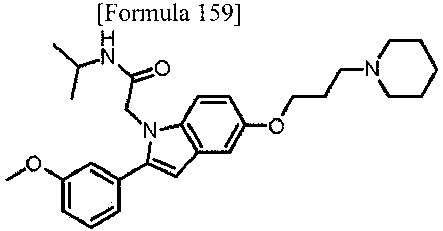

The compound (90 mg) obtained in Reference Example P-B20 was treated by the same procedure as in Reference Example P-B2, yielding the titled compound (35 mg as colorless amorphous mass).
MS (ESI pos.) m/z: 464([M+H]⁺)
¹H-NMR (600 MHz, CDCl₃) δ (ppm); 1.03 (6 H, d, J=6.6 Hz), 1.37 - 2.86 (14 H, m), 3.85 (3 H, s), 4.08 - 4.17 (3 H, m), 4.68 (2 H, s), 5.38 - 5.42 (1 H, m), 6.60 (1 H, s), 6.90 - 6.94 (1 H, m), 6.94 - 6.98 (2 H, m), 6.99 - 7.01 (1 H, m), 7.12 - 7.14 (1 H, m), 7.15 - 7.18 (1 H, m), 7.35 - 7.39 (1 H, m).

### • Example B-21: Synthesis of 2-{2-(3-chlorophenyl)-5-[3-(piperidin-1-yl)propoxy]-1H-pyrrolo[2,3-b]pyridin-1-yl}-N-isopropyl acetamide

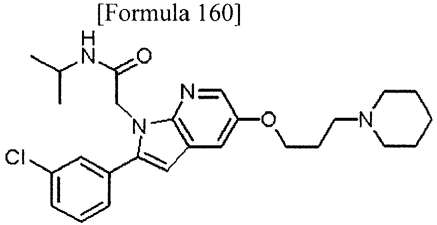

The compound (30 mg) obtained in Reference Example P-B25 was treated by the same procedure as in Example A-11, yielding the titled compound (26.8 mg as pale brown solids).
MS (ESI pos.) m/z: 469([M+H]⁺)
¹H-NMR (600 MHz, CDCl₃) δ (ppm); 1.08 (6 H, d, J=6.4 Hz), 1.41 - 1.52 (4 H, m), 1.65 (4 H, br. s.), 1.99 - 2.16 (2 H, m), 2.46 (4 H, br. s.), 4.03 - 4.09 (1 H, m), 4.10 - 4.13 (2 H, m), 4.79 (2 H, s), 6.30 - 6.36 (1 H, m), 6.53 (1 H, s), 7.39 - 7.44 (2 H, m), 7.45 - 7.47 (1 H, m), 7.48 - 7.51 (1 H, m), 7.58 - 7.61 (1 H, m), 8.10 (1 H, d, J=2.8 Hz).

### • Example B-22: Synthesis of 2-{2-(3-chlorophenyl)-5-[3-(piperidin-1-yl)propoxy]-1H-pyrrolo[3,2-b]pyridin-1-yl}-N-isopropyl acetamide

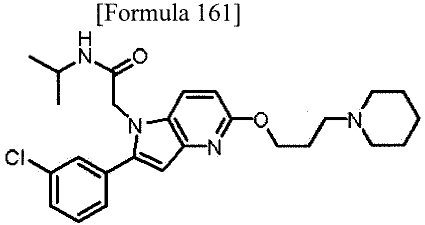

The compound (100 mg) obtained in Reference Example P-B28, 1-piperidinepropanol (150 µL), palladium(II) acetate (8.9 mg), rac-2-(di-t-butylphosphino)-1,1'-binaphthyl (9.8 mg) and cesium carbonate (267 mg) were placed in 1,4-dioxane (10 mL) and the mixture was stirred at an external temperature of 110°C for 23 hours. The insoluble matter was separated by filtration through Celite (registered trademark) and the filtrate was concentrated under reduced pressure. The resulting residue was purified by silica gel column chromatography (Biotage SNAP Cartridge KP-NH; mobile phase: chloroform), reverse-phase silica gel column chromatography (mobile phase: 0.1% TFA MeCN/H₂O = 10/90-90/10; v/v) and silica gel column chromatography (Biotage SNAP Cartridge HP-Sil; mobile phase: chloroform/MeOH = 99/1-70/30; v/v) and the resulting roughly purified product was washed with n-hexane, filtered, and dried to give the titled compound (27.0 mg as pale brown solids).
MS (ESI pos.) m/z: 469([M+H]⁺)
¹H-NMR (600 MHz, DMSO-d₆) δ (ppm); 1.06 (6 H, s), 1.34 - 1.45 (2 H, m), 1.45 - 1.62 (6 H, m), 1.85 - 2.01 (2 H, m), 2.38 (4 H, br. s.), 3.81 - 3.87 (1 H, m), 4.29 - 4.34 (2 H, m), 4.70 (2 H, s), 6.60 (1 H, s), 6.62 (1 H, d, J=9.2 Hz), 7.49 - 7.60 (4 H, m), 7.76 (1 H, d, J=8.7 Hz), 8.16 (1 H, d, J=7.8 Hz).

### • Example C-1: Synthesis of 2-{2-(3-chloro-4-fluorophenyl)-7-[3-(piperidin-1-yl)propoxy]imidazo[1,2-a]pyridin-3-yl}-N-isopropyl acetamide

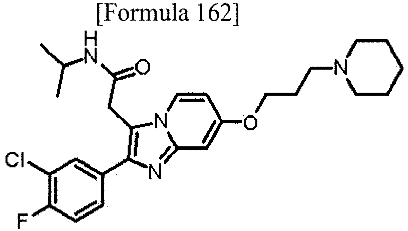

The compound (100 mg) obtained in Reference Example P-C5 was treated by the same procedure as in Example B-1, yielding the titled compound (30 mg as colorless solids). MS (ESI pos.) m/z: 487([M+H]⁺)
¹H-NMR (600 MHz, DMSO-d₆) δ (ppm); 1.10 (6 H, d, J=6.4 Hz), 1.34 - 1.41 (2 H, m), 1.47 - 1.53 (4 H, m), 1.86 - 1.93 (2 H, m), 2.28 - 2.42 (6 H, m), 3.83 - 3.90 (3 H, m), 4.09 (2 H, t, J=6.4 Hz), 6.68 (1 H, dd, J=7.6, 2.5 Hz), 6.94 (1 H, d, J=2.3 Hz), 7.50 (1 H, t, J=8.9 Hz), 7.80 - 7.84 (1 H, m), 7.95 (1 H, dd, J=7.3, 1.8 Hz), 8.26 (1 H, d, J=7.3 Hz), 8.27 - 8.31 (1 H, m).

### • Example C-2: Synthesis of 2-{2-(3-chloro-4-fluorophenyl)-7-[3-(morpholin-4-yl)propoxy]imidazo[1,2-a]pyridin-3-yl}-N-isopropyl acetamide

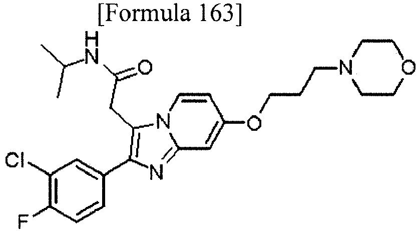

The compound (100 mg) obtained in Reference Example P-C5 was treated by the same procedure as in Example B-1, yielding the titled compound (36 mg as colorless solids). MS (ESI pos.) m/z: 489([M+H]⁺) ¹H-NMR (600 MHz, DMSO-d₆) δ (ppm); 1.10 (6 H, d, J=6.4 Hz), 1.88 - 1.95 (2 H, m), 2.33 - 2.41 (4 H, m), 2.44 (2 H, t, J=7.1 Hz), 3.54 - 3.61 (4 H, m), 3.81 - 3.92 (3 H, m), 4.10 (2 H, t, J=6.4 Hz), 6.67 - 6.70 (1 H, m), 6.95 (1 H, d, J=2.8 Hz), 7.50 (1 H, t, J=8.9 Hz), 7.79 - 7.84 (1 H, m), 7.95 (1 H, dd, J=7.3, 2.3 Hz), 8.26 (1 H, d, J=7.3 Hz), 8.29 (1 H, d, J=7.3 Hz).

### • Example D-1: Synthesis of 2-{1-(3-chlorophenyl)-3-oxo-5-[3-(piperidin-1-yl)propoxy]-1,3-dihydro-2H-indazol-2-yl}-N-isopropyl acetamide

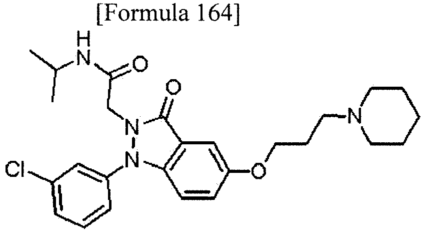

The compound (30 mg) obtained in Reference Example P-D4 was treated by the same procedure as in Example A-11, yielding the titled compound (11.0 mg as pale brown solids).
MS (ESI pos.) m/z: 485([M+H]⁺)
¹H-NMR (600 MHz, CDCl₃) δ (ppm); 1.09 (6 H, d, J=6.4 Hz), 1.42 - 1.50 (2 H, m), 1.61 - 1.70 (4 H, m), 1.99 - 2.09 (2 H, m), 2.36 - 2.60 (6 H, m), 3.96 - 4.03 (1 H, m), 4.04 - 4.08 (2 H, m), 4.31 (2 H, s), 6.12 (1 H, d, J=7.3 Hz), 6.94 (1 H, d, J=8.7 Hz), 7.15 (1 H, dd, J=8.9, 2.5 Hz), 7.18 - 7.22 (2 H, m), 7.31 - 7.33 (1 H, m), 7.36 - 7.44 (2 H, m).

### • Example E-1: Synthesis of 2-{2-(3-chlorophenyl)-5-[4-(piperidin-1-yl)butyl]-4,5,6,7-tetrahydro-1H-pyrrolo[3,2-c]pyridin-1-yl}-N-isopropyl acetamide

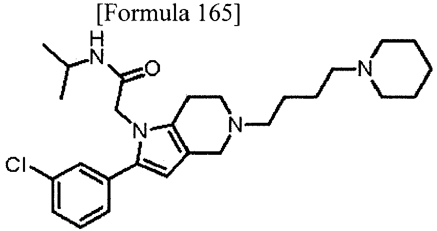

To a MeCN solution (3.0 mL) of the compound (143 mg) obtained in Reference Example P-E3, iPr₂NEt (0.184 mL) and 1-bromo-4-chlorobutane (0.06 mL) were added and the mixture was stirred overnight at 60°C. After concentrating under reduced pressure, the resulting residue was mixed with piperidine (1.5 mL) and the mixture was subjected to reaction under microwave irradiation (180°C x 2 hr). The reaction mixture was concentrated under reduced pressure and the resulting residue was purified by reverse-phase silica gel column chromatography (mobile phase: 0.1% TFA MeCN/H₂O = 10/90-90/10; v/v). The fractions were neutralized with a saturated aqueous solution of NaHCO₃ and extraction was conducted with CHCl₃; after drying with MgSO₄, the desiccant was separated by filtration and the solvents were distilled off under reduced pressure. The resulting purified product was further purified by TLC plate NH (mobile phase: n-Hexane/EtOAc = 1/2; v/v) to give the titled compound (9.4 mg as yellow amorphous mass).
MS (ESI pos.) m/z: 471 ([M+H]⁺)
¹H-NMR (600 MHz, CDCl₃) δ (ppm); 1.07 - 1.10 (6 H, m), 1.41 - 1.48 (2 H, m), 1.53 - 2.22 (8 H, m), 2.28 - 2.47 (6 H, m), 2.54 - 2.59 (2 H, m), 2.60 - 2.64 (2 H, m), 2.81 (2 H, t, J=5.7 Hz), 3.50 (2 H, s), 3.65 (1 H, s), 4.06 - 4.13 (1 H, m), 4.43 (2 H, s), 5.24 (1 H, d, J=8.7 Hz), 6.11 (1 H, s), 7.14 (1 H, dt, J=7.5, 1.5 Hz), 7.24 - 7.32 (3 H, m).

### • Example F-1: Synthesis of 2-{3-(3-chlorophenyl)-7-[3-(piperidin-1-yl)propoxy]imidazo[1,2-a]pyridin-2-yl}-N-isopropyl acetamide

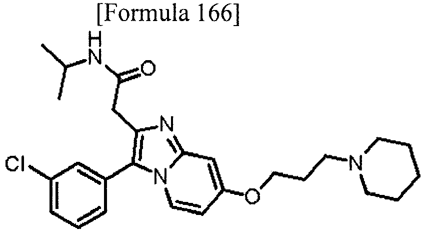

The compound (90 mg) obtained in Reference Example P-F5 was treated by the same procedure as in Example B-1, yielding the titled compound (25 mg as yellow amorphous mass).
MS (ESI pos.) m/z: 469([M+H]⁺)
¹H-NMR (600 MHz, CDCl₃) δ (ppm); 1.16 (6 H, d, J=6.4 Hz), 1.46 (2 H, br. s.), 1.57 - 1.65 (2 H, m), 1.68 (2 H, br. s.), 1.99 - 2.08 (2 H, m), 2.34 - 2.54 (6 H, m), 3.62 (2 H, s), 4.03 - 4.14 (3 H, m), 6.54 (1 H, dd, J=7.6, 2.5 Hz), 6.88 (1 H, d, J=2.8 Hz), 7.24 - 7.26 (1 H, m), 7.32 - 7.36 (1 H, m), 7.39 - 7.42 (1 H, m), 7.43 - 7.48 (2 H, m), 7.93 (1 H, d, J=7.3 Hz).

### Test Example 1

### • Binding test for V1b receptor

Human V1b receptor was transiently expressed in 293FT cells, which were then recovered and homogenated in a 15 mmol/L tris-hydrochloric acid buffer (pH 7.4 and containing 2 mmol/L magnesium chloride, 0.3 mmol/L ethylenediaminetetracetic acid, and 1 mmol/L glycol ether diaminetetraacetic acid). The resulting homogenate was centrifuged at 50,000 × g at 4 °C for 20 minutes. The precipitate was resuspended in a 75 mmol/L tris-hydrochloric acid buffer (pH 7.4 and containing 12.5 mmol/L magnesium chloride, 0.3 mmol/L ethylenediaminetetracetic acid, 1 mmol/L glycol ether diaminetetraacetic acid, and 250 mmol/L sucrose) to give a crude membrane preparation, which was stored at -80°C until the binding test was initiated. In the binding test, the crude membrane preparation was diluted with a 50 mmol/L tris-hydrochloric acid buffer (pH 7.4 and containing 10 mmol/L magnesium chloride and 0.1% bovine serum albumin) and mixed with each test compound and [³H]AVP (final concentration: 0.4 to 1 nmol/L), followed by incubation at room temperature for 60 minutes. The test compound was serially diluted with DMSO so that it would have final concentrations of 0.01 nmol/L to 1 µmol/L at the time of mixing. After the incubation, the mixture was suction filtered through a GF/C filter that was preliminarily impregnated with 0.3% polyethyleneimine. The GF/C filter was dried and after adding a scintillator, the residual radioactivity on the filter was measured using TopCount (PerkinElmer Inc.). The radioactivity in the presence of unlabeled AVP at 10 µmol/L was defined as 0%, and the radioactivity in the absence of unlabeled AVP was defined as 100%. A dose-response curve was plotted from radioactivities in the presence of a test compound at various concentrations, and the 50% inhibitory concentration (IC₅₀ value) of the test compound was calculated. The IC₅₀ values of the compounds of the present invention were in the range from about 1 nM to about 1000 nM. The IC₅₀ values of representative compounds are shown in Table 1 below.

**[Table 1]**

| Ex. No. | IC₅₀ Value (nmol/L) | Ex. No. | IC₅₀ Value (nmol/L) | Ex. No. | IC₅₀ Value (nmol/L) |
|---|---|---|---|---|---|
| A-1 | 14.4 | B-1 | 8.7 | C-1 | 10.7 |
| A-2 | 14.1 | B-2 | 6.4 | C-2 | 9.9 |
| A-3 | 8.3 | B-3 | 10.4 | D-1 | 375 |
| A-4 | 5.0 | B-4 | 4.9 | E-1 | 522 |
| A-5 | 43.3 | B-5 | 8.9 | F-1 | 82.0 |
| A-6 | 29.3 | B-6 | 18.4 | | |
| A-7 | 4.8 | B-7 | 73.4 | | |
| A-8 | 2.5 | B-8 | 52.3 | | |
| A-9 | 2.6 | B-9 | 5.3 | | |
| A-10 | 141 | B-10 | 20.2 | | |
| A-12 | 7.6 | B-11 | 52.1 | | |
| A-13 | 32.9 | B-12 | 45.3 | | |
| A-14 | 12.5 | B-13 | 87.1 | | |
| A-15 | 3.8 | B-14 | 87.2 | | |
| A-16 | 1.7 | B-15 | 8.3 | | |
| A-17 | 176 | B-16 | 166 | | |
| A-18 | 83.6 | B-17 | 0.86 | | |
| A-19 | 3.4 | B-18 | 20.1 | | |
| A-20 | 8.6 | B-19 | 387 | | |
| A-21 | 1.1 | B-20 | 129 | | |
| A-22 | 15.4 | B-21 | 21.4 | | |
| A-23 | 3.1 | B-22 | 47.1 | | |

### Test Example 2

### • Measurement of V1b receptor antagonistic activity

CHO cells so modified as to express human V1b receptor in a stable manner were cultured in Ham's F-12 medium (containing 10% FBS and 0.5 mg/mL Geneticin). On the day before the test, seeding was conducted at a density of 20,000 cells/well in a 96-well poly-D-lysine coated black plate. On the day of the test, the culture medium was removed, and a loading solution (1 × HBSS, 10 mmol/L HEPES, 0.1% bovine serum albumin, 1.25 mmol/L Probenecid, 0.02% Pluronic F-127, 1.5 µmol/L Fluo-4-AM, pH 7.4) was added to each well, followed by incubation in a CO₂ incubator for an hour. After the incubation, the loading solution was removed. A test solution (1 × HBSS, 10 mmol/L HEPES, 0.1% bovine serum albumin, 1.25 mmol/L Probenecid, pH 7.4) containing any one of test compounds was added to wells, followed by incubation in a CO₂ incubator for 30 minutes. The test compound was serially diluted with DMSO so that it would have final concentrations of 0.1 nmol/L to 1 µmol/L at the time of assaying. After the incubation, fluorescence intensity levels were measured and AVP added by means of FDSS (Hamamatsu Photonics K.K.); AVP was added to give a final concentration of 2.5 nmol/L at the time of assaying. At this concentration, AVP shows 70 to 80% of its maximum response. The fluorescence level in a well to which neither test compound nor AVP was added was defined as 0%, and the fluorescence level in a well to which only AVP was added and no test compound was added was defined as 100%. A dose-response curve was plotted from fluorescence levels after the addition of AVP in the presence of a test compound at various concentrations, and the 50% inhibitory concentration (IC₅₀ value) of the compound was calculated. The results are shown in Table 2 below.

**[Table 2]**

| Ex. No. | IC₅₀ Value (nmol/L) |
|---|---|
| A-1 | 212 |
| A-5 | 122 |
| A-6 | 25.9 |
| A-9 | 101 |
| A-11 | 88.0 |
| A-13 | 62.4 |
| A-14 | 53.5 |
| B-1 | 97.0 |
| B-4 | 21.9 |
| B-5 | 8.0 |
| B-9 | 21.4 |
| B-17 | 6.0 |
| B-21 | 50.8 |

### INDUSTRIAL APPLICABILITY

The present invention is able to provide agents for treating or preventing mood disorder (including depression), anxiety disorder, schizophrenia, Alzheimer's disease, Parkinson's disease, Huntington's chorea, eating disorder, hypertension, gastrointestinal disease, drug addiction, epilepsy, cerebral infarction, cerebral ischemia, cerebral edema, head injury, inflammation, immune-related disease, alopecia, and so forth.

## Claims

1. A fused azole derivative represented by Formula (I): 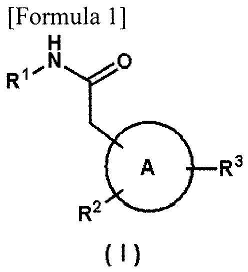 [in Formula (I),
R¹ represents a C₁₋₅ alkyl (the C₁₋₅ alkyl is optionally substituted by one to three groups selected from the group consisting of hydroxy, halogen atoms, cyano, C₃₋₇ cycloalkyl, and C₁₋₅ alkoxy), C₃₋₇ cycloalkyl, or 4- to 8-membered saturated heterocycle;
R² represents aryl or heteroaryl (the aryl and heteroaryl are optionally substituted by one or two groups selected from the group consisting of C₁₋₅ alkoxy, C₁₋₅ alkyl, halogen atoms, trifluoromethyl, trifluoromethoxy, cyano, hydroxy, difluoromethoxy, and C₁₋₅ alkylsulfonyl); R³ represents either the following formula (II), (III) or (IIIa): 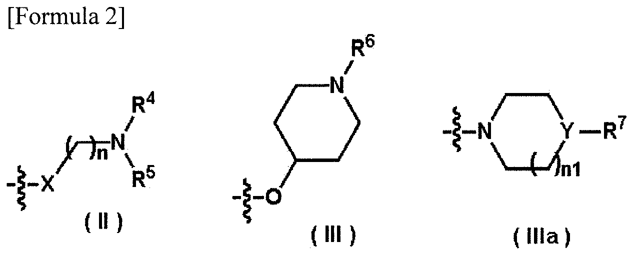 X represents a single bond, an oxygen atom or phenylene (the phenylene is optionally substituted by a halogen atom);
n represents an integer of 1 to 4;
R⁴ and R⁵ which may be the same or different each represent a hydrogen atom, C₁₋₅ alkyl (the C₁₋₅ alkyl is optionally substituted by one to three groups selected from the group consisting of hydroxy, halogen atoms, cyano, C₃₋₇ cycloalkyl, and C₁₋₅ alkoxy), C₃₋₇ cycloalkyl, or a 4-to 8-membered saturated or unsaturated heterocycle containing one or more nitrogen, oxygen or sulfur atoms in the ring (the 4- to 8-membered saturated or unsaturated heterocycle is optionally substituted by one or two groups selected from the group consisting of hydroxy, C₁₋₅ alkyl, C₁₋₅ alkoxy, halogen atoms, cyano, C₂₋₅ alkanoyl, and trifluoromethyl), or
R⁴ and R⁵, together with the adjoining nitrogen atom, may form a 4- to 8-membered saturated or unsaturated heterocycle optionally containing one or more nitrogen, oxygen or sulfur atoms in the ring in addition to the adjoining nitrogen atom (the 4- to 8-membered saturated or unsaturated heterocycle is optionally substituted by one or two groups selected from the group consisting of hydroxy, C₁₋₅ alkyl (the C₁₋₅ alkyl is optionally substituted by one or two hydroxyl groups), C₁₋₅ alkoxy, halogen atoms, cyano, C₂₋₅ alkanoyl, oxo, aminocarbonyl, mono-C₁₋₅ alkylaminocarbonyl, di-C₁₋₅ alkylaminocarbonyl, and trifluoromethyl, and the 4-to 8-membered saturated or unsaturated heterocycle optionally has a C₁₋₅ alkylene group crosslinking two different carbon atoms in the ring), 2-oxa-6-azaspiro[3.3]hept-6-yl, or 2-oxa-7-azaspiro[3.5]non-7-yl;
R⁶ represents C₁₋₅ alkyl, C₃₋₇ cycloalkyl, or a 4- to 8-membered saturated heterocycle;
Y represents a nitrogen atom or the formula CH;
when Y is a nitrogen atom, R⁷ represents C₁₋₅ alkyl;
when Y is the formula CH, R⁷ represents a 4- to 8-membered nitrogen-containing saturated heterocycle;
n1 represents an integer of 1 or 2;
ring A represents any one of the structures in the following formula group (IV)] 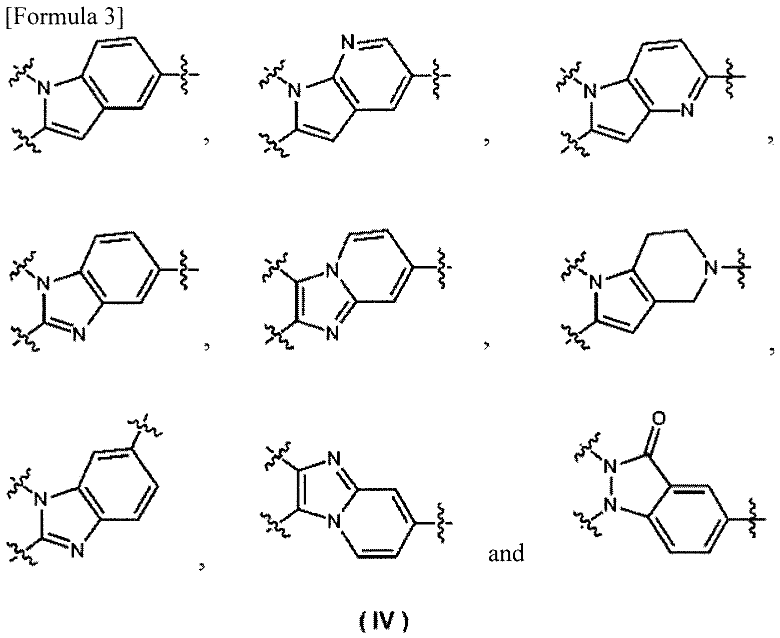 or a pharmaceutically acceptable salt thereof.

2. A fused azole derivative represented by Formula (I): 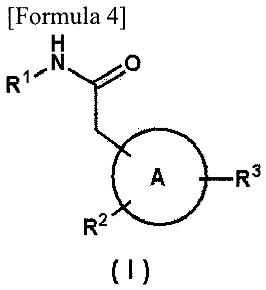 [in Formula (I),
R¹ represents a C₁₋₅ alkyl (the C₁₋₅ alkyl is optionally substituted by one to three groups selected from the group consisting of hydroxy, halogen atoms, cyano, C₃₋₇ cycloalkyl, and C₁₋₅ alkoxy), C₃₋₇ cycloalkyl, or 4- to 8-membered saturated heterocycle;
R² represents aryl or heteroaryl (the aryl and heteroaryl are optionally substituted by one or two groups selected from the group consisting of C₁₋₅ alkoxy, C₁₋₅ alkyl, halogen atoms, trifluoromethyl, trifluoromethoxy, cyano, hydroxy, difluoromethoxy, and C₁₋₅ alkylsulfonyl);
R³ represents either the following formula (II) or (III): 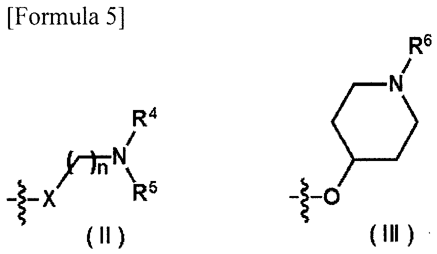 X represents a single bond, an oxygen atom or phenylene (the phenylene is optionally substituted by a halogen atom);
n represents an integer of 1 to 4;
R⁴ and R⁵ which may be the same or different each represent a hydrogen atom, C₁₋₅ alkyl (the C₁₋₅ alkyl is optionally substituted by one to three groups selected from the group consisting of hydroxy, halogen atoms, cyano, C₃₋₇ cycloalkyl, and C₁₋₅ alkoxy), C₃₋₇ cycloalkyl, or a 4-to 8-membered saturated or unsaturated heterocycle containing one or more nitrogen, oxygen or sulfur atoms in the ring (the 4- to 8-membered saturated or unsaturated heterocycle is optionally substituted by one or two groups selected from the group consisting of hydroxy, C₁₋₅ alkyl, C₁₋₅ alkoxy, halogen atoms, cyano, C₂₋₅ alkanoyl, and trifluoromethyl), or
R⁴ and R⁵, together with the adjoining nitrogen atom, may form a 4- to 8-membered saturated or unsaturated heterocycle optionally containing one or more nitrogen, oxygen or sulfur atoms in the ring in addition to the adjoining nitrogen atom (the 4- to 8-membered saturated or unsaturated heterocycle is optionally substituted by one or two groups selected from the group consisting of hydroxy, C₁₋₅ alkyl (the C₁₋₅ alkyl is optionally substituted by one or two hydroxyl groups), C₁₋₅ alkoxy, halogen atoms, cyano, C₂₋₅ alkanoyl, oxo, aminocarbonyl, mono-C₁₋₅ alkylaminocarbonyl, di-C₁₋₅ alkylaminocarbonyl, and trifluoromethyl, and the 4-to 8-membered saturated or unsaturated heterocycle optionally has a C₁₋₅ alkylene group crosslinking two different carbon atoms in the ring), 2-oxa-6-azaspiro[3.3]hept-6-yl, or 2-oxa-7-azaspiro[3.5]non-7-yl;
R⁶ represents C₁₋₅ alkyl, C₃₋₇ cycloalkyl, or a 4- to 8-membered saturated heterocycle;
ring A represents any one of the structures in the following formula group (IV)] 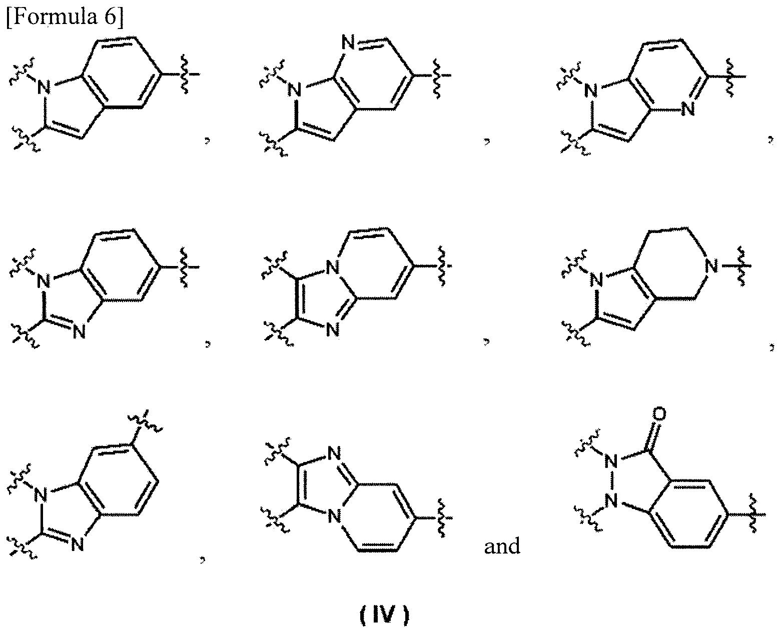 or a pharmaceutically acceptable salt thereof.

3. The fused azole derivative or a pharmaceutically acceptable salt thereof according to Claim 1 or 2, wherein in Formula (I),
R¹ is C₁₋₅ alkyl;
R² represents aryl or heteroaryl (the aryl and heteroaryl are optionally substituted by one or two groups selected from the group consisting of C₁₋₅ alkoxy and halogen atoms);
R⁴ and R⁵ which may be the same or different are each C₁₋₅ alkyl; or
R⁴ and R⁵, together with the adjoining nitrogen atom, may form a 4- to 8-membered saturated heterocycle optionally containing one or more oxygen atoms in the ring in addition to the adjoining nitrogen atom (the 4- to 8-membered saturated heterocycle is optionally substituted by one or two groups selected from the group consisting of hydroxy and C₁₋₅ alkyl, and the 4-to 8-membered saturated heterocycle optionally has a C₁₋₅ alkylene group crosslinking two different carbon atoms in the ring), 2-oxa-6-azaspiro[3.3]hept-6-yl, or 2-oxa-7-azaspiro[3.5]non-7-yl;
R⁶ is C₃₋₇ cycloalkyl.

4. The fused azole derivative or a pharmaceutically acceptable salt thereof according to any one of Claims 1 to 3, wherein in Formula (I),
R² is phenyl or pyridyl (the phenyl and pyridyl are optionally substituted by one or two groups selected from the group consisting of C₁₋₅ alkoxy and halogen atoms).

5. The fused azole derivative or a pharmaceutically acceptable salt thereof according to any one of Claims 1 to 4, wherein in Formula (I),
ring A represents any one of the structures in the following formula group (IX):

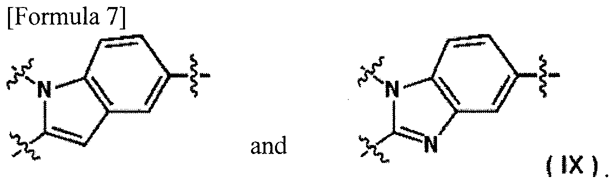

6. An agent for treating or preventing mood disorder, anxiety disorder, schizophrenia, Alzheimer's disease, Parkinson's disease, Huntington's chorea, eating disorder, hypertension, gastrointestinal disease, drug addiction, epilepsy, cerebral infarction, cerebral ischemia, cerebral edema, head injury, inflammation, immune-related disease, or alopecia, the agent comprising the fused azole derivative or pharmaceutically acceptable salt thereof according to any one of Claims 1 to 5 as an active ingredient.

7. A pharmaceutical composition comprising the fused azole derivative or pharmaceutically acceptable salt thereof according to any one of Claims 1 to 5 as an active ingredient.
